# FASCICULE DE BREVET EUROPEEN

(11) **EP 0 755 380 B1**
(45) Date de publication et mention de la délivrance du brevet: **14.10.1998**
(21) Numéro de dépôt: 96903066.7
(22) Date de dépôt: 07.02.1996
(51) Int. Cl.: C07C 279/14, C07C 271/20, A61K 31/16, C07C 247/04

(54) **ANALOGUES DE LA 15-DEOXYSPERGUALINE, LEUR UTILISATION EN THERAPEUTIQUE ET LEUR PROCEDE DE PREPARATION**
15-DEOXYSPERGUALIN-ANALOGE VERBINDUNGEN, IHRE VERWENDUNG ALS THERAPEUTIKA UND VERFAHREN ZU IHRER HERSTELLUNG
15-DEOXYSPERGUALIN ANALOGS, THERAPEUTICAL USE THEREOF, AND METHOD FOR PREPARING SAME

(30) Priorité: 10.02.1995 FR 9501553; 17.08.1995 FR 9509884
(43) Date de publication de la demande: 29.01.1997
(73) Titulaire: FOURNIER INDUSTRIE ET SANTE, F-75008 Paris (FR)
(72) Inventeur: LEBRETON, Luc, F-21000 Dijon (FR); RENAUT, Patrice, F-21121 Hauteville-lès-Dijon (FR); DURAND, Philippe, F-92300 Levallois-Perret (FR)
(74) Mandataire: Clisci, Serge
(86) Numéro de dépôt international: FR9600203
(87) Numéro de publication internationale: WO9624579

(56) Documents cités:
- EP-A- 0 105 193
- EP-A- 0 181 592
- US-A- 4 525 299
- THE JOURNAL OF ANTIBIOTICS, vol. XL, no. 9, 1987, pages 1303-1315, XP002000678 Y. UMEDA ET AL.: "Synthesis and Antitumor Activity of Spergualin Analogues" cité dans la demande

## Description

La présente invention concerne en tant que produits industriels nouveaux des composés de structure apparentée à la 15-déoxyspergualine qui répond à la nomenclature de 7-[(aminoiminométhyl)amino]-N-[2-[[4-[(3-aminopropyl)amino]butyl]amino]-1-hydroxy-2-oxoéthyl]heptanamide. Elle concerne également leur procédé de préparation et leur utilisation en thérapeutique.

### Art antérieur

On sait que la 15-déoxyspergualine (DSG), également connue sous la dénomination commune internationale "Gusperimus", possède une activité intéressante dans le domaine de l'immunosuppression. De nombreuses publications font état de cette activité : on trouvera notamment une série d'articles sur ce sujet dans : "Immunomodulating Drugs" - Annals of the New York Academy of Sciences, Vol 685, pages 123 à 201-.

Cependant, la 15-déoxyspergualine ne présente pas une stabilité chimique satisfaisante et on a cherché à obtenir des composés plus stables, par exemple (i) en remplaçant le groupe α-hydroxyglycine de la 15-déoxyspergualine par divers α- ou ω-aminoacides, (ii) en modifiant la structure du chaînon central, ou encore (iii) en modifiant le chaînon porteur de la fonction guanidine. Des exemples de telles modifications sont décrits dans EP-A-0 181 592, EP-A-0 105 193 et FR-A-2 698 628.

Les modifications portant sur le chaînon spermidine ont été essentiellement étudiées dans J. Antibiot. 40, pages 1303-1315 et la plupart des composés préparés étaient inactifs. Aucune des structures proposées ne montrait une activité au moins équivalente à celle de la DSG et les auteurs concluaient à la présence indispensable de l'enchaînement spermidine.

La présente invention concerne des composés analogues de la 15-déoxyspergualine, mais dont l'enchaînement spermidine a été modifié et qui présentent une activité supérieure aux produits connus.

### Objet de l'invention

La présente invention propose de nouveaux composés dont la structure générale reste apparentée à la 15-déoxyspergualine et qui présentent une activité supérieure aux produits connus de l'art antérieur dans le domaine de l'immunosuppression.

Les produits selon l'invention se distinguent notamment des produits connus de l'art antérieur par la présence d'un groupe méthyle sur l'atome de carbone porteur de la fonction amine primaire du chaînon spermidine de la molécule. Le choix d'une configuration particulière de cet atome de carbone asymétrique (noté ci-après **C) permet également d'améliorer l'activité de ces nouveaux composés.

Les composés analogues de la 15-déoxyspergualine selon l'invention sont caractérisés en ce qu'ils sont choisis parmi l'ensemble constitué par :
**(i)** les produits de formule : 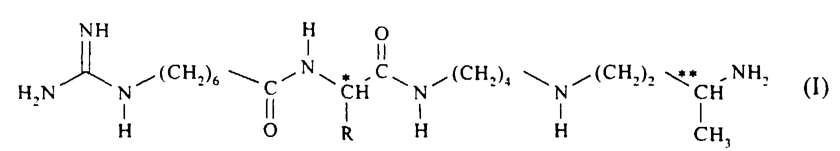 dans laquelle :
   - R représente un atome d'hydrogène, un groupe OH, un groupe OCH₃ ou un groupe CH₂OH,
   - *C, dans le cas où R n'est pas un atome d'hydrogène, est un atome de carbone asymétrique dont la configuration est (R,S), (R) ou (S),
   - **C est un atome de carbone asymétrique de configuration indéterminée (R,S) ou (R) ; et,
**(ii)** leurs sels d'addition.

Selon l'invention, on préconise également un procédé de préparation des composés de formule (I) et de leurs sels d'addition, ledit procédé comprenant la déprotection d'un composé de formule : 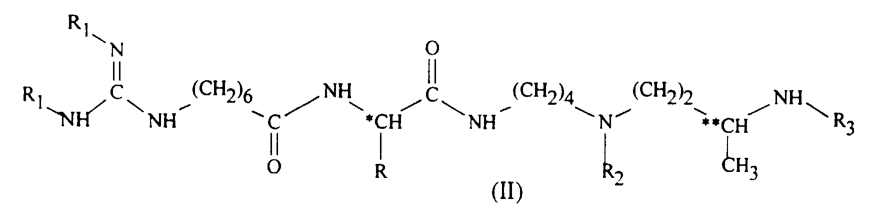 dans laquelle :
- R représente un atome d'hydrogène, un groupe OCH₃, un groupe OH, un groupe CH₂OH, un groupe OR' ou un groupe CH₂OR',
- R' représente un groupe protecteur de la fonction hydroxyle,
- R₁, R₂, R₃, identiques ou différents, représentent chacun un groupe protecteur de la fonction amine,
- *C représente, lorsque R n'est pas l'atome d'hydrogène, un atome de carbone asymétrique, de configuration (R,S), (R) ou (S),
- **C représente un atome de carbone asymétrique de configuration (R,S) ou (R),
selon un ou plusieurs traitements réactionnels connus de l'homme de l'art, pour obtenir le remplacement de tous les groupes protecteurs R₁, R₂, R₃ et R' par un atome d'hydrogène.

Selon l'invention, on préconise également un procédé de préparation des composés de formule (I) dans laquelle *C est de configuration déterminée (R) ou (S) et R représente OH, et de leurs sels d'addition, ledit procédé étant applicable à la préparation de la 11(S)-15-DSG (isomère S de la 15-déoxyspergualine), et comprenant l'étape consistant à obtenir intermédiairement le mélange de diastéréoisomères de formule : 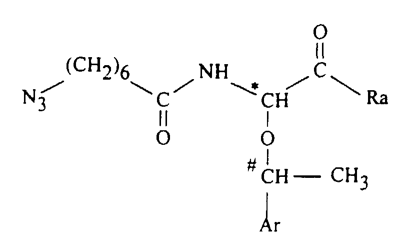 où :
- Ar représente un substituant aromatique, notamment un groupe naphtalényle (i.e. naphtyle) et de préférence le groupe 2-naphtalényle,
- Ra représente un groupe alcoxy en C₁-C₃ ou un groupe -HN-(CH₂)4-N(R₂)-(CH₂)₂-CH(CH₃)-NH(R₃),
- R₁, R₂, et R₃, identiques ou différents, représentent chacun un groupe amino-protecteur, notamment du type benzyloxycarbonyle,
- *C représente un atome de carbone de configuration indéterminée (R,S),
- ^{#}C représente un atome de carbone de configuration absolue déterminée (R) ou (S),
et séparer les deux isomères à l'aide de méthodes connues de l'homme de l'art, comme par exemple la chromatographie préparative sur gel de silice, pour obtenir séparément les composés de formule : 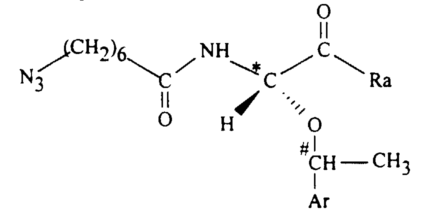 et 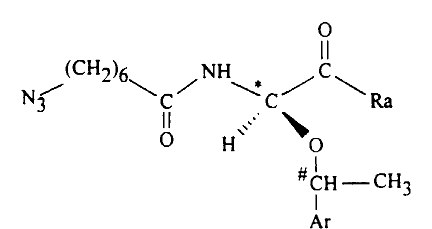 dans lesquelles Ar, Ra et ^{#}C conservent la même signification que ci-dessus.

Les composés de formule ci-dessus, dans laquelle *C est de configuration déterminée (S) ou (R) et Ar, Ra et ^{#}C sont définis comme indiqué ci-dessus, sont nouveaux et constituent l'un des objets de l'invention.

On préconise également l'utilisation d'une substance choisie parmi les composés de formule I et leurs sels d'addition non toxiques pour l'obtention de médicaments destinés à une utilisation en thérapeutique pour le traitement ou la prévention des désordres immunitaires , des maladies inflammatoires hyperréactives, du paludisme, ou à une utilisation en tant que réactif pharmacologique dans le domaine analytique.

### Description détaillée de l'invention

Par "sels d'addition", on entend ici les sels d'addition d'acide obtenus par réaction d'un composé de formule I avec un acide minéral ou un acide organique. Les acides minéraux préférés pour la salification sont les acides chlorhydrique, bromhydrique, phosphorique et sulfurique. Les acides organiques préférés pour la salification sont les acides fumarique, maléique, méthanesulfonique, oxalique, citrique, acétique et trifluoroacétique.

Comme indiqué dans la formule I, les composés selon l'invention comportent un carbone noté *C qui, lorsque R n'est pas un atome d'hydrogène, est un carbone asymétrique, et un second atome de carbone noté **C qui est également un carbone asymétrique. Quand R représente un atome d'hydrogène, la présente invention englobe, parmi les composés de formule I, le racémique où **C est de configuration (R,S), et l'énantiomère où **C est de configuration (R). Quand R n'est pas un atome d'hydrogène, la présente invention englobe, parmi les composés de formule I qui présentent alors deux centres de chiralité, (1) le produit de configuration [(R,S)-*C ; (R,S)-**C] qui est un mélange sensiblement équimoléculaire des quatre stéréoisomères, (2) les produits "hémiracémiques" de configuration [(R,S)-*C ; (R)-**C], [(R)-*C ; (R,S)-**C] et [(S)-*C ; (R,S)-**C], et (3) les diastéréoisomères de configuration [(R)-*C ; (R)-**C] et [(S)-*C ; (R)-**C].

De façon pratique, on préfère les composés de formule I selon l'invention, dans lesquels **C est de configuration (R).

Les composés de formule I peuvent être obtenus selon des méthodes connues en soi, par application de mécanismes réactionnels classiques, notamment des réactions couramment utilisées dans la chimie des peptides, permettant l'obtention de liaisons du type amide.

Le procédé de préparation des composés de formule I que l'on préconise selon l'invention comprend, comme indiqué précédemment, la déprotection d'un composé de formule II.

De façon pratique, les groupes protecteurs R₁, R₂, R₃ que l'on va remplacer par un atome d'hydrogène sont des groupes amino-protecteurs de type connu dans le domaine de la chimie des peptides pour bloquer temporairement les fonctions "amine" non totalement substituées. Parmi les groupes qui conviennent à cet effet, on peut notamment mentionner :
a) les groupes de type oxycarbonyle, comme par exemple les groupes alcoxycarbonyle et benzyloxycarbonyle :
   - Boc : t-butyloxycarbonyle (ou 1,1-diméthyléthoxycarbonyle)
   - Fmoc : 9-fluorénylméthyloxycarbonyle
   - Z : benzyloxycarbonyle (ou phénylméthoxycarbonyle)
   - Z(p-Cl): 4-chlorobenzyloxycarbonyle, ou
   - Z(p-OMe) : 4-méthoxybenzyloxycarbonyle
   d'une part, et
b) les groupes de type benzyle comme par exemple le groupe phénylméthyle (Bn), d'autre part.

Parmi ces groupes amino-protecteurs, les groupes préférés sont les groupes Boc, Z, Fmoc et Bn.

Lorsque dans la formule I, le substituant R comprend une fonction hydroxyle. il peut être nécessaire de la protéger pour effectuer les réactions conduisant aux composés de formule Il Dans ce cas, R peut représenter intermédiairement dans la formule II, un groupe OR' ou CH₂OR' dans lequel R' est un groupe protecteur de la fonction hydroxyle. Parmi les groupes protecteurs de la fonction hydroxyle, on peut citer notamment :
a) les groupes de type benzyle comme par exemple le groupe phénylméthyle (Bn),
b) les groupes de type trialkylsilyle, comme par exemple les groupes triméthvlsilvle et tert.-butyldiméthylsilyle (tBDMS) de formule: 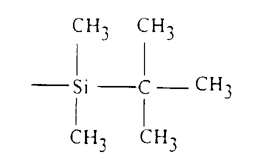
c) le groupe 2-tétrahydropyranyle, et
d) les groupes de type benzyle α-alkylé comme par exemple le groupe 1-(naphtalèn-2-yl)-éthyle, ces groupes présentant l'avantage d'introduire un carbone asymétrique utile pour la séparation des isomères de configuration lorsque cela est nécessaire.

De façon pratique, le procédé de préparation d'un composé de formule I ou de l'un de ses sels d'addition est caractérisé en ce qu'il comprend les étapes consistant à
**(i)** déprotéger un composé de formule II : 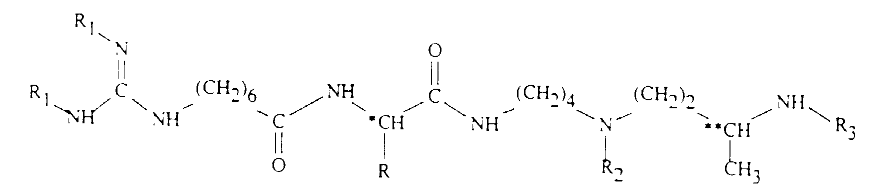 dans laquelle :
   - R représente un atome d'hydrogène, un groupe O-CH₃, un groupe OH, un groupe CH₂OH, un groupe OR' ou un groupe CH₂OR',
   - R' représente un groupe trialkylsilyle ou un groupe de type phénylméthyle, ou un groupe benzyle α-alkylé,
   - R₁, R₂, R₃, identiques ou différents, représentent chacun un groupe amino-protecteur de type alcoxycarbonyle, benzyloxycarbonyle ou benzyle,
   - *C représente, lorsque R n'est pas l'atome d'hydrogène, un atome de carbone asymétrique de configuration (R,S), (R) ou (S),
   - **C représente un atome de carbone asymétrique de configuration (R,S) ou (R),
selon un ou plusieurs traitements suivant la nature des groupes protecteurs, comme par exemple, si l'un au moins des groupes R₁, R₂, R₃ et R' représente un groupe du type alcoxycarbonyle ou trialkylsilyle, par action d'un acide fort tel que notamment l'acide trifluoroacétique, ou, si l'un au moins des groupes R₁, R₂, R₃ ou R' représente un groupe du type benzyle, par hydrogénation catalytique en présence d'un sel de palladium, de charbon palladié ou d'hydroxyde de palladium sur charbon pour obtenir un composé de formule I sous forme de base libre ou de l'un de ses sels d'addition, et, si nécessaire, **(ii)** à partir d'un sel d'addition obtenu selon l'étape (i), obtenir le composé de formule I sous forme de base libre par action d'une base forte, puis obtenir, à partir de ladite base libre, les autres sels d'addition.

Par température ambiante, on entend une température comprise entre 15 et 25 °C. Par température proche ou voisine de la température ambiante, on entend une température se situant entre environ 5 et 40 °C.

Pour la préparation d'un composé de formule II, on peut mettre en oeuvre un procédé choisi parmi les variantes suivantes :
**(a)** la variante A qui comprend les étapes consistant à :
   **(i)** condenser un acide de formule : 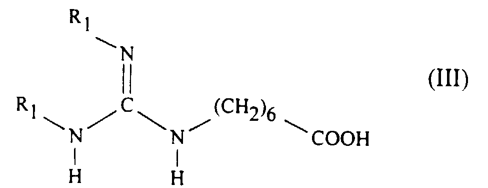 dans laquelle R₁ représente un groupe amino-protecteur, comme par exemple un groupe Boc, avec un dérivé d'aminoacide de formule : 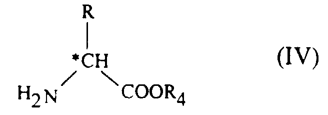 dans laquelle :
      - R représente un atome d'hydrogène ou un groupe CH₂OR',
      - R' représente un groupe protecteur de la fonction hydroxyle, comme par exemple un groupe tBDMS,
      - R₄ représente un groupe alkyle en C₁-C₃, et,
      - *C, lorsque R n'est pas l'atome d'hydrogène, représente un atome de carbone asymétrique de configuration (R,S) (racémique), (R) ou (S),
      par activation de la fonction acide à l'aide d'un agent de couplage de type carbodiimide, notamment le 1,3-dicyclohexylcarbodiimide (DCC), en présence d'un agent nucléophile, notamment le 1-hydroxybenzotriazole (HOBT), dans un solvant organique comme par exemple du dichlorométhane, à une température comprise entre 0 et 40° C, et à raison de 1 mole du composé III pour environ 1 mole du composé IV, pour obtenir un composé de formule : 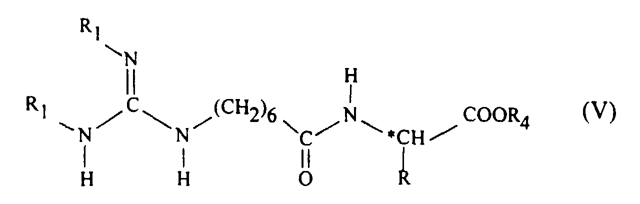 dans laquelle R, R₁, R₄ et *C conservent les mêmes significations que ci-dessus ;
   **(ii)** hydrolyser la fonction ester d'un composé de formule V obtenu,
      - soit selon l'étape (i) ci-dessus, lorsque R représente l'atome d'hydrogène ou un groupe CH₂OR' tel que décrit ci-dessus,
      - soit selon un procédé connu, lorsque R représente un groupe OR', où R' représente un groupe protecteur de la fonction hydroxyle comme par exemple le groupe tBDMS,
      par action d'une solution diluée d'une base, comme par exemple de l'hydroxyde de sodium, en présence d'un solvant miscible à l'eau comme par exemple du 1,2-diméthoxyéthane, à une température voisine de la température ambiante et pendant environ 2 à 30 minutes, pour obtenir un composé de formule : 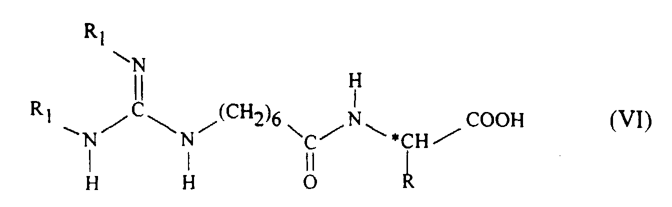 dans laquelle R₁ et *C conservent la même signification que dans le composé de formule V, et R représente un atome d'hydrogène, un groupe CH₂OR' ou un groupe OR', R' représentant un groupe protecteur de la fonction hydroxyle ;
   **(iii)** faire réagir un composé de formule VI, obtenu selon l'étape (ii) ci-dessus, avec un composé de formule : 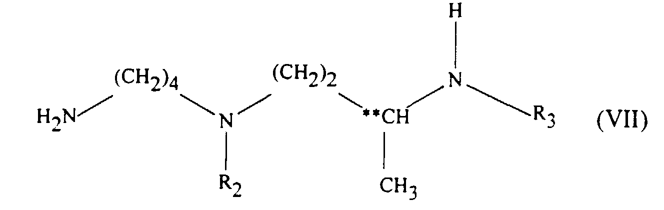 dans laquelle :
      - R₂ et R₃, identiques ou différents, représentent chacun un groupe amino-protecteur, comme par exemple un groupe Boc ou un groupe Bn,
      - **C représente un carbone asymétrique de configuration (R,S) ou (R),
      dans des conditions identiques à celles décrites à l'étape (i) ci-dessus pour obtenir un composé de formule II : 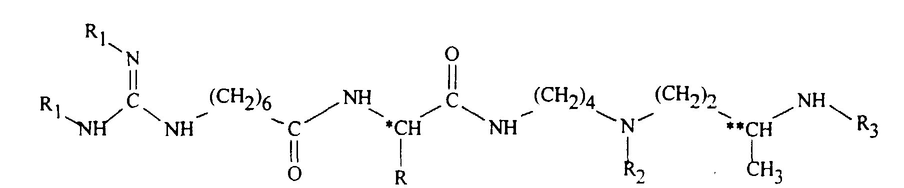 dans laquelle R, R₁, R₂, R_{3,}, *C et **C conservent la même signification que ci-dessus ;
**(b)** la variante B qui comprend les étapes consistant à :
   **(i)** condenser un dérivé d'aminoacide de formule : 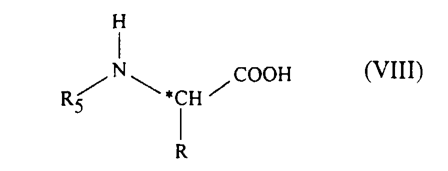 dans laquelle :
      - R représente un atome d'hydrogène ou un groupe CH₂OR' où R' est un groupe protecteur de la fonction hydroxyle,
      - R₅ représente un groupe amino-protecteur, comme par exemple un groupe Fmoc,
      - *C représente, lorsque R n'est pas l'atome d'hydrogène, un atome de carbone asymétrique de configuration (R,S), (R) ou (S),
      avec un composé de formule : 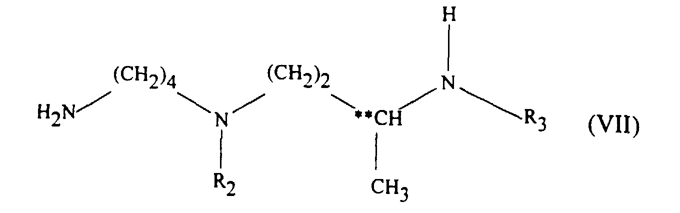 dans laquelle :
      - R₂ et R₃, identiques ou différents, représentent chacun un groupe amino-protecteur, tous deux différents du groupe protecteur R₅ présent dans le composé de formule VIII, et
      - **C représente un carbone asymétrique de configuration (R,S) ou (R),
      dans des conditions analogues à celles du procédé de l'étape (i) de la variante A ci-dessus, pour obtenir un composé de formule : 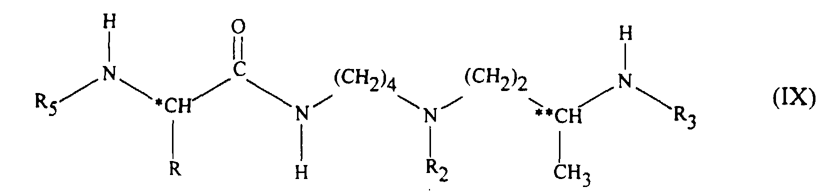 dans laquelle R, R₂, R₃, R₅, *C et **C conservent la même signification que ci-dessus ;
   **(ii)** déprotéger le composé IX, ainsi obtenu, selon un procédé spécifique à la coupure de la liaison N-R₅ comme par exemple, si R₅ est un groupe Fmoc, un traitement avec de la pipéridine, dans un solvant, comme par exemple du dichlorométhane, à température ambiante et pendant environ 1 à 5 heures, pour obtenir un composé de formule : 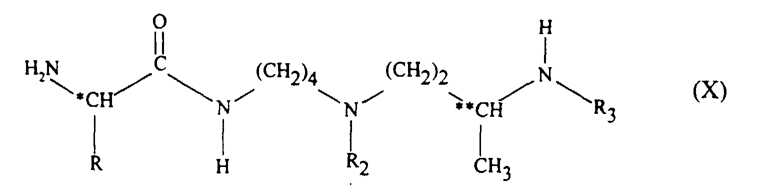 dans laquelle R, R₂ R₃, *C et **C conservent la même signification que ci-dessus ; et,
   **(iii)** condenser le composé amine de formule X, ainsi obtenu, avec un acide de formule : 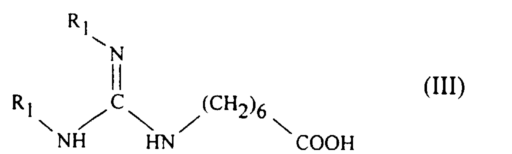 dans laquelle R₁ représente un groupe amino-protecteur, comme par exemple un groupe Boc,
      dans des conditions opératoires analogues à celles décrites à l'étape (i) de la variante A, pour obtenir un composé de formule II : 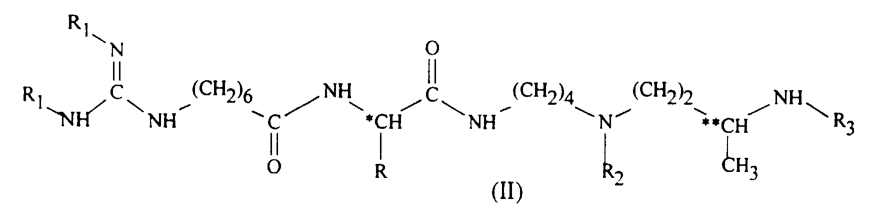 dans laquelle R, R₁, R₂, R₃, *C et **C conservent la même signification que ci-dessus ;
**(c)** la variante C qui comprend les étapes consistant à :
   **(i**_{**a**}**)** faire réagir le 7-azido-heptanamide avec le 2-hydroxy-2-méthoxyacétate de méthyle de formule : 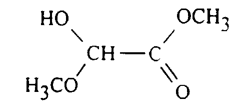 dans un solvant du type hydrocarbure halogéné, notamment le dichlorométhane, en présence d'un agent déshydratant, notamment un tamis moléculaire, à une température comprise entre 25° C et la température de reflux du solvant, pendant 10 à 50 heures, pour obtenir un composé intermédiaire de formule : 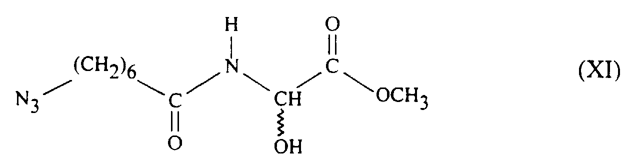
   **(i**_{**b**}**)** faire agir, *in situ,* le composé de formule XI, ainsi obtenu, avec du chlorure de thionyle, à une température d'environ 40° C, pendant 1 à 3 heures, pour obtenir le composé halogéné de formule : 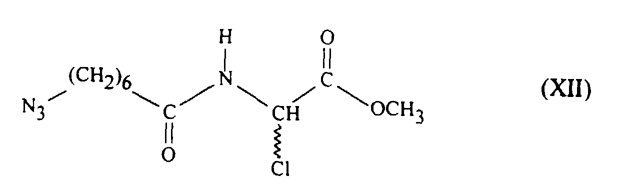
   **(i**_{**c**}**)** faire réagir le composé de formule XII, ainsi obtenu, avec un alcool chiral de configuration déterminée (R) ou (S), de type benzylique, par exemple de formule : 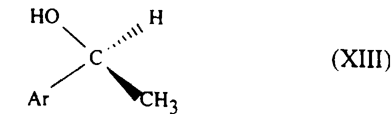 dans laquelle Ar représente un radical aromatique, notamment un groupe naphtalényle (i.e. naphtyle) et de préférence le groupe 2-naphtalényle, dans un solvant du type hydrocarbure halogéné, notamment le dichlorométhane, en présence d'une base, notamment la triéthylamine, à une température comprise entre 10 et 40° C, pendant 5 à 50 heures, pour obtenir le composé de formule : 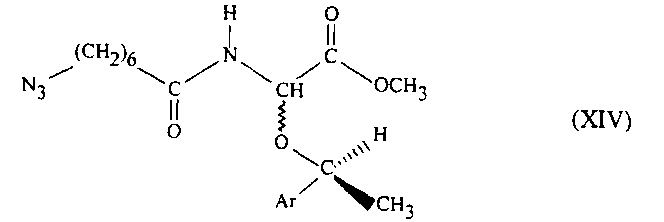 où Ar conserve la même signification que dans le composé de formule XIII ;
   **(ii)** effectuer l'hydrolyse de la fonction ester du composé de formule XIV, ainsi obtenu, par action d'une base en milieu aqueux, notamment une solution aqueuse d'hydroxyde de sodium, dans un solvant du type éther, notamment le 1,2-diméthoxyéthane, à une température voisine de la température ambiante, pour obtenir, après acidification, le composé acide de formule : 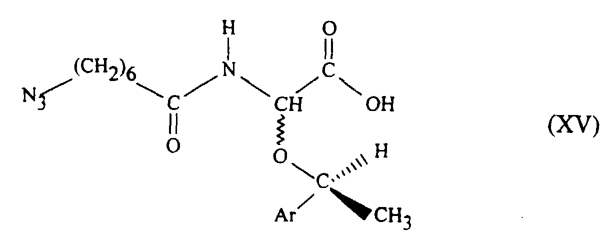 dans laquelle Ar conserve la même signification que ci-dessus ;
   **(iii)** faire réagir le composé de formule XV, ainsi obtenu, avec une amine de formule : 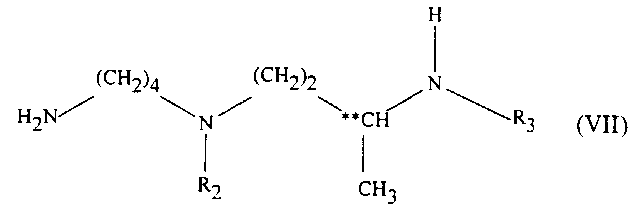 dans laquelle R₂ et R₃ représentent chacun un groupe amino-protecteur sensible à l'hydrogénation notamment un groupe benzyloxycarbonyle (Z), et **C représente un carbone asymétrique de configuration (R,S) ou (R), dans un solvant, notamment un hydrocarbure halogéné, en particulier le dichlorométhane, en présence d'au moins un activateur de couplage de type connu dans la synthèse peptidique, en particulier le 1,3-dicyclohexylcarbodiimide (DCC) et le 1-hydroxybenzotriazole (HOBT), à une température proche de la température ambiante, pendant 10 à 75 heures, pour obtenir le mélange des 2 diastéréoisomères de formule : 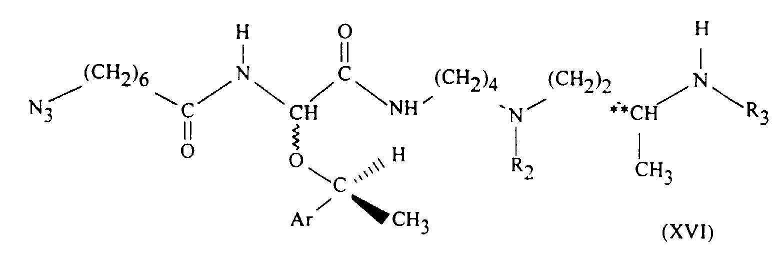 dans laquelle Ar, R₂, R₃ et **C conservent la même signification que ci-dessus ;
   **(iv)** effectuer la séparation des isomères du composé de formule XVI, ainsi obtenu, par exemple au moyen d'une chromatographie sur gel de silice, afin d'obtenir séparément l'un et l'autre des deux composés suivants : 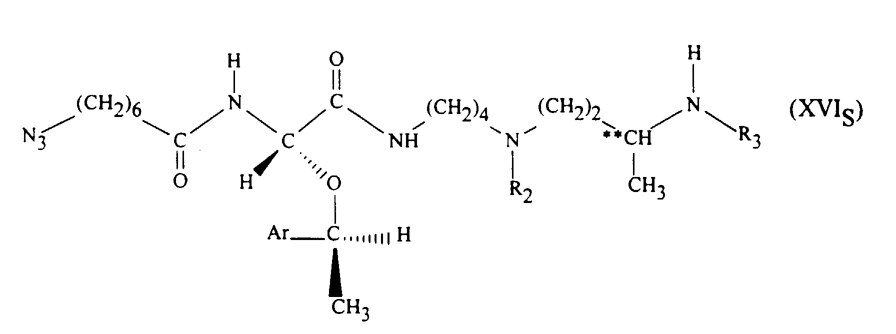 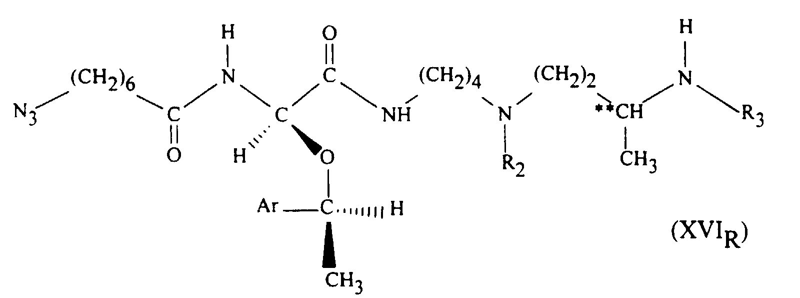 où Ar, R_{1,} R₂, R₃ et **C conservent la même signification que ci-dessus ;
   **(v)** faire réagir le composé de formule XVI_{S}, ainsi obtenu, avec de la triphénylphosphine, en présence d'eau, dans un solvant anhydre, notamment le tétrahydrofuranne, à une température comprise entre 50 et 70° C, pendant 10 à 30 heures, pour obtenir l'amine intermédiaire correspondante que l'on fait réagir *in situ* avec le composé de formule : 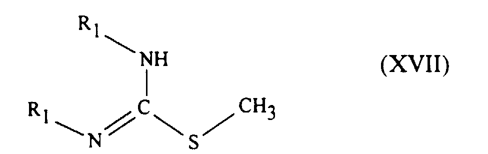 dans laquelle R₁ représente un groupe amino-protecteur, notamment de type benzyloxycarbonyle, pour obtenir après réaction à température proche de la température ambiante, pendant 10 à 48 heures, le composé de formule II : 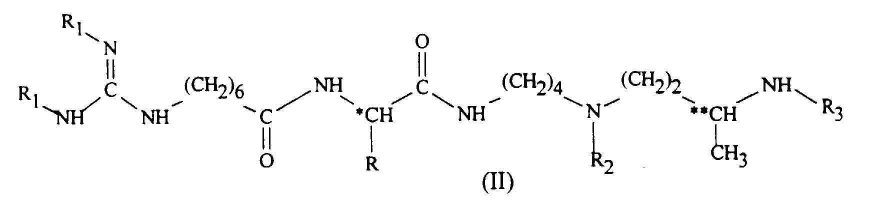 dans laquelle :
      R est le groupe OR',
      R₁, R₂ et R₃ représentent chacun un groupe amino-protecteur, notamment de type benzyloxycarbonyle,
      R' est un groupe de tvpe benzyle α-méthylé de formule : 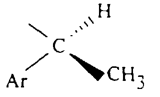
      **C est un carbone asymétrique de configuration (R,S) ou (R), et
      *C est un carbone asymétrique de configuration (S) ;
**(d)** la variante D, qui comprend les étapes consistant à :
   **(i)** faire appel à un composé de formule XIV, obtenu ci-dessus selon l'étape (i_{c}) de la variante C, et effectuer la séparation de ses deux diastéréoisomères, notamment à l'aide d'une chromatographie sur gel de silice, pour obtenir séparément les deux isomères purs de formules : 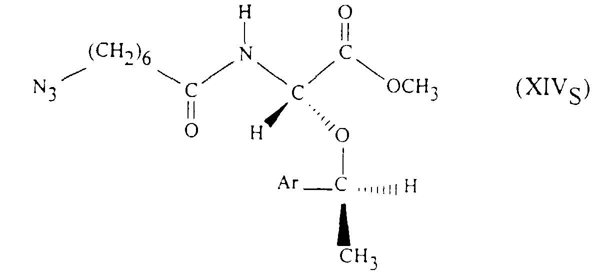 et 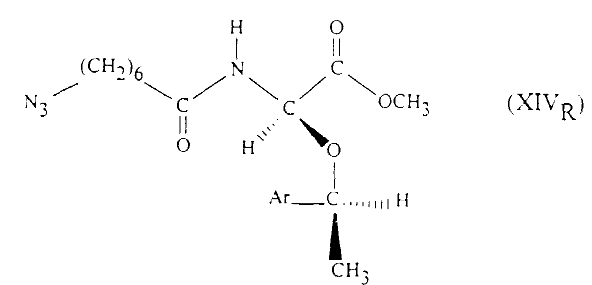 dans lesquelles Ar représente un reste aromatique comme indiqué ci-dessus, notamment un groupe naphtalényle et de préférence le groupe 2-naphtalényle :
   **(ii)** effectuer l'hydrolyse de la fonction ester du composé XIVₛ, ainsi obtenu, dans des conditions identiques à celles décrites à l'étape (ii) de la variante C, pour obtenir l'acide correspondant de formule : 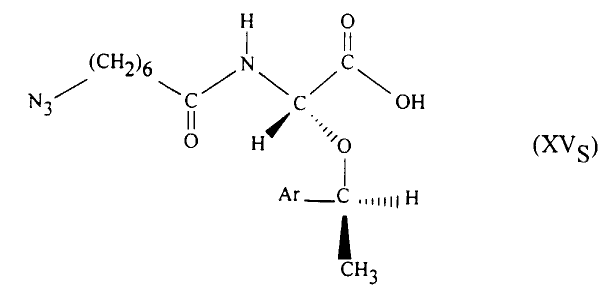 dans laquelle Ar conserve la même signification que ci-dessus ;
   **(iii)** faire réagir le composé XV_{S}, ainsi obtenu, avec une amine de formule VII: 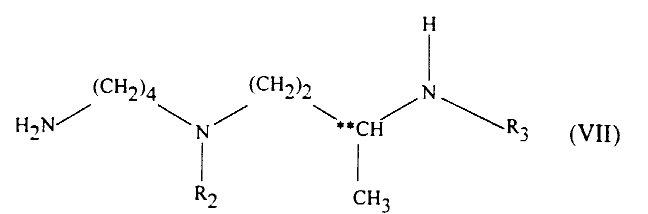 dans laquelle R₂ et R₃ représentent un groupe amino-protecteur sensible à l'hydrogénation, comme par exemple un groupe benzyloxycarbonyle (Z) et **C représente un carbone de configuration (R,S) ou (R),
      dans des conditions identiques à celles décrites à l'étape (iii) de la variante C, pour obtenir le composé de formule : 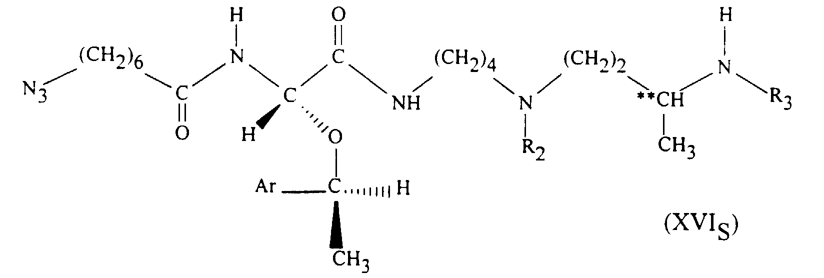 où Ar, R₂, R₃ et **C conservent la même signification que ci-dessus, puis
   **(iv)** traiter le composé de formule XVI_{S}, ainsi obtenu, de façon analogue à l'étape (v) du procédé selon la variante C pour obtenir le composé de formule Il avec les mêmes caractéristiques que dans le cas de ladite variante C.

Le procédé ci-dessus a été décrit en utilisant un alcool chiral de formule XIII dont la configuration est (S) : il est bien entendu que ce procédé s'applique de façon analogue avec un alcool chiral de configuration (R).

Concernant la structure de cet alcool, on entend par "alcool de type benzylique" un alcool dont la fonction hydroxyle est portée par le premier carbone d'un substituant du noyau aromatique : ainsi, par exemple, un composé tel que le 1-indanol [utilisé dans ce cas sous l'une de ses formes (R) ou (S)] est considéré comme étant un alcool de type benzylique.

Les variantes C et D du procédé permettent toutes deux d'obtenir les composés de formule I dans laquelle le carbone *C porteur de la fonction hydroxyle est de configuration déterminée (R) ou (S), et le carbone **C porteur de la fonction amine primaire est de configuration (R,S) ou (R).

Ces deux variantes trouvent également leur utilité dans la préparation de tous les dérivés des acides de formule XV_{S} ou XV_{R} et plus particulièrement, les isomères de configuration déterminée (S) et (R) de la 15-déoxyspergualine [i.e. 11-(S)-15-DSG et 11-(R)-15-DSG] de formules : 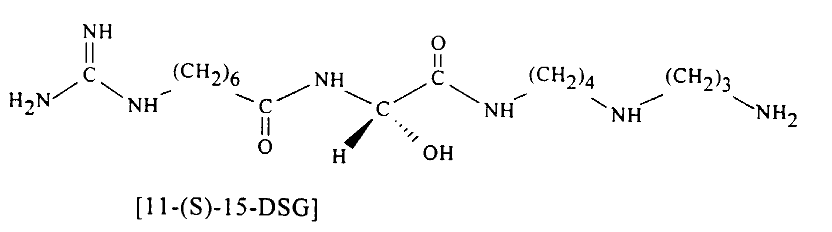 et 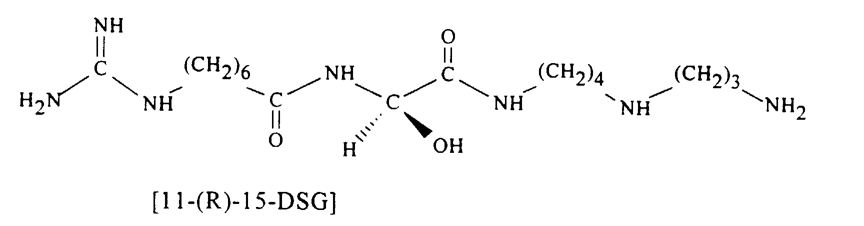 et leurs sels d'addition.

En effet, en utilisant l'une ou l'autre des variantes C et D décrites ci-dessus et en remplaçant l'amine de formule VII par un dérivé analogue de la spermidine de formule NH₂-(CH₂)₄-N(R₂)-(CH₂)₃-NH(R₃), on obtient les composés intermédiaires de structure : 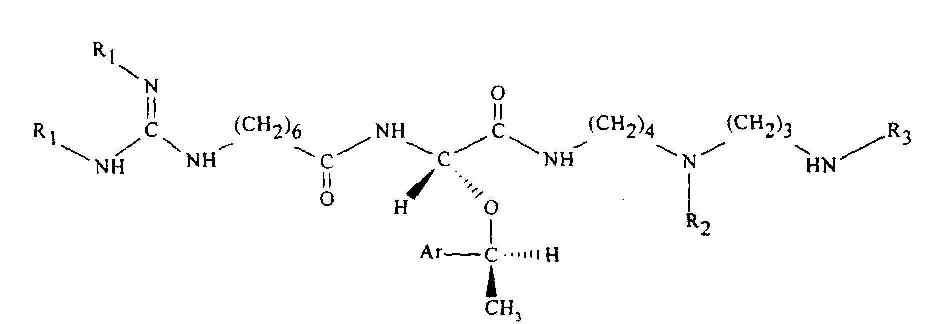 intermédiaire de configuration (S), et 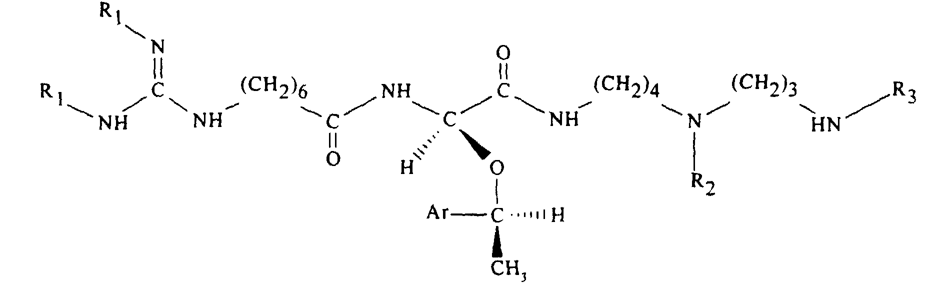 intermédiaire de configuration (R),
de structure apparentée aux composés de formule II, (où R₁, R₂, et R₃ représentent chacun un groupe amino-protecteur, notamment du type benzyloxycarbonyle, et Ar est défini comme indiqué ci-dessus), puis les composés 11-(R)-15-DSG et 11-(S)-15-DSG, avec une excellente pureté optique.

Pour information et de façon pratique, les mécanismes réactionnels principaux de la synthèse de l'isomère 11-(S)-15-DSG selon la variante C et respectivement la variante D sont illustrés ci-après par le schéma 1 et respectivement le schéma 2.

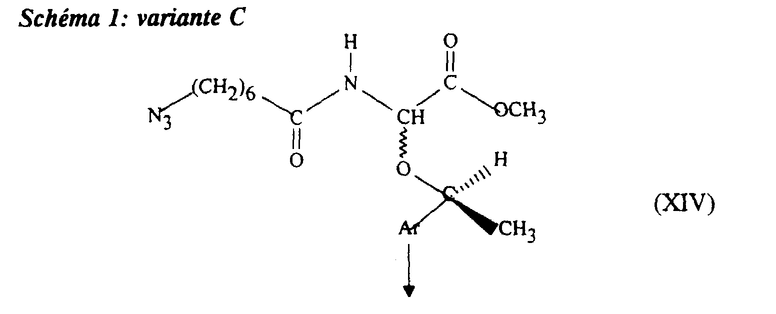

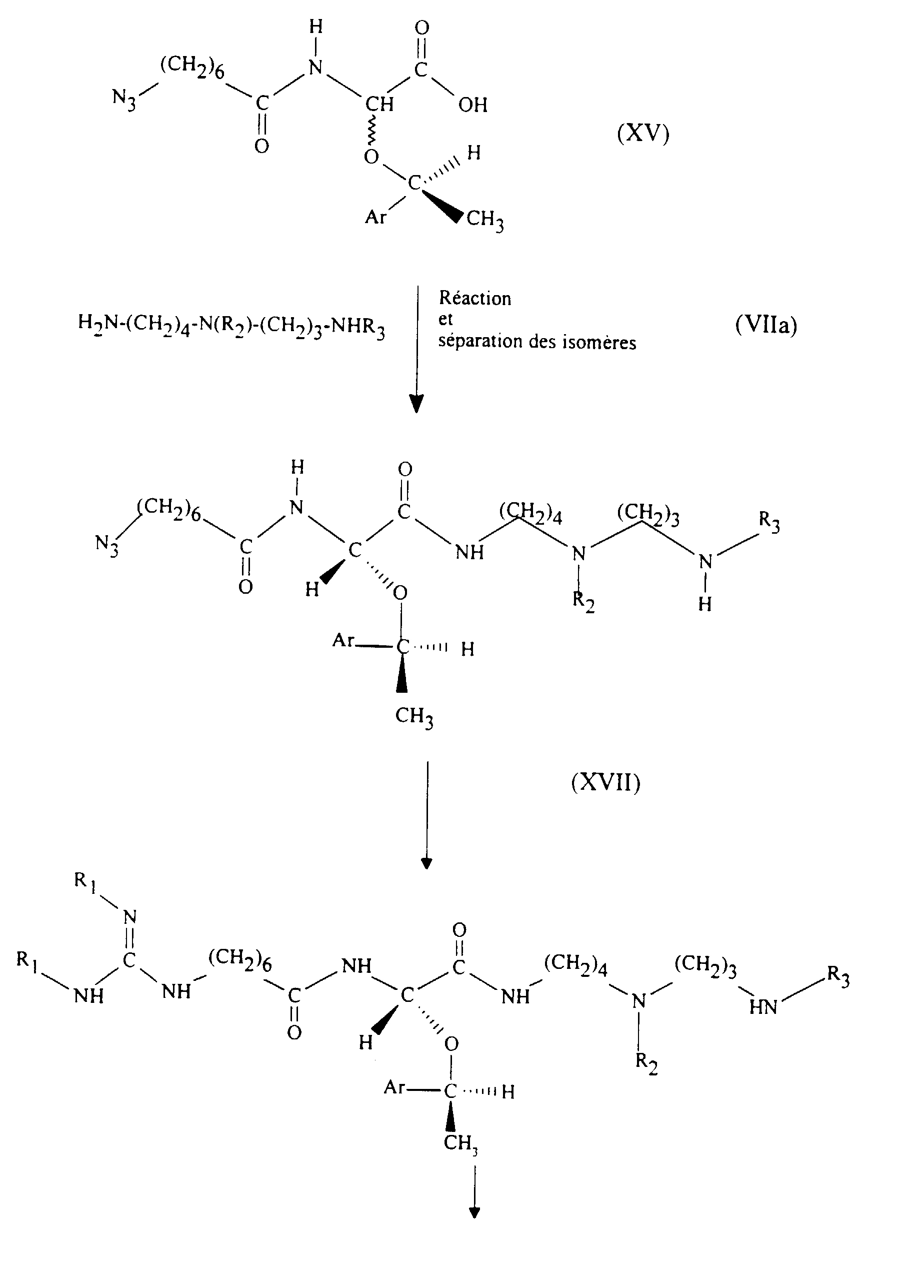

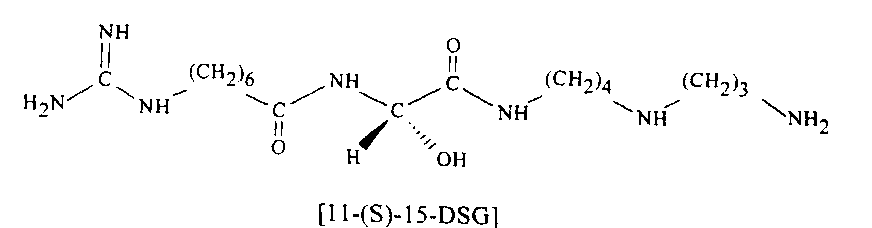

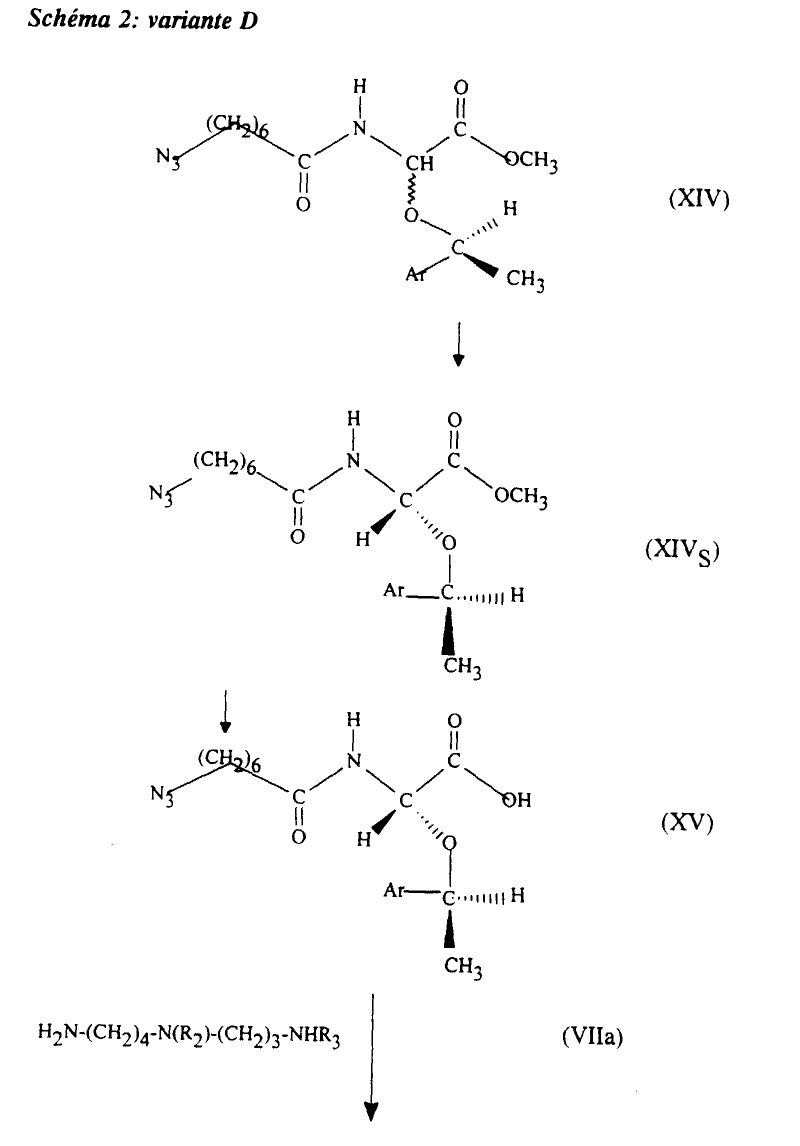

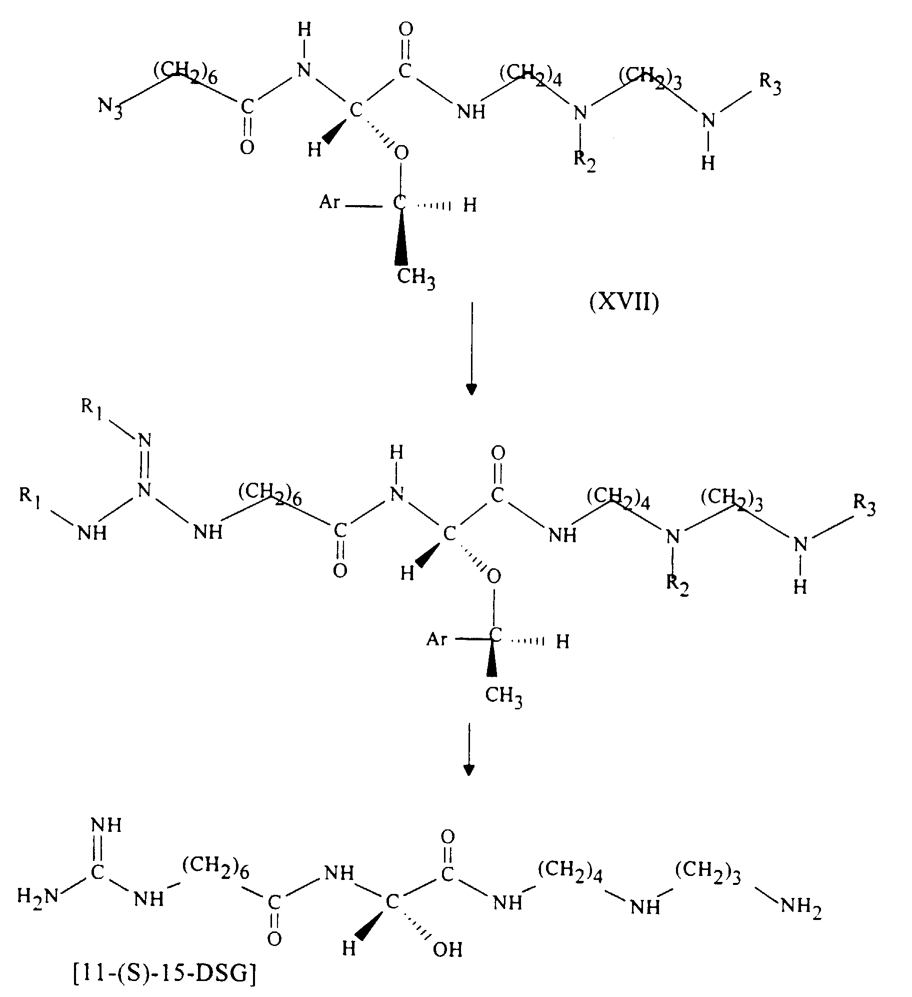

L'acide de formule III dans laquelle R₁ représente le groupe 1,1-diméthyléthoxycarbonyle (Boc) est préparé par réaction du 7-amino-heptanol avec le N,N'-bis(Boc)-S-méthylisothiourée, puis oxydation de l'alcool ainsi obtenu par du dichromate de pyridinium dans le diméthylformamide.

Les composés de formule VII dans laquelle R₂ et R₃ sont identiques et représentent chacun le groupe Boc, peuvent être obtenus au départ d'un composé de formule : 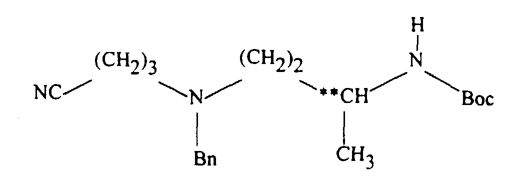 dans laquelle :
- Bn est le groupe phénylméthyle,
- Boc est le groupe 1,1-diméthyléthoxycarbonyle,
- **C est un atome de carbone asymétrique de configuration (R,S) ou (R),
par hydrogénation catalytique en présence de charbon palladié, ce qui permet de remplacer le groupe Bn par un atome d'hydrogène, puis action du dicarbonate de di(tert.-butyle) ce qui conduit au composé de formule : 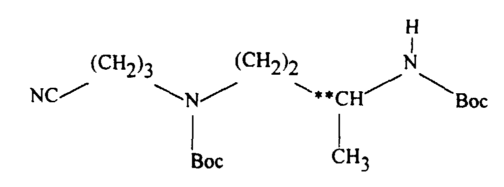 et enfin, hydrogénation catalytique du groupe cyano, en présence de nickel de Raney, ce qui permet d'obtenir le composé de formule VII attendu.

Les composés de formule VII dans laquelle R₂ et R₃ sont identiques et représentent chacun un groupe amino-protecteur phénylméthoxycarbonyle (appelé communément Z) peuvent être obtenus au départ d'un composé de formule :

H₂N-(CH₂)₄-N(Bn)-(CH₂)₂-**CH(CH₃)-NH-Boc

en protégeant l'amine libre au moyen d'un groupe amino-protecteur sensible au milieu alcalin (et stable en milieu acide) comme par exemple un groupe trifluoroacétyle, puis libération des deux autres fonctions amine par action respectivement d'un acide puis de l'hydrogène, protection de ces groupes amine libres au moyen du groupe phénylméthoxycarbonyle et enfin action d'une base pour obtenir le remplacement du groupe amino-protecteur sensible au milieu alcalin par un atome d'hydrogène.

Les composés de formule VII, dans lesquels R₂ et R₃ représentent un groupe Boc ou un groupe Z, sont nouveaux et constituent l'un des objets de l'invention. Ils interviennent en tant qu'intermédiaires de synthèse pour l'obtention des composés de formule I selon l'invention.

L'invention sera mieux comprise à la lecture des exemples qui suivent et des résultats d'essais pharmacologiques obtenus avec les composés selon l'invention, comparativement aux résultats obtenus avec des produits connus de l'art antérieur. La nomenclature utilisée dans les exemples est celle préconisée par les Chemical Abstracts, ainsi un ester du type "...-oate de t-butyle" sera écrit sous la forme "acide ...-oique, 1,1-diméthyléthyl ester".

Dans la partie expérimentale, les préparations sont relatives aux intermédiaires et les exemples sont relatifs aux produits selon l'invention.

Lorsque les composés comportent dans leur structure un carbone asymétrique, l'absence d'indication particulière ou l'indication (R,S) signifie qu'il s'agit d'un mélange sensiblement équimoléculaire des deux énantiomères (i.e. composé "racémique"). Lorsque ces mêmes composés sont dénommés avec un signe (R) ou (S) à la suite immédiate de l'identification de la position d'un substituant, cela signifie que le carbone porteur de ce substituant est de configuration (R) ou (S), conformément aux règles de Cahn, Ingold et Prelog.

Lorsque les composés comportent dans leur structure deux centres d'asymétrie, l'absence d'indication particulière ou la présence du signe (R,S) à la suite immédiate des identifications de position des substituants signifie qu'il s'agit du mélange des quatre stéréoisomères. Lorsque ces mêmes composés sont dénommés avec un signe (R) ou (S) à la suite immédiate de l'identification de position d'un substituant, cela signifie que le carbone porteur de ce substituant est de configuration déterminée (R) ou (S) : si l'un seulement des centres d'asymétrie est dénommé avec un signe de chiralité, le produit décrit sera un mélange sensiblement équimoléculaire de deux diastéréoisomères. Si les deux centres d'asymétrie sont dénommés avec un signe de chiralité, le produit décrit est un stéréoisomère pur.

Les caractéristiques spectrales des signaux de résonance magnétique nucléaire (RMN) sont données pour le proton (¹H) ou pour l'isotope 13 du carbone (¹³C) : on indique le déplacement chimique par rapport au signal du tétraméthylsilane et, entre parenthèses, la forme du signal (s pour singulet, d pour doublet, t pour triplet, q pour quadruplet, m pour multiplet, sl pour signal large) et le nombre de protons concernés par le signal. A titre indicatif, les spectres RMN ¹H ont été réalisés à 300 MHz.

### PREPARATION I

### Acide [(7-hydroxy-heptyl)carbonimidoyl]bis(carbamique), bis(1,1-diméthyléthyl) ester.

On dissout 4,57 g (35.10⁻³ mole) de 7-amino-heptanol et 10,15 g (35.10⁻³ mole) d'acide [[[(1,1-diméthyléthoxy)-carbonyl]amino](méthylthio)méthylène]carbamique, 1,1-diméthyléthyl ester, dans 400 ml de tétrahydrofuranne et on agite pendant 15 heures à température ambiante. On concentre le mélange réactionnel sous pression réduite et on purifie par chromatographie sur gel de silice en éluant avec un mélange méthylcyclohexane/acétate d'éthyle (7/3 v/v). On obtient ainsi 12,6 g du produit attendu sous la forme d'une huile jaune (Rendement = 96 %).
RMN ¹H (CDCl₃) : 1,25-1,6 (m,28H) ; 3,35-3,45 (q,2H) ; 3,63 (q,2H) ; 8,25 (t,1H) ; 11,5 (s,1H).

### PREPARATION II

### Acide [(6-carboxy-hexyl)carbonimidoyl]bis(carbamique), bis(1,1-diméthyléthyl) ester.

On dissout 12,6 g (33,7.10⁻³ mole) du composé obtenu selon la préparation I dans 100 ml de diméthylformamide et on ajoute 25,2 g (67.10⁻³ mole) de dichromate de pyridinium. On maintient le milieu réactionnel sous agitation pendant 2 heures à température ambiante puis on hydrolyse sur 1 1 d'eau. On extrait 3 fois avec de l'éther diéthylique et on lave les phases organiques rassemblées par une solution de sulfate de cuivre, puis à l'eau. La phase organique résultante est ensuite séchée sur sulfate de magnésium puis concentrée sous pression réduite. On obtient ainsi 12 g du produit attendu sous la forme d'une huile (Rendement = 91,6 %).
RMN ¹H (CDCl₃) : 1,25-1,70 (m,26H) ; 2,35 (t,2H) ; 3,40 (q,2H) ; 8,30 (t, 1H) ; 11,5 (sl,1H).

### PREPARATION III

### Acide 3-[[(1,1-diméthyléthoxy)carbonyl]amino]-11-axo-2,4,12-triazatétradéc-2-ènedioïque, 1-(1,1-diméthyléthyl) 14-éthyl ester.

On prépare une solution de 5,28 g (13,64.10⁻³ mole) du composé obtenu selon la préparation II dans 80 ml de dichlorométhane. On refroidit la solution à 0° C puis on ajoute 1,84 g (13,64.10⁻³ mole) d'hydrate de 1-hydroxybenzotriazole (HOBT), 5,63 g (27,28.10⁻³ mole) de N,N'-dicyclohexylcarbodiimide (DCC) puis une solution de 2,1 g (15.10⁻³ mole) de chlorohydrate de glycinate d'éthyle et 1,51 g (15.10⁻³ mole) de triéthylamine dans 20 ml de dichlorométhane. Le mélange réactionnel est maintenu sous agitation pendant 48 heures à température ambiante puis on concentre sous pression réduite. Le résidu est ensuite purifié par chromatographie sur gel de silice en éluant avec un mélange méthylcyclohexane/acétate d'éthyle (6/4 v/v). On obtient ainsi 4,41 g du produit attendu sous forme d'huile incolore (Rendement = 68,6 %).
RMN ¹H (CDCl₃) : 1,25 (t,3H) 1,3-1,8 (m,26H) ; 2,25 (t,2H) ; 3,4 (t,2H) ; 4,0 (d,2H) ; 4,2 (q,2H) ; 6,0 (sl,1H) ; 8,3 (t,1H) ; 11,5 (s,1H).

### PREPARATION IV

### Acide 3-[[(1,1-diméthyléthoxy)carbonyl]amino]-11-oxo-2,4,12-triazatétradéc-2-ènedioïque, 1-(1,1-diméthyléthyl) ester.

On dissout 4,41 g (9,34.10⁻³ mole) du composé obtenu selon la préparation III dans 15 ml de 1,2-diméthoxyéthane puis on ajoute 15 ml de soude 1N. On agite à température ambiante (avantageusement à 20-25° C) pendant 30 minutes puis on ajoute 100 ml de dichlorométhane et on acidifie doucement, en refroidissant et sous bonne agitation, avec de l'acide chlorhydrique 1N, jusqu'à pH = 1. On décante la phase organique puis on extrait la phase aqueuse deux fois avec 100 ml de dichlorométhane. Les phases organiques rassemblées sont séchées sur sulfate de magnésium puis concentrées sous pression réduite. On obtient ainsi 4,1 g du produit attendu sous forme d'une huile jaune (Rendement = 99 %).
RMN ¹H (CDCl₃) : 1,3-1,8 (m,26H) ; 2,25 (t,2H) ; 3,3-3,35 (m,2H) ; 4,0 (d,2H) ; 6,5 (t,1H) ; 8,45 (sl,1H) ; 11,5 (s,1H).

### PREPARATION V

### Acide 3-[[(1,1-diméthyléthoxy)carbonyl]amino]-23-(R)-méthyl-20-phénylméthyl-11,14-dioxo-2,4,12,15,20,24-hexaazapentacos-2-ènedioïque, bis(1,1-diméthyléthyl) ester.

On dissout 4,1 g (9,23.10⁻³ mole) du produit obtenu selon la préparation IV, dans 100 ml de dichlorométhane, puis on ajoute 1,35 g (10.10⁻³ mole) de HOBT et 4,13 g (20.10⁻³ mole) de DCC et on agite à 0° C pendant 30 minutes. On ajoute ensuite 3,3 g (9,45.10⁻³ mole) d'acide [3-[(4-aminobutyl)(phénylméthyl)amino]-1(R)-méthylpropyl]carbamique, 1,1-diméthyléthyl ester, puis on maintient sous agitation pendant 24 heures à température ambiante. Après concentration du milieu réactionnel sous pression réduite, le résidu est purifié par chromatographie sur gel de silice en éluant avec un mélange acétate d'éthyle/éthanol (9/1 v/v). On obtient ainsi, 5,9 g du produit attendu sous forme de solide blanc amorphe (Rendement = 82,5 %).
[α]_{D}^{24,5} = + 0,44 ° (c = 0,45 ; CHCl₃)
RMN ¹H (DMSO d₆): 0,95 (d,3H) ; 1,2-1,7 (m,41H) ; 2,1 (t,2H) 2,3-2,45 (m,4H) ; 3,0-3,1 (m,2H) 3,2-3,4 (m,4H) ; 3,4-3,5 (m,1H) ; 3,6 (d,2H) ; 6,65 (d,1H) ; 7,25-7,35 (m,5H) ; 7,7 (t,1H) ; 7,95 (t,1H) ; 8,3 (t,1H) ; 11,5 (s,1H).

### PREPARATION VI

### Acide 3-[[(1,1-diméthyléthoxy)carbonyl]amino]-23-(R)-méthyl-11,14-dioxo-2,4,12,15,20,24-hexaazapentacos-2-ènedioïque, bis(1,1-diméthyléthyl) ester.

On dissout 5,9 g (7,61.10⁻³ mole) du composé obtenu selon la préparation V dans 120 ml d'éthanol pur, on ajoute 500 mg de charbon palladié à 5 % et on agite sous une atmosphère d'hydrogène pendant 8 heures, à température ambiante. Le catalyseur est ensuite éliminé par filtration puis le filtrat est concentré sous pression réduite. On obtient ainsi 4,94 g du produit attendu sous forme d'un solide amorphe (Rendement = 95 %).
[α]_{D}²⁴ = - 4,8 ° (c = 1,00 ; CHCl₃)
RMN ¹H (DMSO d₆): 1,05 (d,3H) ; 1,2-1,8 (m,41H) ; 2,17 (t,2H) ; 2,7-2,85 (m,4H) ; 3,0-3,15 (m,2H) ; 3,25-3,4 (m,3H) ; 3,45-3,55 (m,1H) ; 3,6 (d,2H) ; 6,35 (d,1H) ; 7,85 (t,1H) ; 8,0 (t,1H) ; 8,3 (t,1H) ; 11.5 (sl,1H).

### EXEMPLE 1

### N-[2-[[4-[(3-(R)-aminobutyl)amino]butyl]amino]-2-oxoéthyl]-7-[(aminoiminométhyl)amino]-heptanamide, tris(trifluoroacétate).

On prépare un mélange de 4 g (5.83.10⁻³ mole) du composé obtenu selon la préparation VI, dans 25 ml de dichlorométhane et 25 ml d'acide trifluoroacétique et on maintient sous agitation à température ambiante pendant 5 heures. Le mélange réactionnel est ensuite concentré sous pression réduite et le résidu d'évaporation est purifié par chromatographie sous moyenne pression (MPLC), en utilisant un gel de silice greffé [de type RP18 (granulométrie 5 à 20 µm)]. L'éluant est un mélange acétonitrile/eau/acide trifluoroacétique (0,8/8/1,2 v/v). Les fractions pures sont lyophilisées et le solide obtenu est redissous dans 100 ml d'eau. La solution obtenue est lavée trois fois avec de l'acétate d'éthyle puis lyophilisée. On obtient ainsi 3,0 g du produit attendu sous forme d'un solide amorphe blanc (Rendement = 70 %).
[α]_{D}²³ = + 1,3° (c = 1,00 ; CH₃OH)
RMN ¹H (DMSO d₆): 1,18 (d,3H) ; 1,2-1,6 (m,12H) ; 1,65-1,85 (m,1H) ; 1,85-2,0 (m,1H) ; 2,10 (t,2H) ; 2,85-3,1 (m,8H) ; 3,2-3,4 (m,1H) ; 3,65 (d,2H) ; 6,9-8,5 (m,12H).
RMN ¹³C (D₂O/Dioxane H₈) : 18,02 ; 23,68 ; 25,81 ; 26,31 ; 26,37 ; 28,51 ; 28,61 ; 31,23 ; 36,19 ; 39,22 ; 41,89 ; 43,44 ; 44,61 ; 46,10 ; 48,16 ; 158,20 ; 172,37 ; 178,71.

### EXEMPLE 2

### N-[2-[[4-[(3-(R,S)-aminobutyl)amino]butyl]amino]-2-oxoéthyl]-7-[(amino-iminométhyl)amino]heptanamide, tris(trifluoroacétate).

En opérant de façon analogue au schéma de synthèse mis en oeuvre pour l'exemple 1, mais en utilisant l'acide [3-[(4-aminobutyl)phénylméthyl)amino]-1-(R,S)-méthylpropyl]carbamique, 1,1-diméthyléthyl ester, on obtient le produit attendu sous forme de solide blanc amorphe.
RMN ¹H (DMSO d₆): 1,18 (d,3H) ; 1,2-1.35 (m,4H) ; 1,4-1,60 (m,8H) ; 1,65-1,85 (m,1H) ; 1,85-2,0 (m,1H) ; 2,10 (t,2H) ; 2,8-3,2 (m,8H) ; 3,2-3,4 (m,1H) ; 3,65 (d,2H) ; 6,8-8,7 (m,12H).
RMN ¹³C (D₂O + Dioxanne H₈) : 18,01; 23,68 ; 25,81 ; 26,31 ; 26,37 ; 28,51 ; 28,60 ; 31,23 ; 36,18 ; 39,21 ; 41,88 ; 43,44 ; 44,61 ; 46,03 ; 48,16 ; 157,54 ; 172,38 ; 178,72.

### PREPARATION VII

### Acide [3-[(3-cyanopropyl)amino]-1-(R)-méthylpropyl]carbamique, 1,1-diméthyléthyl ester.

On dissout 21 g (60,8.10⁻³ mole) d'acide [3-[(3-cyanopropyl)(phénylméthyl)amino]-1-(R)-méthylpropyl]carbamique, 1,1-diméthyléthyl ester dans 400 ml d'éthanol puis on ajoute 0,25 ml d'acide chlorhydrique 10 M, puis 1,2 g de charbon palladié à 5 %. Le mélange est agité sous atmosphère d'hydrogène à température ambiante, à pression atmosphérique. Après 24 heures de réaction, le catalyseur est éliminé par filtration, puis le filtrat est concentré sous pression réduite. Le résidu est purifié par chromatographie sur gel de silice en éluant avec un mélange acétate d'éthyle/éthanollanunoniaque (6/3/0,1 v/v). On obtient ainsi 6 g du produit attendu sous forme d'huile (Rendement = 34 %).
RMN ¹H (CDCl₃) : 1,15 (d,3H) ; 1,3-1,9 (m,13H) ; 2,4-2,7 (m,6H) ; 3,4-3,85 (m, 1H) ; 4.7-4.9 (sl, 1H).

### PREPARATION VIII

### Acide [3-[(3-cyanopropyl)[(1,1-diméthyléthoxy)carbonyl]amino]-1-(R)-méthylpropyl]carbamique, 1,1-diméthyléthyl ester.

On prépare une solution de 5,82 g (22,92.10⁻³ mole) du composé obtenu selon la préparation VII dans 100 ml de tétrahydrofuranne et on ajoute 3.45 g (34.23.10⁻³ mole) de triéthylamine puis 5.97 g (27,38.10⁻³ mole) de dicarbonate de di(tert.-butyle) [structure chimique : O[CO₂C(CH₃)₃]₂]. Le mélange réactionnel est maintenu sous agitation pendant 15 h, à température ambiante. Après concentration du milieu réactionnel sous pression réduite, le produit brut est purifié par chromatographie sur gel de silice en éluant avec un mélange méthylcyclohexane/acétate d'éthyle (9/1 puis 8/2 v/v). On obtient ainsi 5,6 g du produit attendu sous forme d'huile (Rendement = 68 %).
RMN ¹H (CDCl₃) : 1,15 (d,3H) ; 1,4-1,6 (m,20H) ; 1,8-1,95 (m,2H) ; 2,35 (t,2H) ; 3,1-3,4 (m,4H) ; 3.55-3,70 (m,1H) ; 4.25-4.55 (sl,1H).

### PREPARATION IX

### Acide [3-[(4-aminobutyl)[(1,1-diméthyléthoxy)carbonyl]amino]-1(R)-méthylpropyl]carhamique, 1,1-diméthyléthyl ester.

On dissout 5.5 g (15,5.10⁻³ mole) du composé obtenu selon la préparation VIII dans 100 ml de méthanol. on ajoute 400 mg de nickel de Raney et on agite le mélange sous atmosphère d'hydrogène. à température ambiante, sous une pression de 3.10⁵ Pa pendant 24 h. Le mélange réactionnel est ensuite titré pour éliminer le catalyseur puis concentré sous pression réduite. Le résidu est purifié par chromatographie sur gel de silice en éluant par un mélange méthylcyclohexane/acétate d'éthyle (6/4 v/v) puis acétate d'éthyle/éthanol/ammoniaque (6/3/0,2 v/v). On obtient ainsi 3,96 g du produit attendu sous forme d'huile (Rendement = 71 %).
[α]_{D}²¹ = + 41,3 ° (c = 3 ; CHCl₃)RMN ¹H (CDCl₃) ; 1.15 (d,3H) ; 1,4-1,7 (m,22H) ; 1,8 (s,4H) ; 2,75 (t,2H) ; 3.05-3.35 (m,4H) ; 3,55-3,7 (m,1H) ; 4,35-4,6 (sl,1H).

### PREPARATION X

### Acide 3-[[(1,1-diméthyléthoxy)carbonyl]amino]-13-(carboxy)-15,15,16,16-tétraméthyl-11-oxo-14-oxa-2,4,12-triaza-15-silaheptadéc-2-énoïque, 1,1-diméthyléthyl ester.

On dissout 1,73 g (2,94.10⁻³ mole) d'acide 3-[[(1,1-diméthyléthoxy)carbonyl]amino]-13-(méthoxycarbonyl)-15,15,16,16-tétraméthyl-11-oxo-14-oxa-2,4, 12-triaza-15-silaheptadéc-2-ènoïque, 1,1-diméthyléthylester, dans 4 ml de 1,2-diméthoxyéthane et on ajoute 4 ml d'une solution aqueuse molaire d'hydroxyde de sodium. Le mélange réactionnel est agité pendant 10 minutes à température ambiante puis on ajoute 20 ml d'eau et 20 ml de dichlorométhane, et on acidifie sous bonne agitation jusqu'à pH 2 à l'aide d'acide chlorhydrique 1N. Après séparation de la phase organique, on extrait la phase aqueuse avec 3 fois 25 ml de dichlorométhane. Les phases organiques rassemblées sont séchées sur sulfate de magnésium puis concentrées sous pression réduite. On obtient ainsi 1,68 g du produit attendu sous forme d'huile incolore (Rendement quantitatif).
RMN ¹H (CDCl₃) : 0,1-0,15 (m,6H) ; 0,85-1 (m,9H) ; 1,3-1,8 (m,26H) ; 2,25 (t,2H) ; 3,2-3,45 (m,2H) ; 5,55 (d,1H) ; 7,0-7,1 (sl,1H) ; 8,3-8,7 (m,1H) ; 11-12 (sl,1H).

### PREPARATION XI

### Acide 3-[[(1,1-diméthyléthoxy)carbonyl]amino]-20-[(1,1-diméthyléthoxy)carbonyl]-23-(R)-méthyl-13-[[(1,1-diméthyléthyl)diméthylsilyl]oxy]-11,14-dioxo-2,4,12,15,20,24-hexaazapentacos-2-ènedioïque, bis(1,1-diméthyléthyl) ester.

On dissout 1,68 g (2,92.10⁻³ mole) du produit obtenu selon la préparation X dans 30 ml de dichlorométhane et on refroidit à 0° C. On ajoute 0,4 g (3.10⁻³ mole) de HOBT, puis 1,24 g (6.10⁻³ mole) de DCC. On agite ce mélange pendant 15 minutes puis on ajoute 1,05 g (2,92.10⁻³ mole) du composé obtenu selon la préparation IX et on maintient le milieu réactionnel sous agitation à température ambiante pendant 48 heures. Le solvant est ensuite éliminé sous pression réduite et le résidu est purifié par chromatographie sur gel de silice en éluant avec un mélange acétate d'éthyle/méthylcyclohexane (1/1 v/v) puis avec de l'acétate d'éthyle. On obtient ainsi 0,78 g du produit attendu sous forme d'un solide amorphe (Rendement = 30 %).
[α]_{D}^{19,5} = - 0,1 ° (c = 1 ; CHCl₃)
RMN ¹H (DMSO d₆): 0,079 (s,3H) ; 0,137 (s,3H) ; 0,88 (s,9H) ; 1,05 (d,3H) ; 1,1-1,95 (m,50H) ; 2,14 (t,2H) ; 3-3,4 (m,9H) ; 5,60 (d, 1H) ; 6,7 (sl,1H) ; 7,75-7,85 (sl,1H) ; 8,3 (t,1H) ; 8,62 (d, 1H) ; 11,5 (s,lH).

### EXEMPLE 3

### N-[2-[[4-[(3-R)-aminobutyl)amino]butyl]amino]-1-(R,S)-méthoxy-2-oxoéthyl]-7-[(aminoiminométhyl)amino]heptanamide, tris(trifluoroacétate).

On dissout 31 mg (38,7.10⁻⁶ mole) du composé obtenu selon la préparation XI dans 10 ml d'acide trifluoroacétique et 1 ml de méthanol. On maintient ensuite ce mélange sous agitation pendant 4 heures puis on élimine le solvant sous pression réduite à température ambiante. Le produit brut est ensuite purifié par chromatographie sur silice greffée [de type RP 18 (granulométrie : 5 à 20 µm)], en éluant à l'aide d'un mélange acétonitrile/eau/acide trifluoroacétique (1,5/8/0,5 puis 2/8/0,1 v/v). On obtient ainsi 16 mg du produit attendu sous forme d'un solide blanc amorphe (Rendement = 55 %).
[α]_{D}²⁰ = + 1,2 ° (c = 1,53 ; H₂O)
RMN ¹H (DMSO d₆): 1,18 (d,3H) ; 1,2-1,35 (m,4H) ; 1,35-1,65 (m,8H) ; 1,65-1,85 (m,1H) ; 1,85-2,0 (m,1H) ; 2,1-2,3 (m,2H) ; 2,8-3,2 (m,8H) ; 3,22 (m,4H) ; 5,26 (d,1H) ; 6,8-7,4 (sl,3H) ; 7,55 (t,1H) ; 7,8-8,05 (m,4H) ; 8,15 (t,1H) ; 8,48 (d,1H) ; 8,5-8,65 (m,2H).

### EXEMPLE 4

### 7-[(aminoiminométhyl)amino]-N-[2-[[4-[(3-(R)-aminobutyl)amino]butyl]amino]-1-(R,S)-hydroxy-2-oxoéthyl]-éthyl]heptanamide, tris(trifluoroacétate).

On met en solution 150 mg (0,164.10⁻³ mole) du composé obtenu selon la préparation XI dans 4 ml d'acide trifluoroacétique et on maintient sous agitation à température ambiante pendant 4 heures. Après élimination du solvant sous pression réduite, le produit est purifié par la technique FPLC (Fast Protein Liquid Chromatography) sur un gel de type C.M. SEPHAROSE® FAST FLOW (Pharmacia) en éluant avec de l'eau pure, puis avec une solution de chlorure de sodium dont la concentration augmente progressivement de 0 à 1 M suivant un gradient sensiblement linéaire, présentant un palier de concentration à 0,4 M. Les fractions contenant le produit attendu sont lyophilisées et le solide blanc obtenu est dessalé par chromatographie sur une colonne de SEPHADEX® LH 20 (Pharmacia) en éluant par du méthanol. La purification finale du produit est effectuée par une chromatographie sur gel de silice greffée de type RP 18 "Varian Bond Elut" en éluant avec de l'eau pure, puis par un mélange acétonitrile/eau/acide trifluoroacétique (7/0,5/0,2 v/v). On obtient ainsi 10 mg du produit attendu sous forme d'un solide blanc amorphe.
[α]_{D}²²= + 1,5° (c = 0,45 ; CH₃OH)
RMN 1H (D₂O) : 1,25-1,4 (m,7H) ; 1,45-1,8 (m,8H) ; 1,85-2,05 (m,1H) ; 2,1-2,20 (m,1H) ; 2,25 (t,2H) ; 3,0-3,3 (m,8H) : 3,4-3,55 (m,1H) ; 5,41 (s,1H).
RMN ¹³C (H₂O + Dioxane H₈) : 18,34 ; 23,70 ; 25,6 ; 26,12 ; 26,22 ; 28,38 ; 28,51 ; 31,14 ; 36,33 ; 39,37 ; 41,94 ; 44,66 ; 46,28 : 48,22 ; 72,54 ; 157,25 ; 171,79 ; 178,41.

### PREPARATION XII

### Acide 3-[[(1,1-diméthyléthoxy)carbonyl]amine]-23-(R,S)-méthyl-20-(phénylméthyl)-13-[[(1,1-diméthyléthyl)diméthysilyl]oxy]-11,14-dioxo-2,4,12,15,20, 24-hexaazapentacos-2-ènedioïque, bis(1,1-diméthyléthyl)ester.

En opérant de façon analogue au procédé de la préparation XI, mais en utilisant de l'acide [3-[(4-aminobutyl)(phénylméthyl)amin]-1-(R,S)-méthylpropyl]carbamique, 1,1-diméthyléthyl ester, on obtient le produit attendu sous forme d'huile, avec un rendement de 49 %.
RMN ¹H (CDCl₃) : 0,105 (s,3H) ; 0,21 (s,3H) ; 0,90 (s,9H) ; 1,04 (d,3H) ; 1,3-1,7 (m,41H) ; 2,20 (m,2H) 2,41 (m,2H) 2,55 (m,1H) ; 3,1-3,75 (m,7H) ; 5,3 (sl,1H) ; 5,7 (d,1H) ; 6,4 (sl,1H) ; 6,7 (sl,1H) ; 7,20-7,30 (m,5H) ; 8,3 (sl,1H) ; 11,5 (s,1H).

### PREPARATION XIII

### Acide 3-[[(1,1-diméthyléthoxy)carbonyl]amino]-23-(R,S)-méthyl-13-(R,S)-[[(1,1-diméthyléthyl)dimétbysilyl]oxy]-11,14-dioxo-2,4,12,15, 20,24-hexaazapentacos-2-ènedioïque, bis(1,1-diméthyléthyl)ester.

On dissout 300 mg (0,33.10⁻³ mole) du composé obtenu selon la préparation XII dans 30 ml de méthanol et on ajoute 30 mg d'hydroxyde de palladium. Le mélange est agité sous atmosphère d'hydrogène à pression atmosphérique et à température ambiante pendant 15 min puis le catalyseur est éliminé par filtration. Après concentration, le produit brut est purifié par chromatographie sur gel de silice en éluant avec un mélange acétate d'éthyle/éthanol/ammoniaque (6/3/0,1 v/v) On obtient ainsi 200 mg du produit attendu sous forme d'une huile (Rendement : 74 %)
RMN ¹H (CDCl₃) : 0.011 (s,3H) ; 0.021 (s,3H) ; 0,9 (s,9H) ; 1,13 (d,3H) ; 1,3-1,9 (m,41H) ; 2.20 (m,2H) ; 2.7 (m,4H) ; 3,1-3,6 (m,5H) ; 4,2-4,4 (sl,1H) ; 4,8 (sl,1H) ; 5,7 (d,1H) ; 6.7-6.9 (m,2H) ; 8,3 (Sl,1H) : 11,5 (s, 1H).

### EXEMPLE 5

### N-[2-[[4-[(3-(R,S)-aminobutyl)amino]butyl]amino]-1-(R,S)-hydroxy-2-oxoéthyl]-7-[(aninoiminométhyl)amino]heptanamide, tris(trifluoroacétate).

On agite 200 mg (0,245.10⁻³ mole) du composé obtenu selon la préparation XIII dans 10 ml d'acide trifluoroacétique. à température ambiante pendant 45 minutes. Après évaporation du solvant. le composé est purifié par chromatographie sur gel de silice greffée de type RP 18 5-20N, en éluant avec un mélange acétonitrile/eau/acide trifluoroacétique (2/8/0,1 v/v). Après lyophilisation. on obtient 82 mg du produit attendu sous forme d'un solide blanc (Rendement : 45 %).
RMN ¹H (DMSO) : 1,15 (d,3H) ; 1.2-1.35 (m,4H) ; 1.35-1.55 (m,8H) ; 1,65-1.8 (m,1H) ; 1,85-2,0 (m,1H) ; 2.1-2.2 (t,2H) ; 2,85-3,35 (m,9H) ; 5,4 (d,1H) : 6,5 (sl,1H) ; 6,7-7,4 (sl,3H) ; 7,6 (t,1H) ; 7,8-8.1 (m,5H) ; 8.4-8.7 (m.3H).

### PREPARATION XIV

### Acide 13-(S)-[[(9H-fluorèn-9-yl)-méthexycarbonyl]amine]-3-(R)-méthyl-12-oxo-14-(phénylméthoxy)-6-(phénylméthyl)-2,6,11-triazatétradécanoïque, 1,1-diméthyléthyl ester.

On dissout 1.71 g (4,1.10⁻³ mole) de N-[(9H-fluorèn-9-yl)méthoxycarbonyl]-O-phénylméthyl-(L)-sérine dans 60 ml de dichlorométhane. On refroidit à 0° C et on ajoute 0,55 g (4.10⁻³ mole) de HOBT et 1,54 g (7,5.10⁻³ mole) de DCC en solution dans 20 ml de dichlorométhane. On maintient sous agitation pendant 0,5 heure, puis on ajoute 1,30 g (3,72.10⁻³ mole) d'acide N-[3-[(4-aminobutyl)(phénylméthyl)amino]-1-(R)-méthylpropyl]carbamique, 1,1-diméthyléthyl ester et on laisse le mélange réactionnel sous agitation à température ambiante pendant 16 heures. On élimine le solvant sous pression réduite et on purifie le résidu par chromatographie sur gel de silice en éluant avec un mélange acétate d'éthyle/méthylcyclohexane (1/1 v/v) puis avec de l'acétate d'éthyle pur. On obtient ainsi 2,7 g du produit attendu sous forme d'un solide cristallisé blanc (Rendement = 96 %).
[α]_{D}²³ = + 11° (c = 1,1; CHCl₃)
F = 118° C
RMN ¹H (CDCl₃) : 1,02 (d,3H) ; 1,3-1,8 (m,15H) ; 2,25-2,6 (m,4H) ; 3,15-3.3 (m,2H) ; 3,35-3,75 (m,4H) ; 3,8-3,95 (m,1H) ; 4,15-4,6 (m,6H) ; 5,3 (d,1H) ; 5,6-5,8 (sl,1H) ; 6,35-6,55 (sl,1H) ; 7,15-7,45 (m,14H) ; 7,55 (d,2H) ; 7,75 (d,2H).

### PREPARATION XV

### Acide 13-(S)-amino-3-(R)-méthyl-12-oxo-14-phénylméthoxy-6-phénylméthyl-2,6,11-triazatétradécanoïque, 1,1-diméthyléthyl ester.

On dissout 2,56 g (3,42.10⁻³ mole) du composé obtenu selon la préparation XIV dans 100 ml de dichlorométhane et on ajoute 5 g de pipéridine. On maintient sous agitation pendant 3 heures à température ambiante puis on concentre sous pression réduite. On ajoute 2 fois 10 ml de toluène en fin de concentration pour chasser l'excès de pipéridine. Le résidu est ensuite purifié par chromatographie sur gel de silice en éluant avec un mélange acétate d'éthyle/méthanol (8/2 v/v). On obtient ainsi 1,68 g du produit attendu sous forme d'une huile visqueuse jaune (Rendement = 90 %).
[α]_{D}²² = + 10 ° (c = 1,1; CHCl₃)
RMN ¹H (CDCl₃) : 1,03 (d,3H) ; 1,4-1,7 (m,15H) ; 2,3-2,65 (m,4H) ; 3,15-3,30 (m,2H) ; 3,4-3,8 (m,6H) ; 4,5 (s,2H) ; 5,35-5,55 (sl,1H) ; 7,2-7,5 (m,10H) [(protons amine et amide non détectés)].

### PREPARATION XVI

### Acide 3-[[(1,1-diméthyléthoxy)carbonyl]amino]-13-(S)-[phénylméthoxyméthyl]-23-(R)-méthyl-20-phénylméthyl-11,14-dioxo-2,4,12,15,20,24-hexaazapentacos-2-ènedioïque, bis(1,1-diméthyléthyl) ester.

On mélange 1,46 g (3,79.10⁻³ mole) du composé obtenu à la préparation Il et 60 ml de dichlorométhane puis refroidit à 0° C. On ajoute 0,51 g (3,79.10⁻³ mole) de HOBT et 1,56 g (7,56.10⁻³ mole) de DCC, en solution dans 20 ml de dichlorométhane. Après 30 minutes sous agitation, on ajoute 1,60 g (3.10⁻³ mole) du composé obtenu selon la préparation XV et on maintient sous agitation pendant 16 heures à température ambiante. Le mélange réactionnel est ensuite concentré sous pression réduite et purifié par chromatographie sur gel de silice en éluant avec un mélange acétate d'éthyle/méthylcyclohexane (6/4 v/v) puis acétate d'éthyle pur. On obtient ainsi 2,27 g du produit attendu sous forme d'huile (Rendement = 83 %). [α]_{D}²² = + 4,2 ° (c = 4,7 ; CHCl₃)
RMN ¹H (CDCl₃) : 1,03 (d,3H) ; 1,2-1,8 (m,41H) ; 2,2 (t,2H) ; 2,3-2,65 (m,4H) ; 3,1-3,8 (m,8H) ; 3,85 (dd,1H) ; 4,55 (q,3H) ; 5,3-5,4 (m,1H) ; 6,3-6,6 (m,2H) 7,2-7,4 (m,10H) ; 8,3 (t,1H) ; 11,5 (s,1H).

### PREPARATION XVII

### Acide 3-[[(1,1-diméthyléthoxy)carbonyl]amino]-13-(S)-[phénylméthoxyméthyl]-23-(R)-méthyl-11,14-dioxo-2,4,12,15,20,24-hexaazapentacos-2-ènedioïque, bis(1,1-diméthyléthyl) ester.

On prépare une solution de 1,65 g (1,84.10⁻³ mole) du composé obtenu selon la préparation XVI, dans 100 ml d'éthanol. On ajoute 200 mg de charbon palladié à 10 % et on agite ce mélange réactionnel sous atmosphère d'hydrogène, à pression atmosphérique, pendant 24 heures à température ambiante. Le catalyseur est éliminé par filtration et le filtrat est concentré sous pression réduite. Le résidu est purifié par chromatographie sur gel de silice en éluant avec un mélange acétate d'éthyle/éthanol/ammoniaque (6/3/0,1 v/v). On obtient ainsi 1 g du produit attendu sous forme d'huile visqueuse jaune (Rendement = 67 %).
RMN ¹H (CDCl₃) : 1,15 (d,3H) ; 1,3-1,9 (m,41H) ; 2,2 (t,2H) ; 2,5-2,7 (m,4H) ; 3,2-3,3 (m,2H) ; 3,4 (td,2H) ; 3,5 (t,1H) ; 3,65-3,75 (t,1H) ; 3,85 (m,1H) ; 4,454,65 (m,3H) ; 4,84,9 (sl,1H) ; 6,4-6,5(sl,1H) ; 6,45 (t,1H) ; 7,25-7,4 (m,5H) ; 8,3 (t,1H) ; 11,5 (s,1H).

### PREPARATION XVIII

### Acide 3-[[(1,1-diméthyléthoxy)carbonyl]amino]-13-(S)-(hydroxyméthyl)-23-(R)-méthyl-11,14-dioxo-2,4,12,15,20,24-hexaazapentacos-2-ènedioïque, bis-(1,1-diméthyléthyl) ester.

En opérant de façon analogue à la préparation XVII, au départ du composé obtenu selon la préparation XVII, mais en effectuant l'hydrogénation sur un milieu réactionnel acidifié jusqu'à pH 3,5 avec de l'acide chlorhydrique concentré, on obtient le produit attendu avec un rendement de 95 %.
[α]_{D}²² = - 5,6 ° (c = 1,0 ; CHCl₃)
RMN ¹H (CDCl₃) : 1,2 (d,3H) ; 1,3-2,0 (m,41H) ; 2,35 (t,2H) ; 2,8-3,55 (m,9H) ; 3,7-3,9 (m,2H) ; 4,04,1 (m,1H) ; 4,85-4,95 (sl,1H) ; 7,35 (t,1H) ; 7,4 (d,1H) ; 8,3 (t,1H) ; 8,6-8,8 (sl,1H) ; 9,3-9,5 (sl,1H) ; 11,5 (s,1H).

### EXEMPLE 6

### N-[2-[[4-[(3-(R)-aminobutyl)amino]butyl]amino]-1-(S)-hydroxyméthyl-2-oxoéthyl]-7-[(aminoiminométhyl)amino]heptanamide, tris-(trifluoroacétate).

On prépare une solution de 1,10 g (1,54.10⁻³ mole) du composé obtenu selon la préparation XVIII dans 15 ml de dichlorométhane, on ajoute 15 ml d'acide trifluoroacétique et on maintient sous agitation pendant 16 heures à température ambiante. Le milieu réactionnel est ensuite concentré sous pression réduite et purifié par chromatographie sur gel de silice greffée de type RP 18, en éluant avec un mélange eau/acide trifluoroacétique/acétonitrile (8/1/1 v/v). Les fractions contenant le produit pur désiré sont lyophilisées puis reprises avec 100 ml d'eau distillée, extraites avec de l'acétate d'éthyle et à nouveau lyophilisées. On obtient ainsi 610 mg du produit attendu sous forme de solide amorphe blanc (Rendement = 52 %).
[α]_{D}²² = - 3,2 ° (c = 1,0 ; CH₃OH)
RMN ¹H (DMSO d₆): : 1,2 (d,3H) ; 1,2-1,35 (m,4H) ; 1,4-1,6 (m,8H) ; 1,70-1,85 (m,1H) ; 1,85-2,0 (m,1H) ; 2,14 (t,2H) ; 2,85-3,15 (m,8H) ; 3,25-3,4 (m,1H) ; 3,55 (d,2H) ; 4,2 (q,1H) ; 4,84,95 (sl,1H) ; 6,85-7,4 (sl,3H) ; 7,65 (t,1H) ; 7,80 (d,1H) ; 7,9 (t,1H) ; 7,95-8,1 (m,3H) ; 8,5-8,8 (s1,3H).
RMN ¹³C (D₂O + Dioxane H₈) : 17,77 ; 23,43 ; 25,58 ; 26,06 ; 26,09 ; 28,25 ; 28,35 ; 30,97 ; 35,86 ; 39,06 ; 41,65 ; 44,35 ; 45,81 ; 47,90 ; 56,54 ; 61,65 ; 158,00 ; 172,54 ; 178,1.

### PREPARATION XIX

### 7-bromo-heptanamide

On prépare un mélange de 25 g (0,131 mole) de 7-bromoheptanenitrile dans 100 ml d'acide chlorhydrique concentré (d = 1,19) et on maintient le mélange sous agitation pendant 12 heures à température ambiante (avantageusement à 15-20° C). On verse ensuite ce mélange sur 300 g de glace, puis on filtre le précipité blanc obtenu. Après lavage à l'eau et séchage, le produit brut est recristallisé dans un mélange acétate d'éthyle/méthylcyclohexane. On obtient ainsi 26,2 g du produit attendu sous forme de cristaux blancs (Rendement = 95 %).
F = 84° C

### PREPARATION XX

### 7-azido-heptanamide

On ajoute 16,4 g (0,25 mole) d'azoture de sodium à une solution de 26,2 g (0,126 mole) de 7-bromo-heptanamide dans 150 ml de DMSO. Après 3,5 heures d'agitation à 80° C, le milieu réactionnel est versé sur de l'eau et extrait avec de l'acétate d'éthyle. La phase organique est lavée à l'aide d'une solution de chlorure de sodium puis séchée et concentrée sous pression réduite. Le solide obtenu est recristallisé dans un mélange acétate d'éthyle/éther isopropylique. On obtient ainsi 14 g du produit attendu sous forme d'un solide cristallisé blanc (Rendement = 65 %).
F = 62° C

### PREPARATION XXI

### Acide 2-[(7-azido-1-oxo-heptyl)amino]-2-[1 -(S)-(naphtalèn-2-yl)éthoxy]-acétique, méthyl ester

On prépare un mélange de 4,4 g (26,1.10⁻³ mole) de 7-azidoheptanamide, 2,9 ml (29,2.10⁻³ mole) de 2-hydroxy-2-méthoxyacétate de méthyle dans 250 ml de dichlorométhane et on porte à reflux pendant 24 heures au moyen d'un dispositif tel que le condensat du reflux traverse un lit de tamis moléculaire 4 Å (environ 30 g). Après retour à température ambiante, le dispositif destiné à éliminer le méthanol est supprimé, on ajoute au milieu réactionnel 2,29 ml (31.10⁻³ mole) de chlorure de thionyle, puis on porte à nouveau à reflux pendant 1.75 h. Le mélange réactionnel est ensuite concentré sous pression réduite. puis repris par 50 ml de dichlorométhane. On ajoute 2,29 ml (31.10⁻³ mole) de triéthylamine, et 4,5 g (26.10⁻³ mole) de (S)-(-)-α-méthyl-2-naphtalèneméthanol [ou 1-(S)-(naphtalen-2-yl)éthanol] en solution dans 50 ml de dichlorométhane. Le mélange réactionnel est agité pendant 24 heures à température ambiante puis lavé successivement avec 100 ml d'acide chlorhydrique 1N et une solution de chlorure de sodium. Après séchage, la phase organique est concentrée sous pression réduite et le résidu est purifié par chromatographie sur gel de silice en éluant avec un mélange hexane/2-propanol 19/1 v/v) On obtient ainsi 6.67 g du produit attendu sous forme d'une huile incolore (Rendement = 62 %).
[α]_{D}²⁵ = - 68° (c = 1,16 ; CHCl₃)
RMN ¹H (CDCl₃) : 1,05-1,67 (m,8H) ; 1,52 (d,3Hy) ; 1,58 (d, 3Hx) ; 1,73-1,9 (2m,2Hy) ; 2,28 (dd,2Hx) ; 3,17 (t,2Hy) ; 3,27 (t,2Hx) ; 3,68 (s,3Hx) ; 3,8 (s,3Hy) 4.96 (m,1H) ; 5.55 (d,1Hx) ; 5,86 (d,1Hy) ; 6.18 (d,1Hy) ; 6,55 (d,1Hx) ; 7,4-7,5 (m,3H) ; 7,8-7,9 (m,4H).
(Les protons indiqués Hx et Hy sont attribuables aux épimères respectivement x et y du composé analysé)

### PREPARATION XXII

### Acide 3-(R)-méthyl-6-phénylméthyl-12-oxo-13,13,13-trifluoro-2,6,11-triazatridécanoïque, 1,1-diméthyléthyl ester

On prépare une solution de 3,12 g (8,9.10⁻³ mole) d'acide [1-(R)-méthyl-3-[(phénylméthyl)(4-aminobutyl)amino]propyl]carbamique, 1,1-diméthyl éthyl ester et 1,37 ml (9,8.10⁻³ mole) de triéthylamine dans 20 ml de dichlorométhane, et on ajoute goutte à goutte une solution de 1,39 ml (9,84.10⁻³ mole) d'anhydride trifluoroacétique dans 10 ml de dichlorométhane, en maintenant la température du milieu réactionnel à 0° C. Le mélange est ensuite agité 1,5 h à environ 20° C puis lavé successivement avec une solution d'acide chlorhydrique 1N, une solution de bicarbonate de sodium et de l'eau. La phase organique est séchée et concentrée sous pression réduite. Le résidu obtenu est purifié par chromatographie sur gel de silice en éluant avec un mélange acétate d'éthyle/méthylcyclohexane (1/1 ; v/v). On obtient ainsi 2,6 g du produit attendu sous forme d'un solide blanc hygroscopique (Rendement = 65 %).
[α]_{D}²² = 0° (c= 1 ; CH₃OH)
RMN ¹H (DMSO d₆) : 0,94 (d,3H) ; 1,35-1,56 (m,15H) ; 2,31-2,37 (m, 4H) ; 3,11 (m,2H) ; 3,47 (m,3H) ; 7,19-7.29 (m,5H) ; 9,38 (t,1H).

### PREPARATION XXIII

### N-[4-[(3-(R)-aminobutyl)-(phénylméthyl)amino]butyl]-2,2,2-trifluoroacétamide (dichlorhydrate)

On prépare un mélange de 2,8 g (6,8.10⁻³ mole) du composé obtenu selon la préparation XXII dans 32 ml d'une solution de chlorure d'hydrogène 1M dans l'acétate d'éthyle et on maintient la solution résultante sous agitation pendant 24 h à température ambiante. Le milieu réactionnel est ensuite concentré sous pression réduite et on obtient ainsi 2.34 g du produit attendu sous forme d'un solide amorphe et hvgroscopique (Rendement = 89 %).
[α]_{D}²²= +1,86° (c= 1,04 : CH₃OH)
RMN ¹H (DMSO d₆) : 1,14-1,18 (m,3H) ; 1,4-1,5 (m,2H) : 1,7-1,8 (m, 2H) ; 1,9-2 (m, 1H) ; 2,1-2,2 (m,1H) ; 3,13-3,48 (m,7H) ; 4,32 (s,2H) ; 7,47-7,48 (m,3H) ; 7,6 (m,2H) ; 8,1 (m,3H) ; 9,5 (m,1H) ; 10,7 (m,1H).

### PREPARATION XXIV

### N-[4-[(3-(R)-aminobutyl)amino]butyl]-2,2,2-trifluoroacétamide (dichlorhydrate)

On prépare une solution de 2,34 g (5,56 10⁻³ mole) du composé selon la préparation XXIII dans 50 ml de méthanol et on ajoute 0.585 ml (6,67.10⁻³ mole) d'acide chlorhydrique concentré. puis 200 mg de charbon palladié à 10 %. Le mélange est agité sous atmosphère d'hydrogène à une pression de 10⁵ Pa et à température ambiante pendant 8 h. Après séparation du catalyseur par filtration, le filtrat est concentré sous pression réduite. On obtient ainsi 1,72 g du produit attendu sous forme d'une poudre blanche (Rendement = 98 %).
F = 210-215° C
[α]_{D}²⁵ = + 2 ° (c= 1,15 ; CH₃OH)

### PREPARATION XXV

### Acide 3-(R)-méthyl-6-(phénylméthoxycarbonyl)-12-oxo-13,13,13-trifluorométhyl-2,6,11-triaza-tridécanoïque, phénylméthyl ester

On dissout 1 g (2,32.10⁻³ mole) du composé obtenu selon la préparation XXIV dans 15 ml de méthanol et on ajoute goutte à goutte une solution aqueuse de bicarbonate de sodium jusqu'à obtention d'un pH égal à 9. On refroidit le mélange à 0° C et on ajoute 1 ml de chloroformiate de benzyle (6,97.10⁻³ mole). On maintient ensuite le pH à 9 par addition de la solution de bicarbonate de sodium. Lorsque le pH est stable, on maintient le milieu réactionnel sous agitation pendant 2 h, puis on neutralise jusqu'à pH 7 à l'aide d'une solution diluée d'acide chlorhydrique. Le mélange est en partie concentré sous pression réduite de façon à éliminer le méthanol et la phase aqueuse résiduelle est extraite par de l'acétate d'éthyle. La phase organique obtenue est lavée avec une solution saturée de chlorure de sodium, séchée puis concentrée sous pression réduite. Le résidu obtenu est purifié par chromatographie sur gel de silice en éluant avec un mélange acétate d'éthyle/méthylcyclohexane (1/1 v/v). On obtient ainsi 0,88 g du produit attendu sous forme d'une huile épaisse (Rendement = 72 %) ; ce produit est ensuite cristallisé dans l'éther isopropylique.
F = 74° C
[α]_{D}²⁵ = - 5° (c= 0,98 ; CHCl₃).

### PREPARATION XXVI

### Acide-N-[4-amino-butyl]-N-[3-(R)-[(phénylméthoxycarbonyl)amino]butyl]carbamique, phénylméthyl ester

On prépare une solution de 0,92 g (1,75.10⁻³ mole) du composé obtenu selon la préparation XXV dans 20 ml de 1,2-diméthoxyéthane et on ajoute 8,8 ml d'une solution aqueuse d'hydroxyde de sodium 1N. On maintient sous agitation pendant 2 heures à température ambiante puis on ajoute 50 ml d'une solution aqueuse saturée de chlorure de sodium et on extrait à l'aide d'acétate d'éthyle. La phase organique est séchée puis concentrée sous pression réduite. On obtient ainsi 0.75 g du produit attendu sous forme d'une huile épaisse (Rendement = 100%).
RMN ¹H (DMSO d₆) : 1,03 (m,3H) ; 1,2-1,25 et 1,43-1,46 (m,2H) 1,59 (m,
2H) ; 3,17-3.47 (m,6H) ; 3,48 (m,1H) ; 5.0 (s,2H) : 5,05 (s,2H) ; 7,22 (d,1H) ; 7,25-7,5 (m,10H).

### PREPARATION XXVII

### Acide 2-[(7-azido-1-oxo-heptyl)amino]-2-[1-(S)-(naphtalèn-2-yl)éthoxy]-acétique

On dissout 4,75 g (11,5.10⁻³ mole) de l'ester obtenu selon la préparation XXI dans 50 ml de 1,2-dimélhoxyéthane et on ajoute goutte à goutte 13,8 ml d'une solution aqueuse d'hydroxyde de sodium 1N. Le mélange est agité pendant 1 h à température ambiante puis acidifié jusqu'à pH 2 à l'aide d'une solution d'acide chlorhydrique 1N. On ajoute 50 ml d'une solution saturée de chlorure de sodium, puis on extrait par de l'acétate d'éthyle. La phase organique est séchée puis concentrée sous pression réduite, à température ambiante. On obtient ainsi 4,6 g du produit attendu sous forme d'une huile épaisse (Rendement = 99 %).
[α]_{D}²⁵ = - 76° (c= 0,96 ; CHCl₃).
RMN ¹H (DMSO d₆) : 1,09-1,54 (m,8H) ; 1,43 et 1,47 (2d,3H) ; 2,18 (m, 2Ha) ; 2,50 (m,2Hb) ; 3,24 (t,2Ha) ; 3,31 (t,2Hb) ; 4,8 (q,1H) ; 5,25 (d,1Ha) ; 5,49 (d,1Hb) ; 7,45-7,55 (m,3H) ; 7,79 (s,1H) ; 7,85-7,93 (m,3H) ; 8,72 (d,1Hb) ; 8,80 (d,1Hb) ; 13(m,1H).

### PREPARATION XXVIII

### Acide 21-azido-13-(S)-[1-(S)-(naphtalèn-2-yl)éthoxy]-6-(phénylméthoxycarbonyl)-3-(R)-méthyl-12,15-dioxo-2,6,11,14-tétraaza-henéicosanoïque, phénylméthyl ester

On prépare une solution de 4,58 g (11,5.10⁻³ mole) de l'acide obtenu selon la préparation XXVII dans 50 ml de dichlorométhane et on ajoute 1,55 g (11,5.10⁻³ mole) de HOBT et 2,6 g (12,6.10⁻³ mole) de DCC. Après 0,5 h d'agitation à température ambiante, on ajoute 4,93 g (11,5.10⁻³ mole) du produit obtenu selon la préparation XXVI et on maintient sous agitation pendant 24 heures à température ambiante. Le mélange réactionnel est ensuite concentré sous pression réduite et le résidu est repris dans 50 ml d'acétate d'éthyle. Les insolubles sont éliminés par filtration et le filtrat est concentré sous pression réduite. Le produit brut ainsi obtenu contenant les deux isomères, est purifié par chromatographie sur gel de silice ["Kieselgel 60 Merck" (granulométrie : 15 à 40 µm)] en éluant avec un mélange acétate d'éthyle/éther isopropylique (7/3 v/v) Cette purification permet la séparation des deux isomères (le produit attendu, dont le carbone en position 13 est de configuration (S) est élué avant son isomère dans lequel ledit carbone en position 13 est de configuration (R)). On obtient ainsi 3,7 g du produit attendu sous forme d'un solide non cristallisé (Rendement = 40 %, soit 80 % si l'on considère un isomère). On obtient simultanément 3,2 g du second isomère.
[α]_{D}²⁵ = - 41° (c= 0,99 ; CHCl₃).
RMN ¹H (DMSO d₆) : 1.04 (m,3H) ; 1,42 (d,3H) , 1,28-1,6 (m, 14H) ; 2,1-2,2 (m,2H) ; 3.0-3,17 (m,6H) ; 3,31 (t,2H) ; 3,49 (m,1H) ; 4,8 (q,1H) ; 4,99 (s,2H) ; 5,04 (s,2H) ; 5.17 (d,1H) ; 7,2 (d,1H) ; 7,22-7,32 (m,10H) ; 7,48-7,57 (m,3H) ; 7,8-7.9 (m,4H) ; 8,0 (m,1H) ; 8,64 (d,1H).

### PREPARATION XXVIII bis

### Acide 21-azido-13-(S)-[1-(S)-(naphtalèn-2-yl)éthoxy]-6-(phénylméthoxy-carbonyl)-3-(R,S)-méthyl-12,15-dioxo-2,6,11,14-tétraazahenéicosanoïque, phénylméthyl ester

En opérant selon le procédé de la préparation XXVIII, en remplaçant le produit de la préparation XXVI par son racémique, à savoir l'acide N-[4-amino-butyl]-N-[3-(R,S)-[(phénylméthoxycarbonyl)amino]butyl]carbamique, phénylméthyl ester, on obtient le produit attendu.

### PREPARATION XXIX

### Acide 3-[(phénylméthoxycarbonyl)amino]-20-(phénylméthoxycarbonyl)-13-(S)-[1-(S)-(naphtalèn-2-yl)éthoxy]-23-(R)-méthyl-11,14-dioxo-2,4,12,15,20, 24-hexaazapentacos-2-ènedioïque, bis(phénylméthyl) ester

On prépare une solution de 3,55 g (4,47.10⁻³ moles du composé obtenu selon la préparation XXVIII dans 50 ml de tètrahydrofuranne et on ajoute 1,41 g (5,36.10⁻³ mole) de de triphénylphosphine et 100 µl (5,36.10⁻³ mole) d'eau. Le mélange est porté à retlux du solvant pendant 15 heures, puis refroidi à température ambiante. On ajoute alors 1,92 g (5,36.10⁻³ mole) d'acide [[[(phénylméthoxy)carbonyl]amino](méthylthio)méthylène]carbamique, phénylméthyl ester (autre dénomination : N,N'-bis[benzyloxycarbonyl]-S-méthyl-isothiourée) et on maintient sous agitation pendant 24 heures à température ambiante. Après concentration sous pression réduite, le résidu est purifié par chromatographie sur gel de silice en éluant avec de l'acétate d'éthyle. Le produit purifié obtenu est cristallisé dans l'éther éthylique, puis recristallisé dans un mélange butanone/éther isopropylique. On obtient ainsi 2,5 g du produit attendu sous forme d'une poudre blanche (Rendement = 52 %).
F = 58° C
[α]_{D}²⁵ = - 34° (c= 1 ; CHCl₃).

### PREPARATION XXIX bis

### Acide 3-[(phénylméthoxycarbonyl)amino]-20-(phénylméthoxycarbonyl)-13-(S)-[1-(S)-(naphtalèn-2-yl)éthoxy]-23-(R,S)-méthyl-11,14-dioxo-2,4,12,15,20, 24-hexaazapentacos-2-ènedioïque, bis(phénylméthyl) ester

En opérant selon le procédé de la préparation XXIX, en remplaçant le produit de la préparation XXVIII par celui de la préparation XXVIII bis, on obtient le produit attendu.

### EXEMPLE 7

### 7-[(aminoiminométhyl)amino]-N-[2-[[4-[(3-(R)-aminobutyl)amino]butyl]amino]-1-(S)-hydroxy-2-oxoéthyl]heptanamide (triacétate)

On prépare une solution de 130 mg (0,118.10⁻³ mole) du composé obtenu selon la préparation XXIX, dans 3 ml de dioxane que l'on mélange ensuite à 50 ml d'une solution 1N d'acide acétique dans l'eau. On ajoute ensuite 100 ml de catalyseur de Pearlman (hydroxyde de palladium sur charbon contenant environ 20 % de palladium), puis on agite le mélange sous atmosphère d'hydrogène, sous une pression de 20,10⁵ Pas, à température ambiante, pendant environ 30 heures. Cette opération est répétée une fois après avoir remplacé le catalyseur usagé par du catalyseur neuf. Après séparation du catalyseur par filtration, le filtrat est lavé par 50 ml d'éther éthylique, puis lyophilisé. On obtient ainsi 40 mg du composé attendu sous forme d'un solide blanc très hygroscopique (Rendement = 70 %). La pureté est supérieure à 99 % (HPLC).
[α]_{D}²⁰ = -11° [c= 1,99 ; CH₃COOH(1N)H₂O)]
RMN ¹H (D₂O) ; 1,26-1,28 (m,7H) ; 1,4-1,7 (m,8H) ; 1,8-2,0 (m, 1H) ; 3,0-3,12 (m,6H) ; 3,22 (m,2H) 3,41 (m,1H) ; 5,37 (s,1H).
RMN ¹³ c (D₂O) : 18,05 ; 23,73 ; 24,10 ; 25.66 ; 26,28 ; 28,48 ; 28,5 ; 31,2 ; 36,3 ; 39,3 ; 41,9 ; 44,6 ; 46,0 ; 48,15 ; 72,7 ; 157,5 ; 172,16 ; 178,3 ; 181,14.

### EXEMPLE 8

### 7-[(aminoiminométhyl)amino]-N-[2-[[4-[(3-(R,S)-aminobutyl)amino]butyl]amino]-1-(S)-hydroxy-2-oxoéthyl]heptanamide (triacétate)

En opérant selon le procédé de l'exemple 7. en remplaçant le produit de la préparation XXIX par celui de la préparation XXIX bis, on obtient le produit attendu.

### PREPARATION XXX

### Acide 2-(S)-[(7-azido-1-oxo-heptyl)amino]-2-[1-(S)-(naphtalèn-2-yl)-éthoxy]acétique, méthyl ester

Le mélange d'isomères obtenu selon la préparation XXI est séparé par chromatographie sur gel de silice [phase Matrex AMICON, silice sphérique Si 100-15s ; taille des pores : 100Å; taille des particules : 15µm]. L'éluant utilisé est un mélange dichlorométhane/acétate d'éthyle (95/5 ; v/v). Au départ de 4,5 g de mélange on obtient 2,05 g du composé attendu, (sous forme cristallisée ainsi que 1,41 g de l'isomère (R) et 1,04 g de mélange des deux isomères.
F = 32° C
[α]_{D}³⁰ = - 50° (c = 0,94 ; CHCl₃)
RMN ¹H (CDCl₃) : 1,37-1,39 (m,4H) ; 1,53 (d,3H) ; 1,55-1,7 (m, 4H) ; 2.27 (d,2H) ; 3,26 (t,2H) ; 3,68 (s,3H) ; 4.97 (q,1H) ; 5,55 (d,1H) ; 6,52 (d,1H) ; 7,44-7,54 (m,3H) ; 7,81-7,87 (m,4H).
La configuration absolue du carbone en position 2 de ce composé a été vérifiée par spectrographie aux rayons X.

### PREPARATION XXXI

### Acide 2-(S)-[(7-azido-1-oxo-heptyl)amino]-2-[1-(S)-(naphtalèn-2-yl)-éthoxy]acétique

En opérant de façon analogue au procédé de la préparation XXVII, au départ du composé obtenu selon la préparation XXX, on obtient l'acide attendu sous forme huileuse avec un rendement de 100 %.
[α]_{D}²⁵ = - 40° (c = 1,1 ; CHCl₃)
RMN ¹H (DMSO d₆) : 1,35-1,39 (m,4H) ; 1,56 (d,3H) ; 1,56-1,71 (m, 4H) ; 2,28 (t,2H) ; 3,26 (t,2H) ; 4,99 (q,1H) ; 5,52 (d,1H) ; 6,48 (d,1H) ; 7,44-7,55 (m,3H) ; 7,8-7,9 (m,4H).

### PREPARATION XXXII

### Acide 21-azido-13-(S)-[1-(S)-(naphtalèn-2-yl)éthoxy]-6-(phénylméthoxy-carbonyl)-3-(R)-méthyl-12,15-dioxo-2,6,11,14-tétraazahenéicosanoïque, phénylméthyl ester

Ce produit, identique au composé de la préparation XXVIII, est obtenu selon le même mode de synthèse que celui décrit dans la préparation XXVIII, mais dans le cas présent la purification par chromatographie n'est effectivement pas nécessaire.

A partir du produit de la préparation XXXII, le composé de l'exemple 7 est obtenu en suivant les modalités opératoires déjà décrites ci-dessus (préparation XXIX et exemple 7).

### PREPARATION XXXIII

### Acide 2-[(7-azido-1-oxoheptyl)amino]-2-(phénylméthoxy)acétique, méthyl ester

En opérant selon le procédé de la préparation XXI, mais en remplaçant le (S)-(-)α-méthyl-2-naphtalèneméthanol par l'alcool benzylique, on obtient le produit attendu sous forme d'une huile incolore (Rendement = 67 %).
RMN ¹H (CDCl₃) : 1,3-1,5 (m,4H) ; 1,5-1,75 (m,4H) ; 2,25 (t, 2H) ; 3,25 (t,2H) ; 3,8 (s,3H) ; 4,71 (q,2H) ; 5,73 (d,1H) ; 6,50 (d,1H) ; 7,25-7,45 (m.5H).

### PREPARATION XXXIV

### Acide 2-[(7-azido-1-oxoheptyl)amino]-2-(phénylméthoxy)acétique

En opérant selon le procédé de la préparation XXVII, mais en remplaçant le produit de la préparation XXI par le produit de la préparation XXXIII, on obtient le produit attendu.
RMN ¹H (CDCl₃) : 1,3-1,5 (m,4H) ; 1,55-1,55 (m,4H) ; 2,26 (t, 2H) ; 3,25 (t,2H) : 4,7-4,8 (m,2H) ; 5,75 (d,1H) ; 6,59 (d, 1H) ; 7,25-7,4 (m,5H).

### PREPARATION XXXV

### Acide 21-azido-13-(R,S)-(phénylméthoxy)-6-(phénylméthoxycarbonyl)-3-(R)-méthyl-12,15-dioxo-2,6,11,14-tétrazahenéicosanoïque, phénylméthyl ester

En opérant selon le procédé de la préparation XXVIII, mais en remplaçant le produit de la préparation XXVII par le produit de la préparation XXXIV, et sans opérer de séparation des isomères lors de la purification sur gel de silice, on obtient le produit attendu sous forme d'une huile de couleur jaune (Rendement = 92 %).
RMN ¹H (CDCl₃) : 1,05-1,75 (m,17H) ; 2,26 (t,2H) ; 2,95-3,5 (m, 8H) ; 3,6-3,8 (m,1H) ; 4,54,75 (m,2H) ; 5,07 (s,2H) ; 5,09 (s,2H) ; 5,55-5,7 (m,1H) ; 6,4-6.9 (m,2H) 7,2-7,45 (m,16H).

### PREPARATION XXXVI

### Acide 3-[(phénylméthoxycarbonyl)mnino]-20-(phénylméthoxycarbonyl)-13-(R,S)-(phénylméthoxy)-23-(R)-méthyl-11,14-dioxo-2,4,12,15,20,24-hexaazapentacos-2-ènedioïque, bis(phénylméthyl) ester

En opérant selon le procédé de la préparation XXIX, mais en remplaçant le produit de la préparation XXVIII par le produit de la préparation XXXV, on obtient le produit attendu sous forme de solide amorphe (Rendement = 69 %).
RMN ¹H (CDCl₃) : 0,95-1,1 (m,3H) ; 1,2-1,7 (m,14H) ; 2,1-2,25 (m, 2H) ; 3-3,4 (m,8H) ; 3,4-3,55 (m,1H) ; 4,49 (q,2H) ; 4,99 (s,2H) ; 5,02 (s,2H) ; 5,03 (s,2H) ; 5,20 (s,2H) ; 5,46 (d, 1H); 7,15-7,5 (m,26H) ; 8,12 (t,1H) ; 8,39 (t,1H) ; 8,63 (d, 1H) ; 11,59 (s,1H).

### EXEMPLE 4 bis

### 7-[(aminoiminométhyl)amino]-N-[2-[[4-[(3-(R)-aminobutyl)amino]butyl]amino]-1-(R,S)-hydroxy-2-oxoéthyl]heptanamide, triacétate.

En opérant selon le procédé de l'exemple 7, mais en remplaçant le produit de la préparation XXIX par le produit de la préparation XXXVI, on obtient le produit attendu sous forme d'un solide amorphe jaune pâle (Rendement = 81 %).
[α]_{D}²¹ = + 0,5° (c= 1 ; MeOH)
RMN ¹H (D₂O) : 1,2-1,4 (m,7H) ; 1,5-1,75 (m,8H) ; 1,85 (s, 9H) ; 1,9-2,15 (m,2H) ; 2,23 (m,2H) ; 3-3,2 (m,6H) ; 3,2-3,3 (m,2H) ; 3,35-3,5 (m,1H) ; 5,37 (s,1H).
RMN ¹³C (D₂O) : 18,05 ; 23,75 ; 24,10 ; 25,67 ; 26,30 (2C) ; 28,50 ; 28,55 ; 31,27 ; 36,30 ; 39,33 ; 41,88 ; 44,63 ; 46,07 ; 48,17 ; 72,76 ; 157,5 ; 172,16 ; 178,34 ; 181,14.

### PREPARATION XXXVII

### Acide 21-azido-13-(S)-[1-(S)-(naphtalèn-2-yl)éthoxy]-6-(phénylméthoxycarbonyl)-12,15-dioxo-2,6,11,14-tetraazahenéicosanoïque, phénylméthyl ester

En opérant de façon analogue au procédé de la préparation XXVIII, au départ du composé obtenu selon la préparation XXVII et de l'acide [4-aminobutyl]-[3-[(phénylméthoxycarbonyl)amino]propyl]carbamique, phénylméthyl ester, après purification par chromatographie sur gel de silice et élution avec un mélange acétate d'éthyle/éther isopropylique (6/4 v/v), on obtient le produit attendu avec un rendement de 30 % [soit 60 % si on considère l'isomère (S)].
[α]_{D}²⁰ = - 40,7° (c= 5,4 ; CHCl₃).
RMN ¹H (DMSO d₆) : 1,44 (d,3H) ; 1,2-1,6 (m,14H) ; 2,17 (m, 2H) ; 2,9-3,05 (m,6H) ; 3,31 (t,2H) ; 4,8 (q,1H) ; 4,99 (s,2H) ; 5,04 (s,2H) ; 5,17 (d,1H) ; 7,27-7,32 (m,11H) ; 7,81 (s,1H) ; 7,86-7,9 (m,3H) ; 8,0 (m,1H) ; 8,64 (d,1H).

### PREPARATION XXXVIII

### Acide 3-[(phénylméthoxycarbonyl)amino]-20-(phénylméthoxycarbonyl)-13-(S)-[1-(S)-(naphtalèn-2-yl)éthoxy]-11,14-dioxo-2,4,12,15,20,24-hexaazapentacos-2-ènedioïque, bis(phénylméthyl) ester

En opérant de façon analogue au procédé de la préparation XXIX, au départ du composé obtenu selon la préparation XXXVII, on obtient le produit attendu avec un rendement de 77 %.
F = 98° C
[α]_{D}²⁵ = - 33° (c= 1 ; CHCl₃).

### EXEMPLE 9

### 7-[(aminoiminométhyl)amino]-N-[2-[[4-[(3-aminopropyl)amino]butyl]-amino]-1-(S)-hydroxy-2-oxoéthyl]heptanamide, trichlorhydrate.

En effectuant une hydrogénation de façon analogue au procédé de l'exemple 7, au départ du composé de la préparation XXXVIII, on obtient l'isomère S de la 15-déoxyspergualine sous forme de tris-acétate qui est ensuite converti en tri-chlorhydrate à l'aide d'une solution d'acide chlorhydrique puis lyophilisé. Les caractéristiques physiques de ce produit sont identiques à celles publiées dans la littérature (J. Antibiot., 1987, 1316-1324).

L'activité immunosuppressive des produits selon l'invention a été mise en évidence à l'aide d'un test dit de réaction du greffon contre l'hôte. Des souris mâles B6D2F1 (hybrides de première génération C57B1/6 x DBA/2) sont immunodéprimées par une injection intrapéritonéale (i.p.) de cyclophosphamide. Trois jours plus tard (jour 0 de l'expérience : J0), elles reçoivent par voie intraveineuse 4 x 10⁷ splénocytes de souris C57B1/6. Les animaux sont ensuite répartis par lot de 8 au minimum et reçoivent un traitement journalier de J1 à J5 et de J7 à J10 par voie i.p. Le groupe contrôle reçoit le véhicule seul. La mortalité est suivie jusqu'à J60. Les résultats, exprimés par la valeur moyenne de la survie en jours à la dose indiquée, sont regroupés dans le tableau I où les valeurs données sont significatives selon le test de Logrank (probabilité inférieure ou égale à 5%). Pour comparaison, on a également indiqué dans le tableau I les valeurs obtenues avec des produits de structure apparentée, à savoir :
- **Produit A :**: la 15-déoxyspergualine (composé racémique sous forme de tri-chlorhydrate)
- **Produit B :**: 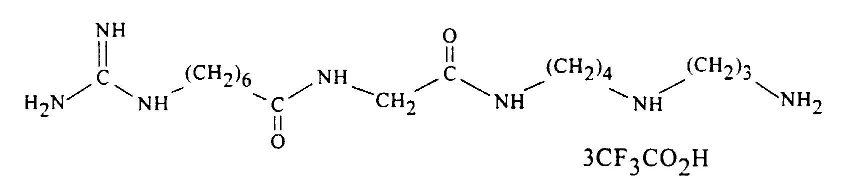
- **Produit C :**: 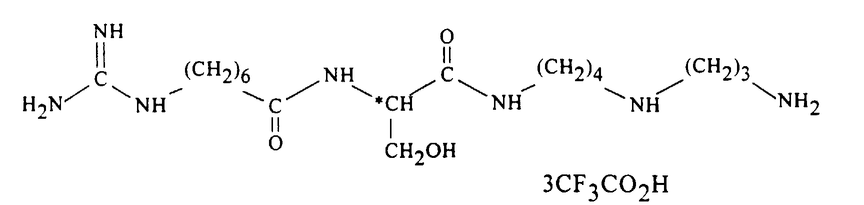
où *C est de configuration (S).

Les résultats du tableau I montrent que les produits de formule I et leurs sels d'addition non-toxiques selon l'invention présentent une meilleure activité que les produits de l'art antérieur, ou nécessitent une plus faible posologie pour obtenir une activité équivalente.

Les produits de formule I et leurs sels d'addition non-toxiques selon l'invention sont utiles en thérapeutique, eu égard à la réaction du greffon contre l'hôte suite à une greffe vascularisée ou non. en tant qu'agents immunosuppresseurs curatifs ou préventifs. notamment dans la prévention du rejet d'organes allogéniques ou xénogéniques vasculaires ou non, dans le traitement des maladies auto-immunes génétiquement définies ou acquises (comme par exemple le lupus érythémateux disséminé, la sclérose en plaques, la polyarthrite rhumatoïde), dans le traitement des maladies inflammatoires chroniques, telles que par exemple les rhumatismes articulaires, dans le traitement ou la prévention de maladies inflammatoires hyperréactives telles que par exemple les colites ulcératives ou l'asthme. ainsi que dans toutes les pathologies où un désordre immunitaire apparaît être la cause ou le facteur responsable du maintien d'un état clinique dégradé.

Les produits de formule I et leurs sels d'addition non-toxiques selon l'invention peuvent également être administrés en complément de drogues anticancéreuses cytotoxiques afin d'en limiter les effets secondaires et en complément de l'administration de produits issus des biotechnologies. notamment les cytokines recombinantes. les anticorps mono- et polyclonaux afin de diminuer l'apparition des anticorps protecteurs produits par le patient.

Les produits de formule I et leurs sels d'addition non-toxiques selon l'invention peuvent être utilisés dans le traitement curatif de parasitoses. en particulier dans le cas du paludisme.

Les produits de formule I et leurs sels d'addition non-toxiques selon l'invention peuvent être administrés par voie orale. par voie injectable (notamment par voie intramusculaire ou intraveineuse), par voie topique (notamment sous forme de crème pour application locale, de gouttes oculaires), par voie transdermique. par voie rectale sous forme de suppositoire, ou par inhalation.

Les composés de formule I et leurs sels d'addition sont également utiles en tant que réactifs pour dosage analytique notamment en pharmacologie, en particulier dans le cas de l'étude des maladies auto-immunes.

### Meilleur mode

Le meilleur mode (i.e. le mode que l'on préfère pour la mise en oeuvre de la présente invention) consiste à faire appel à un composé de formule I où R est OH. *C est de configuration (R,S) ou (S) et **C est de configuration (R,S) ou (R) ou à l'un de ses sels d'addition non-toxiques. en tant qu'agent immunosuppresseur.

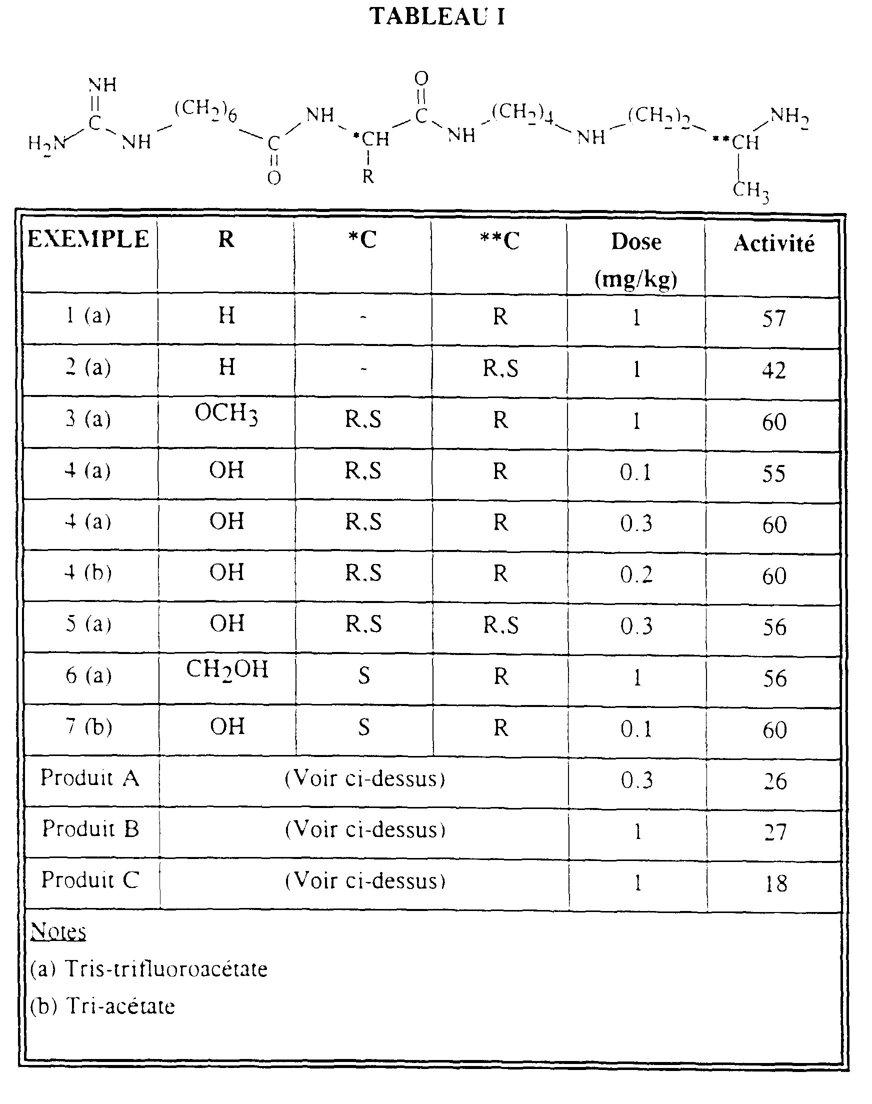

## Revendications

1. Composé appartenant à la famille des analogues de la 15-déoxyspergualine, caractérisé en ce qu'il est choisi parmi l'ensemble constitué par :
**(i)** les composés de formule : 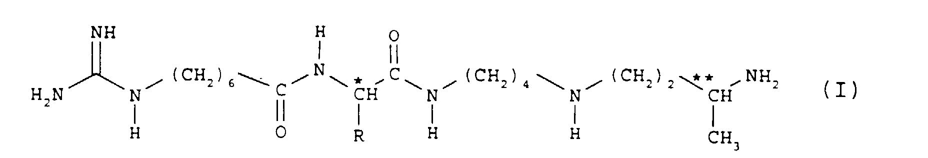 dans laquelle
- R représente un atome d'hydrogène, un groupe OH, un groupe OCH₃ ou un groupe CH₂OH,
- *C, dans le cas où R n'est pas un atome d'hydrogène, est un atome de carbone asymétrique de configuration (R,S), (R) ou (S),
- **C est un atome de carbone asymétrique de configuration (R,S) ou (R), et
**(ii)** leurs sels d'addition.

2. Composé selon la revendication 1, caractérisé en ce que l'atome de carbone asymétrique **C est de configuration (R).

3. Composé selon la revendication 1, caractérisé en ce que l'atome de carbone asymétrique *C est de configuration (S) et R est le groupe OH.

4. Procédé de préparation d'un composé de formule I ou l'un de ses sels d'addition selon la revendication 1, ledit procédé étant caractérisé en ce qu'il comprend les étapes consistant à :
**(i)** déprotéger un composé de formule II : 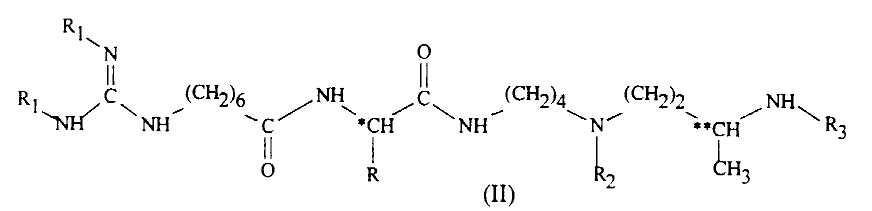 dans laquelle :
- R représente un atome d'hydrogène, un groupe OCH₃, un groupe OH, un groupe CH₂OH, un groupe OR' ou un groupe CH₂OR',
- R' représente un groupe protecteur de la fonction hydroxyle,
- R₁, R₂ , R₃, identiques ou différents, représentent chacun un groupe protecteur de la fonction amine,
- *C représente, lorsque R n'est pas un atome d'hydrogène, un atome de carbone asymétrique de configuration (R,S), (R) ou (S),
- **C représente un atome de carbone asymétrique de configuration (R,S) ou (R),
selon un ou plusieurs traitements réactionnels connus de l'homme de l'art, pour obtenir le remplacement de tous les groupes protecteurs R₁, R₂ , R₃ et R' par un atome d'hydrogène, et
**(ii)** si necessaire, à partir d'un sel d'addition obtenu selon l'étape (i), obtenir le composé de formule I sous forme de base libre par action d'une base forte, puis obtenir, à partir de ladite base libre les autres sels d'addition.

5. Procédé selon la revendication 4, caractérisé en ce qu'il comprend également les étapes consistant à :
**(a)** selon la variante A :
**(i)** condenser un acide de formule : 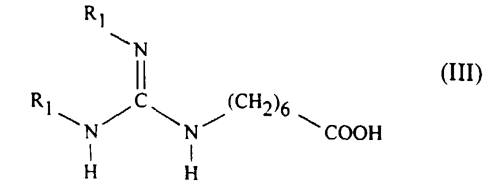 dans laquelle R₁ représente un groupe amino-protecteur, avec un dérivé d'aminoacide de formule : 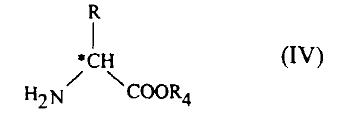 dans laquelle :
- R représente un atome d'hydrogène ou un groupe CH₂OR',
- R' représente un groupe protecteur de la fonction hydroxyle,
- R₄ représente un groupe alkyle en C₁-C₃, et,
- *C, lorsque R n'est pas l'atome d'hydrogène, représente un atome de carbone asymétrique de configuration (R,S), (R) ou (S),
par activation de la fonction acide à l'aide d'un agent de couplage de type carbodiimide, en présence d'un agent nucléophile, dans un solvant organique, à une température comprise entre 0 et 40° C, et à raison de 1 mole du composé III pour environ 1 mole du composé IV, pour obtenir un composé de formule : 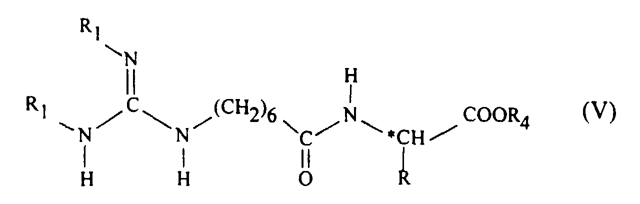 dans laquelle R, R₁, R₄ et *C conservent les mêmes significations que ci-dessus ;
**(ii)** hydrolyser la fonction ester d'un composé de formule V, ainsi obtenu,
- soit selon l'étape (i) ci-dessus lorsque R représente l'atome d'hydrogène ou un groupe CH₂OR' tel que décrit ci-dessus,
- soit selon un procédé connu lorsque R représente un groupe OR', où R' représente un groupe protecteur de la fonction hydroxyle notamment Si(CH₃)₂ C(CH₃)₃,
par action d'une base diluée, en présence d'un solvant miscible à l'eau, à une température voisine de la température ambiante (540 °C), et pendant environ 2 à 30 minutes, pour obtenir un composé de formule : 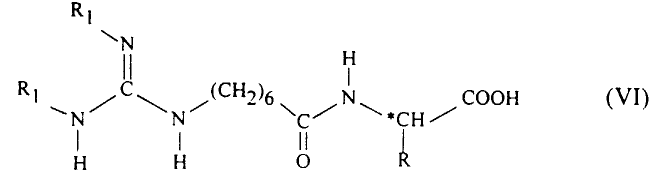 dans laquelle R₁ et *C conservent la même signification que dans le composé de formule V, et R représente un atome d'hydrogène, un groupe CH₂OR' ou un groupe OR', R' représentant un groupe protecteur de la fonction hydroxyle ;
**(iii)** faire réagir un composé de formule VI, ainsi obtenu, avec un composé de formule : 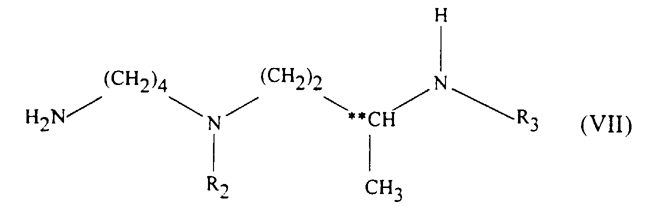 dans laquelle :
- R₂ et R₃, identiques ou différents, représentent chacun un groupe amino-protecteur, et
- **C représente un carbone asymétrique de configuration (R,S) ou (R), dans des conditions identiques à celles décrites à l'étape (i) ci-dessus pour obtenir un composé de formule II ; 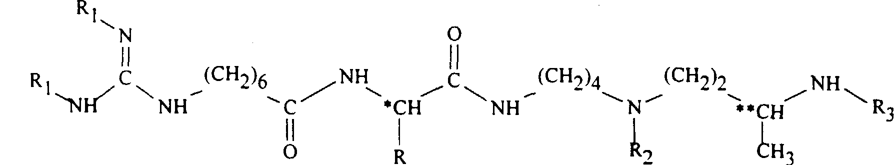 dans laquelle R, R₁, R₂, R_{3,}, *C et **C conservent la même signification que ci-dessus ;
**(b)** selon la variante B :
**(i)** condenser un dérivé d'aminoacide de formule : 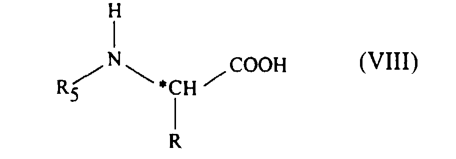 dans laquelle :
- R représente un atome d'hydrogène ou un groupe CH₂OR' où R' est un groupe protecteur de la fonction hydroxyle,
- R₅ représente un groupe protecteur de la fonction amine, et
- *C représente, lorsque R n'est pas l'atome d'hydrogène, un atome de carbone asymétrique de configuration (R,S), (R) ou (S),
avec un composé de formule : 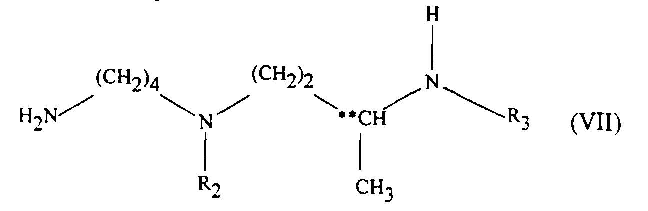 dans laquelle :
- R₂ et R₃, identiques ou différents, représentent chacun un groupe amino-protecteur, tous deux différents du groupe protecteur R₅ présent dans le composé de formule VIII, et
- **C représente un atome de carbone asymétrique de configuration (R,S) ou (R),
dans des conditions analogues à celles du procédé de l'étape (i) de la variante A ci-dessus, pour obtenir un composé de formule : 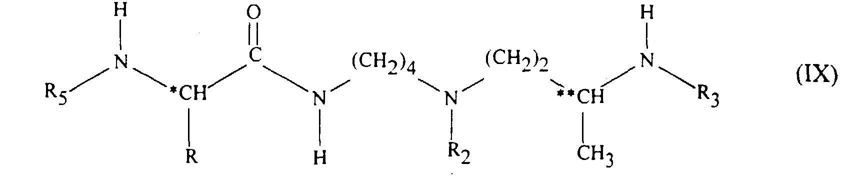 dans laquelle R, R₂, R₃, R₅, *C et **C conservent la même signification que ci-dessus ;
**(ii)** déprotéger le composé IX, ainsi obtenu, selon un procédé spécifique au clivage de la liaison N-R₅, pour obtenir un composé de formule : 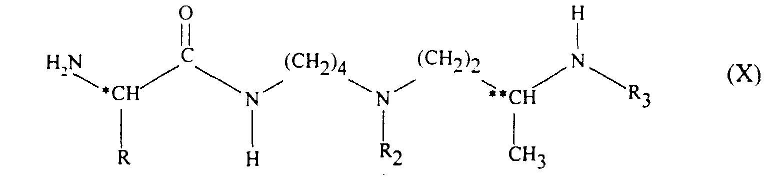 dans laquelle R, R₂, R₃, *C et **C conservent la même signification que ci-dessus ; et,
**(iii)** condenser le produit amine de formule X, ainsi obtenu, avec un acide de formule : 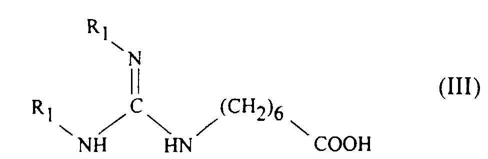 dans laquelle R₁ représente un groupe amino-protecteur,
dans des conditions opératoires analogues à celles décrites pour le procédé (i) de la variante A, pour obtenir un composé de formule II : 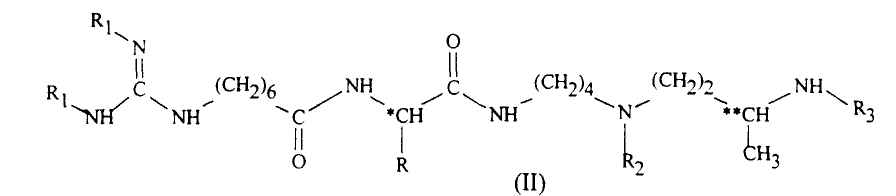 dans laquelle R, R₁, R₂, R₃, *C et **C conservent la même signification que ci-dessus ;
**(c)** selon la variante C :
**(i**_{**a**}**)** faire réagir le 7-azido-heptanamide avec le 2-hydroxy-2-méthoxyacétate de méthyle de formule : 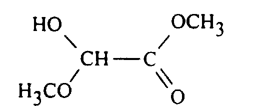 dans un solvant du type hydrocarbure halogéné, en présence d'un agent déshydratant, notamment un tamis moléculaire, à une température comprise entre 25° C et la température de reflux du solvant, pendant 10 à 50 heures, pour obtenir un composé intermédiaire de formule : 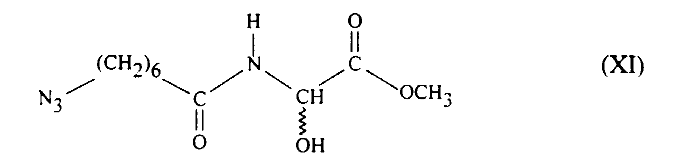
**(i**_{**b**}**)** faire agir, *in situ,* le composé de formule XI, ainsi obtenu, avec du chlorure de thionyle, à une température d'environ 40° C, pendant 1 à 3 heures, pour obtenir le composé halogéné de formule : 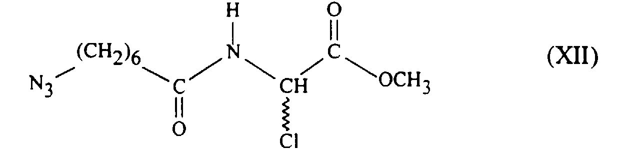
**(i**_{**c**}**)** faire réagir le composé de formule XII, ainsi obtenu, avec un alcool chiral de configuration déterminée (R) ou (S), de type benzylique, en particulier de formule : 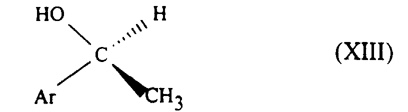 dans laquelle Ar représente un radical aromatique,
dans un solvant du type hydrocarbure halogéné, en présence d'une base, à une température comprise entre 10 et 40° C, pendant 5 à 50 heures, pour obtenir le composé de formule : 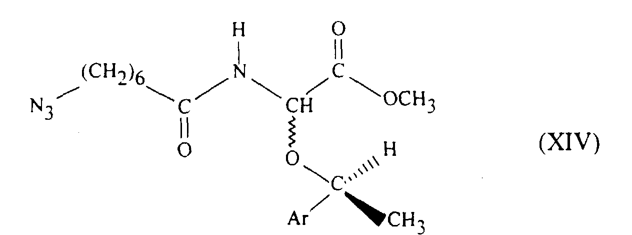 où Ar conserve la même signification que dans le composé de formule XIII
**(ii)** effectuer l'hydrolyse de la fonction ester du composé de formule XIV, ainsi obtenu, par action d'une base en milieu aqueux, dans un solvant du type éther, à une température voisine de la température ambiante, pour obtenir, après acidification, le composé acide de formule : 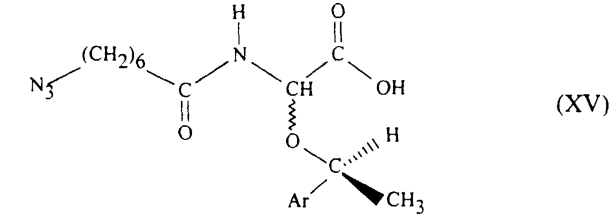 dans laquelle Ar conserve la même signification que ci-dessus ;
**(iii)** faire réagir le composé de formule XV. ainsi obtenu, avec une amine de formule 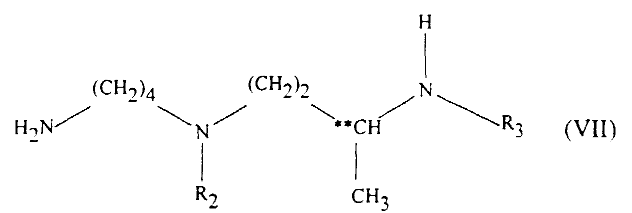 dans laquelle R₂ et R₃ représentent un groupe amino-protecteur sensible à l'hydrogénation, et **C représente un carbone asymétrique de configuration (R,S) ou (R),
dans un solvant, en présence d'au moins un activateur de couplage de type connu dans la synthèse peptidique, à une température proche de la température ambiante, pendant 10 à 75 heures, pour obtenir le composé de formule : 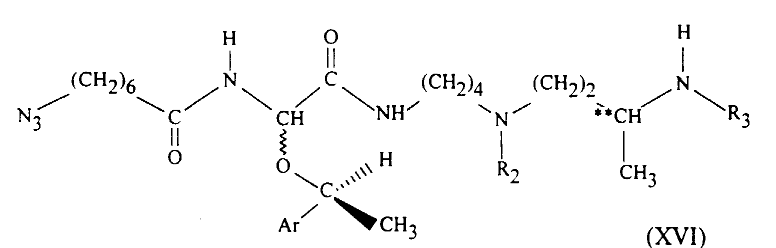 où Ar, R_{2,} R₃ et **C conservent la même signification que ci-dessus ;
**(iv)** effectuer la séparation des isomères du composé de formule XVI, par exemple au moyen d'une chromatographie sur gel de silice, afin d'obtenir séparément l'un et l'autre des deux composés suivants : 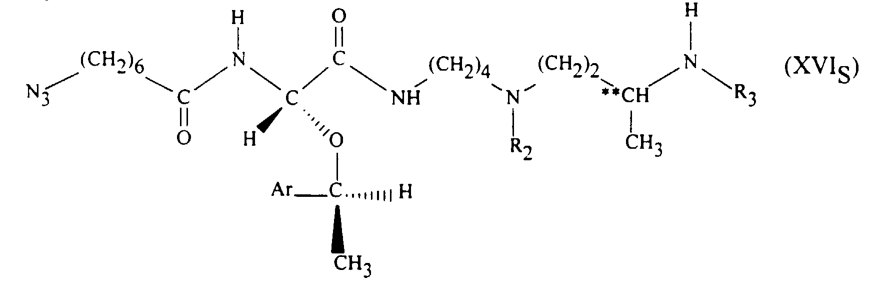 et 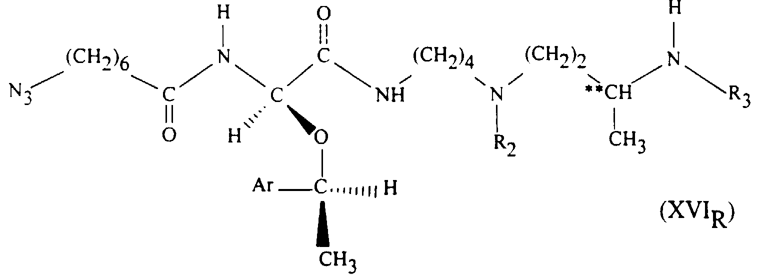 où Ar, R₁, R₂ et R₃ et **C conservent la même signification que ci-dessus ;
**(v)** faire réagir le composé de formule XVI_{S}, ainsi obtenu, avec de la triphénylphosphine, en présence d'eau, dans un solvant anhydre, à une température comprise entre 50 et 70° C, pendant 10 à 30 heures, pour obtenir l'amine intermédiaire correspondante que l'on fait réagir *in situ* avec le composé de formule : 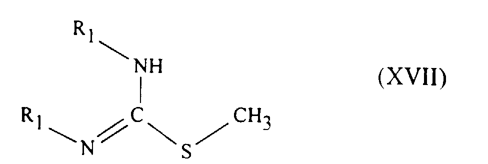 dans laquelle R₁ représente un groupe amino-protecteur, pour obtenir après réaction à température proche de la température ambiante, pendant 10 à 48 h, le composé de formule II : 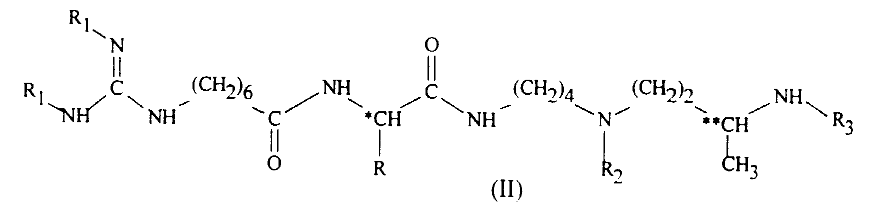 dans laquelle :
R est le groupe OR',
R₁, R₂, R₃ sont des groupes amino-protecteurs de type benzyloxycarbonyle,
R' est un groupe de type benzyle α-méthylé de formule : 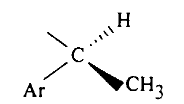
**C est un carbone asymétrique de configuration (R,S) ou (R),
*C est un carbone asymétrique de configuration (S) ; et,
**(d)** selon la variante D :
**(i)** faire appel à un composé de formule XIV, obtenu ci-dessus selon la première étape du procédé de la variante C, et effectuer la séparation de ses deux diastéréoisomères, notamment à l'aide d'une chromatographie sur gel de silice, pour obtenir séparément les deux isomères purs de formules : 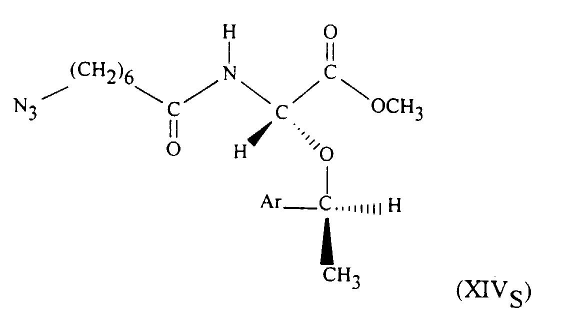 et 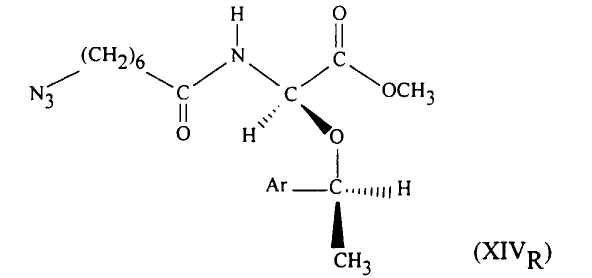 dans lesquelles Ar représente un reste aromatique comme indiqué ci-dessus ;
**(ii)** effectuer l'hydrolyse de la fonction ester du composé XIV_{S}, ainsi obtenu, dans des conditions identiques à celles décrites dans la variante C, pour obtenir l'acide correspondant de formule : 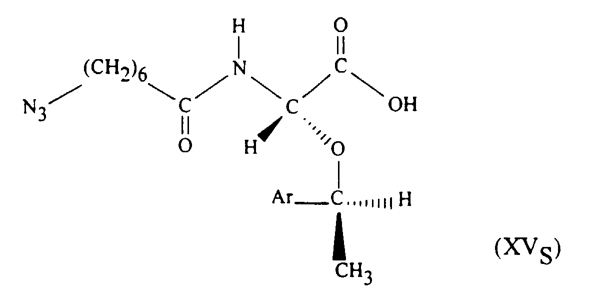 dans laquelle Ar conserve la même signification que ci-dessus ;
**(iii)** faire réagir le composé XV_{S}, ainsi obtenu, avec une amine de formule VII: 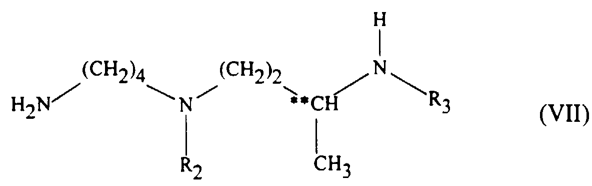 dans laquelle R₂ et R₃ représentent un groupe amino-protecteur sensible à l'hydrogénation, et **C représente un atome de carbone de configuration (R,S) ou (R),
dans des conditions identiques à celles décrites à la variante C, pour obtenir le composé de formule : 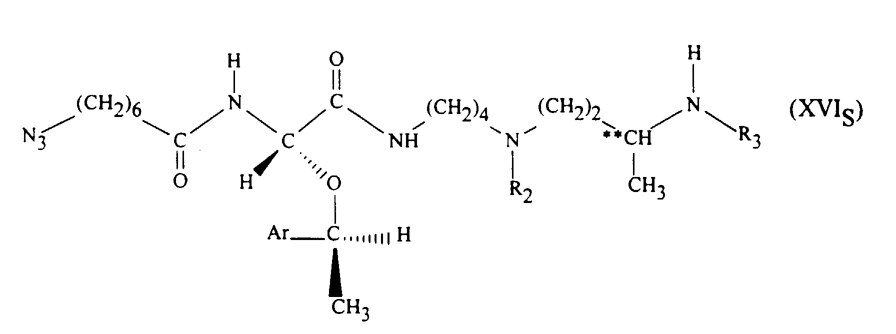 où Ar, R₂, R₃ et **C conservent la même signification que ci-dessus ;
**(iv)** traiter le composé de formule XVI_{S}, ainsi obtenu. de façon analogue à l'étape (v) du procédé selon la variante C pour obtenir le composé de formule II avec les mêmes caractéristiques que dans le cas de ladite variante C.

6. Procédé de préparation d'un composé de formule I dans lequel l'atome de carbone *C est de configuration (R) ou (S) ou de l'un de ses sels d'addition, caractérisé en ce qu'il comprend les mécanismes opératoires consistant à :
- obtenir intermédiairement le mélange de diastéréoisomères de formule : 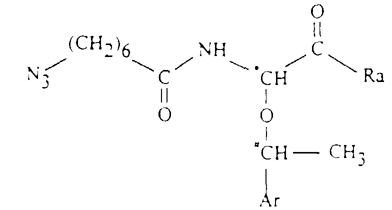 où :
- Ar représente un radical aromatique,
- Ra représente un groupe alcoxy en C₁-C₃ ou un groupe -HN-(CH₂)₄-N(R₂)-(CH₂)₂-CH(CH₃)-NH(R₃) obtenu par réaction de l'amine de formule VII,
- R₁, R_{2,} et R_{3,} identiques ou différents. représentent chacun un groupe amino-protecteur,
- *C représente un atome de carbone de configuration (R,S),
- ^{#}C représente un atome de carbone de configuration (R) ou (S),
et
- séparer les deux isomères à l'aide de méthodes connues de l'homme de l'art, comme par exemple la chromatographie préparative sur gel de silice pour obtenir séparément les composés de formule : 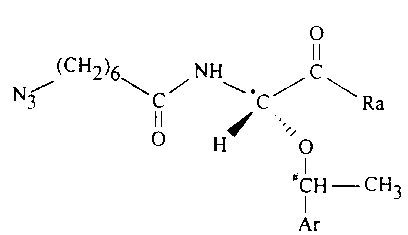 et 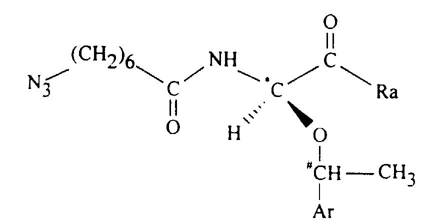 dans lesquelles Ar, Ra et ^{#}C conservent la même signification que ci-dessus.

7. Utilisation du procédé selon la revendication 6 pour l'obtention de l'isomère S de la 15-déoxyspergualine [11-(S)-15-DSG], ladite utilisation étant caractérisée en ce qu'elle met en oeuvre les réactions décrites avec un composé de structure spermidine de formule NH₂-(CH₂)₄-N(R₂)-(CH₂)₃-NH(R₃), à la place de l'amine de formule VII.

8. Composé intermédiaire, utile pour la préparation des composés de formule (I) et/ou des isomères 11-(S)-15-DSG et 11-(R)-15-DSG, caractérisé en ce qu'il répond à la formule 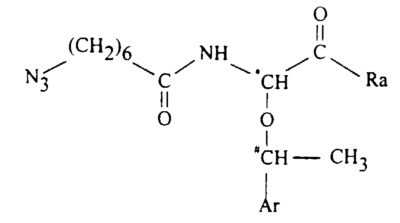 où :
- Ar représente un substituant aromatique,
- Ra représente un groupe alcoxy en C₁-C₃ ou un groupe -HN-(CH₂)₄-N(R₂)-(CH₂)₂-CH(CH₃)-NH(R₃),
- R₁, R₂, et R₃, identiques ou différents, représentent chacun un groupe amino-protecteur, notamment du type benzyloxycarbonyle, et
- ^{#}C représente un atome de carbone de configuration (S) ou (R).

9. Composé intermédiaire, utile dans la synthèse d'un composé de formule (I) selon la revendication 1, caractérisé en ce qu'il est choisi parmi l'ensemble constitué par les composés de formule : 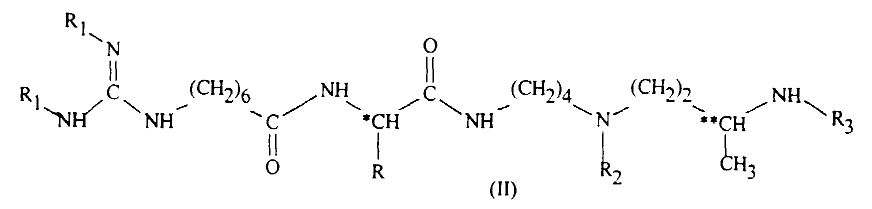 dans laquelle :
- R représente un atome d'hydrogène, un groupe OCH₃, un groupe OH, un groupe CH₂OH, un groupe OR' ou un groupe CH₂OR',
- R' représente un groupe protecteur de la fonction hydroxyle,
- R₁, R₂, R₃, identiques ou différents, représentent chacun un groupe amino-protecteur,
- *C représente, lorsque R n'est pas l'atome d'hydrogène, un atome de carbone asymétrique de configuration (R,S), (R) ou (S),
- **C représente un atome de carbone asymétrique de configuration (R,S) ou (R).

10. Composé intermédiaire, utile dans la synthèse d'un composé de formule I selon la revendication 1, caractérisé en ce qu'il répond à la formule : 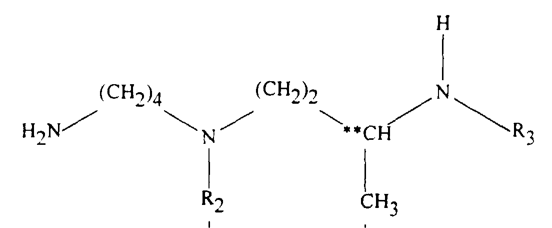 dans laquelle R₂ et R₃ sont identiques et représentent chacun un groupe 1,1-diméthyléthoxycarbonyle (Boc) ou un groupe phénylméthoxycarbonyle (Z) et **C représente un atome de carbone asymétrique de configuration (R,S) ou (R).

11. Utilisation d'une substance immunosuppressive, choisie parmi l'ensemble constitué par les composés de formule I et leurs sels d'addition non-toxiques selon la revendication 1, pour l'obtention d'un médicament destiné à une utilisation en thérapeutique vis-à-vis des désordres immunitaires.

12. Utilisation d'une substance choisie parmi l'ensemble constitué par les composés de formule I et leurs sels d'addition non-toxiques selon la revendication 1, pour l'obtention d'un médicament destiné à une utilisation en thérapeutique vis-à-vis du paludisme.

13. Utilisation d'une substance choisie parmi l'ensemble constitué par les composés de formule I et leurs sels d'addition non-toxiques selon la revendication 1, pour l'obtention d'un médicament destiné à une utilisation en thérapeutique vis-à-vis des maladies inflammatoires hyperréactives, notamment les colites ulcératives ou l'asthme.

14. Utilisation d'une substance choisie parmi l'ensemble constitué par les composés de formule I et leurs sels d'addition selon la revendication 1, en tant que réactif pharmacologique

15. Composition thérapeutique caractérisée en ce qu'elle renferme, en association avec un excipient physiologiquement acceptable, au moins un composé choisi parmi l'ensemble constitué par les composés de formule I et leurs sels d'addition non-toxiques selon la revendication 1.

## Patentansprüche

1. Verbindung aus der Gruppe der 1 5-Desoxyspergualin-Analogen, dadurch gekennzeichnet, daß sie aus der Menge gewählt ist, die folgendes umfaßt:
(I) die Verbindungen mit der allgemeinen Formel: 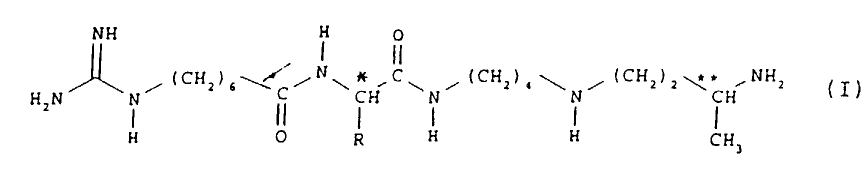 bei welcher
- R ein Wasserstoffatom, eine OH-Gruppe, eine OCH₃-Gruppe oder eine CH₂OH-Gruppe repräsentiert,
- *C in dem Fall, daß R kein Wasserstoffatom ist, ein asymmetrisches Kohlenstoffatom mit der (R, S), (R) oder (S) ist,
- **C ein asymmetrisches Kohlenstoffatom mit der Konfiguration (R, S) oder (R) ist,
und
(II) deren Additionssalze.

2. Verbindung nach Anspruch 1, dadurch gekennzeichnet, daß das asymmetrische Kohlenstoffatom **C eine (R)-Konfiguration aufweist.

3. Verbindung nach Anspruch 1, dadurch gekennzeichnet, daß das asymmetrische Kohlenstoffatom *C eine (S)-Konfiguration aufweist und R die OH-Gruppe ist.

4. . Verfahren zur Herstellung einer Verbindung der allgemeinen Formel oder eines ihrer Additionssalze nach Anspruch 1, dadurch gekennzeichnet, daß es die folgenden Schritte umfaßt:
(I) Aufheben des Schutzes einer Verbindung der allgemeinen Formel II: 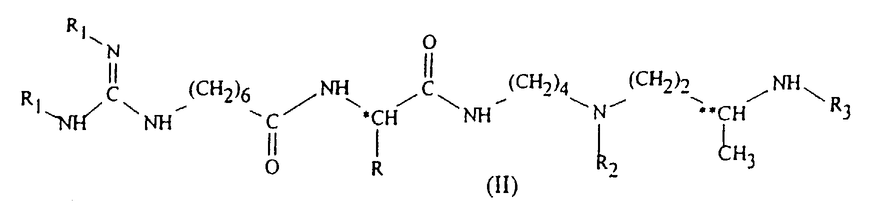 bei welcher:
- R ein Wasserstoffatom, eine OCH₃-Gruppe, eine OH-Gruppe, eine CH₂OH-Gruppe, eine OR'-Gruppe oder eine CH₂OR'-Gruppe repräsentiert,
- R' eine Schutzgruppe der Hydroxylfunktion darstellt,
- R₁, R₂, R₃ jeweils identisch oder unterschiedlich sind und eine Schutzgruppe der Aminfunktion repräsentieren,
- C in dem Fall, daß R kein Wasserstoffatom ist, ein asymmetrisches Kohlenstoffatom mit der Konfiguration (R, S), (R) oder (S) ist,
- C ein asymmetrisches Kohlenstoffatom mit der Konfiguration (R, S) oder (R) ist, nach einer oder mehrerer dem Fachmann bekannter Reaktionsbehandlungen zur Herbeiführung des Austausches aller Schutzgruppen R₁, R₂, R₃ und R' gegen ein Wasserstoffatom, und
(II) gegebenenfalls Erhalten der Verbindung der allgemeinen Formel I in Form einer freien Base aus einem nach Arbeitsschritt (I) erhaltenen Additionssalz durch Einwirkung einer starken Base und anschließendes Erhalten der anderen Additionssalze aus dieser Base.

5. Verfahren nach Anspruch 4, dadurch gekennzeichnet, daß es auch die folgenden Schritte umfaßt:
(a) entsprechend Variante A:
(I) Kondensieren einer Säure der allgemeinen Formel: 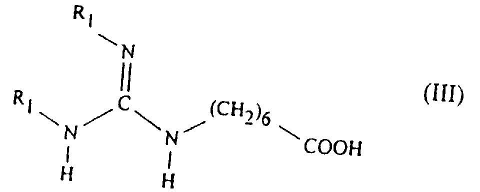 bei welcher R₁ eine Aminoschutzgruppe mit einem Aminosäurederivat der allgemeinen Formel: 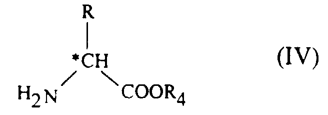 repräsentiert, in welcher:
- R ein Wasserstoffatom oder eine CH₂OR'-Gruppe darstellt,
- R' eine Schutzgruppe der Hydroxylfunktion bezeichnet,
- R₄ eine Alkylgruppe mit C₁-C₃ repräsentiert, und
- *C in dem Fall, daß R kein Wasserstoffatom ist, ein asymmetrisches Kohlenstoffatom mit der (R, S), (R) oder (S) ist,
durch Aktivieren der Säurefunktion mit Hilfe eines Kopplungsmittels vom Carbodiimid-Typus unter Anwesenheit eines nukleophilen Mittels in einem organischen Lösungsmittel bei einer Temperatur zwischen 0 und 40 ° und in einer Menge von 1 Mol der Verbindung III auf etwa 1 Mol der Verbindung IV, um eine Verbindung der allgemeinen Formel: 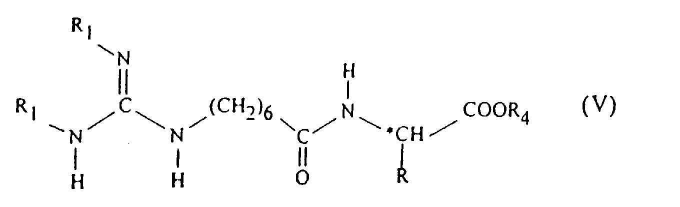 zu erhalten, bei welcher R, R₁, R₄ und *C dieselbe Bedeutung wie bei der Verbindung der allgemeinen Formel V behalten und R ein Wasserstoffatom, eine CH₂OR'-Gruppe oder eine OR'-Gruppe darstellt, wobei R' eine Schutzgruppe der Hydroxylfunktion repräsentiert;
(III) Reagieren einer Verbindung der so erhaltenen allgemeinen Formel VI mit einer Verbindung der allgemeinen Formel: 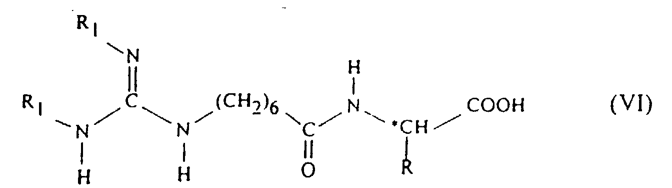 bei welcher:
- R₂ und R₃ jeweils identisch oder unterschiedlich sind und eine Schutzgruppe der Aminfunktion repräsentieren, und
- **C ein asymmetrisches Kohlenstoffatom mit der Konfiguration (R, S) oder (R) ist, unter identischen Bedingungen wie vorstehend in Schritt (I) angegeben, um eine Verbindung der allgemeinen Formel II zu erhalten: 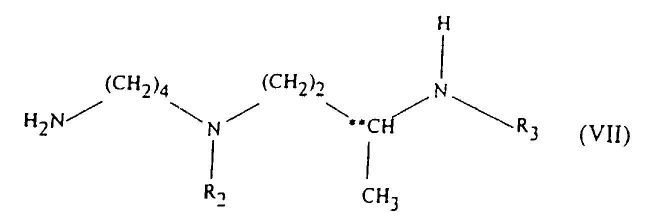 bei welcher R, R₁, R₂, R₃, *C und **C dieselbe Bedeutung wie vorstehend behalten, und **C einen asymmetrischen Kohlenstoff mit der Konfiguration (R,S) oder (R) darstellt, unter identischen Bedingungen wie jene, die vorstehend in Schritt (1) beschrieben sind, um eine Verbindung der allgemeinen Formel II 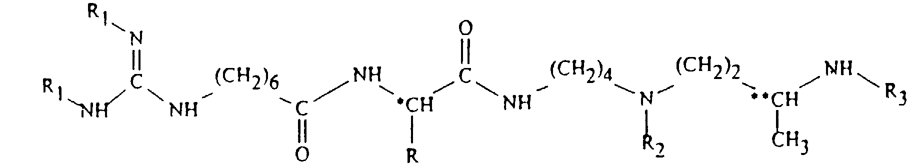 zu erhalten, bei welcher R, R₁, R₂, R₃, *C und **C dieselbe Bedeutung wie vorstehend behalten.
(b) entsprechend Variante B:
(I) Kondensieren eines Aminosäurederivats der allgemeinen Formel: 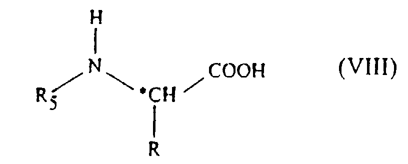 bei welcher
- R ein Wasserstoffatom oder eine CH₂OR'-Gruppe darstellt, wobei R' eine Schutzgruppe der Hydroxylfunktion bezeichnet,
- R₅ eine Schutzgruppe der Aminfunktion repräsentiert, und
- *C in dem Fall, daß R kein Wasserstoffatom ist, ein asymmetrisches Kohlenstoffatom mit der (R, S), (R) oder (S) ist,
mit einer Verbindung der allgemeinen Formel: 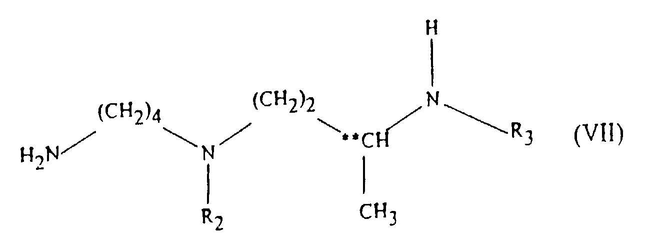 bei welcher:
- R₂ und R₃ jeweils identisch oder unterschiedlich sind, eine Schutzgruppe der Aminfunktion repräsentieren, und beide von der in der Verbindung der allgemeinen Formel VIII vorhandenen Schutzgruppe R₅ verschieden sind, und
- **C ein asymmetrisches Kohlenstoffatom mit der Konfiguration (R, S) oder (R) ist, unter identischen Bedingungen wie vorstehend in Schritt (I) bei der Variante A angegeben, um eine Verbindung der allaemeinen Formel II zu erhalten: 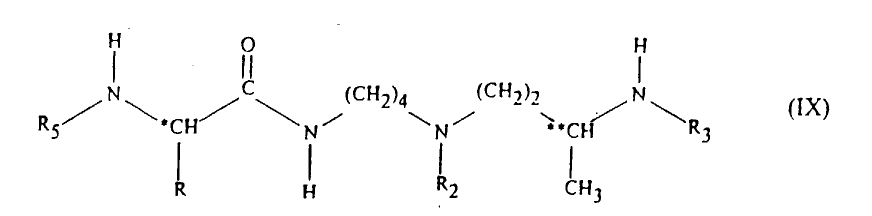
bei welcher R, R₂, R₃, R₅, *C und **C dieselbe Bedeutung wie vorstehend behalten;
(II) Aufheben des Schutzes der so erhaltenen Verbindung IX nach einem speziellen Verfahren mit Spaltung der Verbindung N-R₅, um eine Verbindung der allgemeinen Formel: 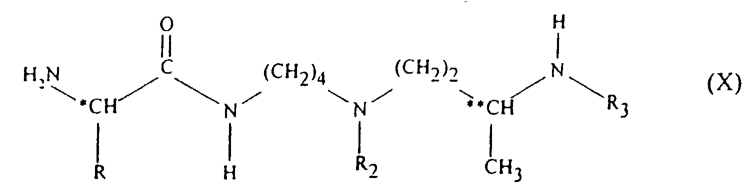 zu erhalten, bei welcher R, R₂, R₃, R₅, *C und **C dieselbe Bedeutung wie vorstehend behalten;
(III) Kondensieren des so erhaltenen Aminprodukts der allgemeinen Formel X mit einer Säure der allgemeinen Formel: 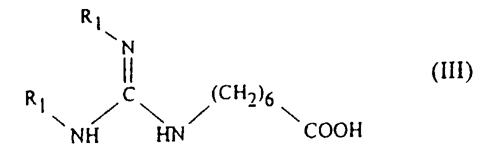 bei welcher R₁ eine Aminoschutzgruppe darstellt,
unter analogen Arbeitsbedingungen wie vorstehend für das Verfahren (I) gemäß Variante A beschrieben, um eine Verbindung der allgemeinen Formel II 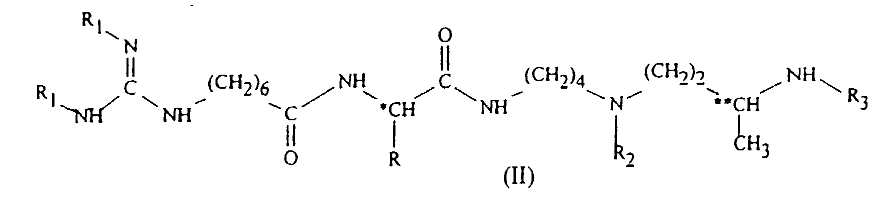 zu erhalten, bei welcher R, R₂, R₃, R₅, *C und **C dieselbe Bedeutung wie vorstehend behalten;
(c) entsprechend Variante C:
(Iₐ) Umsetzen von 7-Acidoheptanamid mit Methyl-2-Hydroxy-2-Methoxyacetat der allgemeinen Formel: 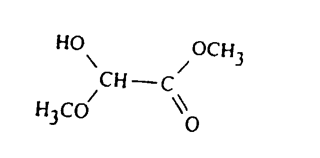 in einem Lösungsmittel vom Typ eines halogenierten Kohlenwasserstoffs unter Anwesenheit eines Trocknungsmittels, insbesondere eines Molekularsiebs, 10 bis 50 Stunden lang bei einer Temperatur zwischen 25 °C und der Rückflußtemperatur des Lösungsmittels, um eine Zwischenverbindung der allgemeinen Formel: 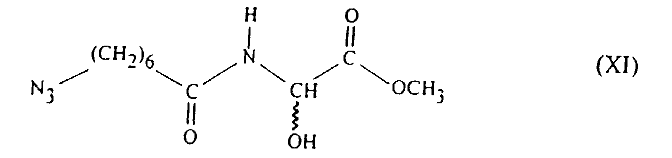 zu erhalten;
(I_{b}) Umsetzen in situ der so erhaltenen Verbindung der allgemeinen Formel Xl mit Thionylchlorid 1 bis 3 Stunden lang bei einer Temperatur von etwa 40 °C, um die halogenierte Verbindung der allgemeinen Formel: 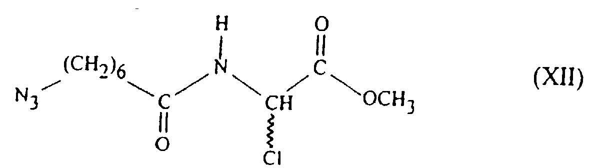 zu erhalten,
(I_{c}) Umsetzen der so erhaltenen Verbindung der allgemeinen Formel XII mit einem Chiralkol mit vorgegebener (R)- oder (S)-Konfiguration vom Benzyltyp, der insbesondere folgender allgemeinen Formel entspricht: 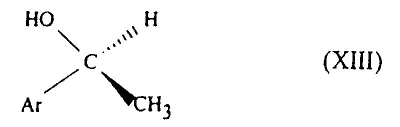 wobei Ar ein aromatisches Radikal darstellt,
in einem Lösungsmittel vom Typ eines halogenierten Kohlenwasserstoffs in Anwesenheit einer Basis 5 bis 50 Stunden lang bei einer Temperatur zwischen 10 und 40 °C, um die Verbindung der allgemeinen Formel: 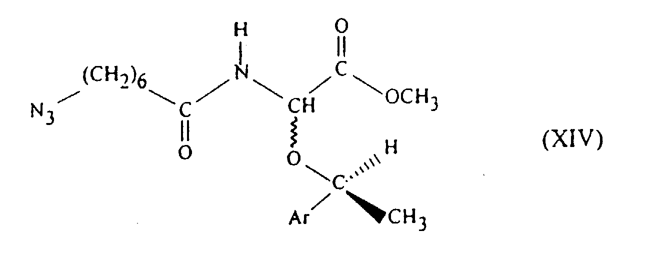 zu erhalten, bei welcher Ar dieselbe Bedeutung wie bei der Verbindung der allgemeinen Formel XIII behält,
(II) Vornehmen der Hydrolyse der Esterfunktion der so erhaltenen Verbindung der allgemeinen Formel XIV durch Einwirkung einer Base in wäßrigem Medium in einem Lösungsmittel vom Ethertyp bei einer Temperatur nahe der Umgebungstemperatur, um nach Säuerung die saure Verbindung der allgemeinen Formel: 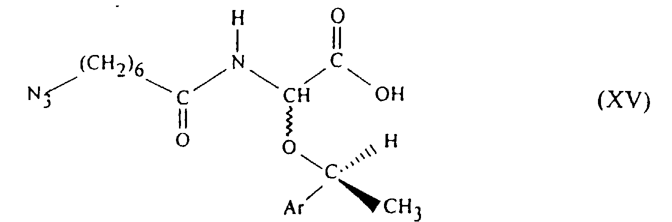 zu erhalten, bei welcher Ar dieselbe Bedeutung wie vorstehend angegeben behält;
(III) Umsetzen der so erhaltenen Verbindung der allgemeinen Formel XV mit einem Amin der allgemeinen Formel: 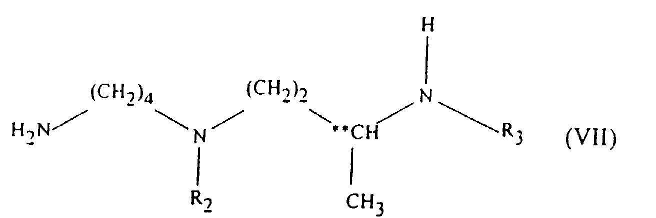 bei welcher R₂, und R₃ eine gegenüber Hydrierung empfindliche Aminoschutzgruppe darstellen und **C dieselbe ein asymmetrisches Kohlenstoffatom mit der Konfiguration (R, S) oder (R) repräsentiert,
in einem Lösungsmittel in Anwesenheit mindestens eines Kopplungsaktivators von dem von der Peptidsynthese her bekannten Typ 10 bis 75 Stunden lang bei einer Temperatur nahe der Umgebungstemperatur, um die Verbindung der allgemeinen Formel: 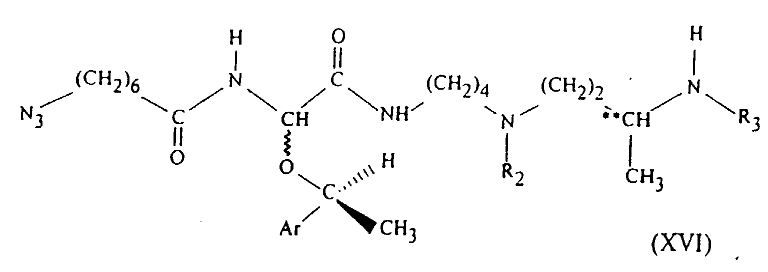 zu erhalten, bei welcher Ar, R₂, R₃ und **C dieselbe Bedeutung wie vorstehend behalten;
(IV) Vornehmen der Trennung der Isomeren der Verbindung der allgemeinen Formel XVI, beispielsweise mit Hilfe der Siliziumgel-Chromatographie, um voneinander getrennt die beiden folgenden Verbindungen zu erhalten: 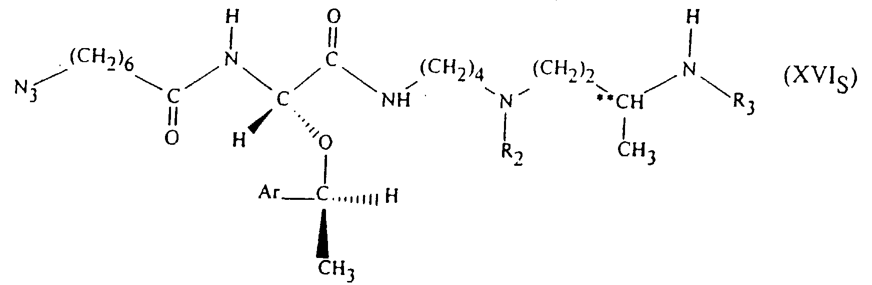 und 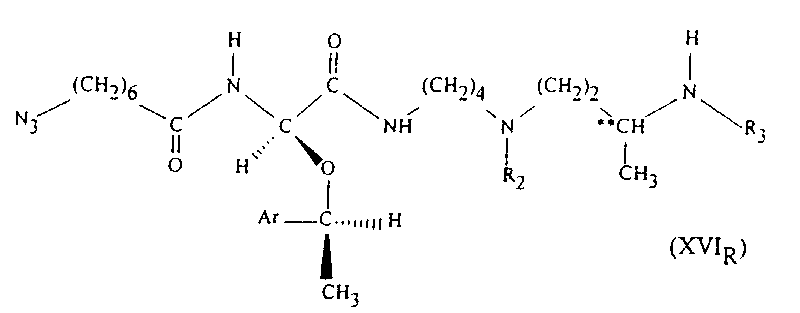 bei welchen Ar, R₂, R₃ und **C dieselbe Bedeutung wie vorstehend behalten;
(V) Umsetzen der so erhaltenen Verbindung der allgemeinen Formel XVIₛ mit Triphenylphosphin in Anwesenheit von Wasser in einem wasserfreien Lösungsmittel 10 bis 30 Stunden lang bei einer Temperatur zwischen 50 und 70 °C, um das entsprechende Zwischenamin zu erhalten, das in situ mit der Verbindung der allgemeinen Formel: 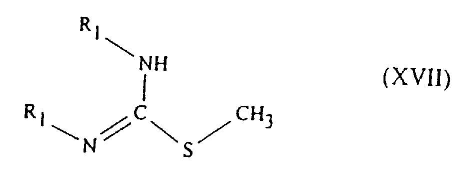 umgesetzt wird, bei welcher R₁ eine Aminoschutzgruppe repräsentiert, um nach 10 bis 48-stündiger Umsetzung bei einer Temperatur nahe der Umgebungstemperatur die Verbindung der allgemeinen Formel II: 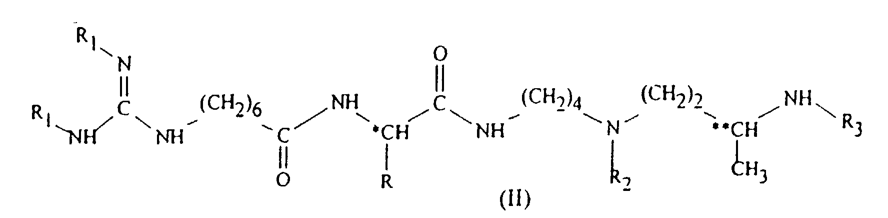 zu erhalten, bei welcher:
- R die OR'-Gruppe ist,
- R₁, R₂, R₃ Aminoschutzgruppen vom Typus Benzyloxycarbonyl sind,
- R' eine Gruppe vom Typ Benzyl-α-Methyl der allgemeinen Formel: 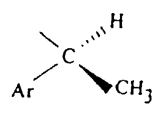
- ist,
- **C ein asymmetrisches Kohlenstoffatom mit der Konfiguration (R, S) oder (R) ist,
- *C ein asymmetrisches Kohlenstoffatom mit der Konfiguration (S) ist, und
(d) entsprechend Variante D:
(I) Einsetzen einer nach der vorstehend beschriebenen ersten Stufe des Verfahrens gemäß Variante C erhaltenen Verbindung der allgemeinen Formel XIV und Vornahme der Trennung ihrer beiden Diastereoisomeren, insbesondere mittels der Siliziumgel-Chromatographie, um die beiden reinen Isomeren der Formel: 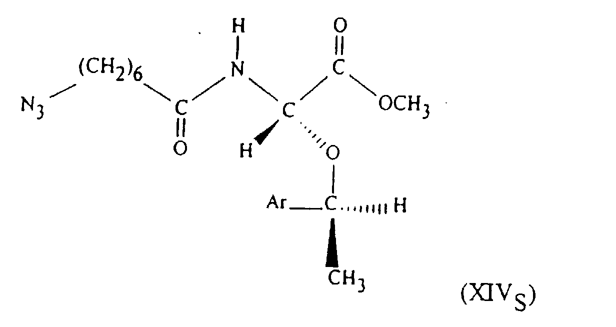 und 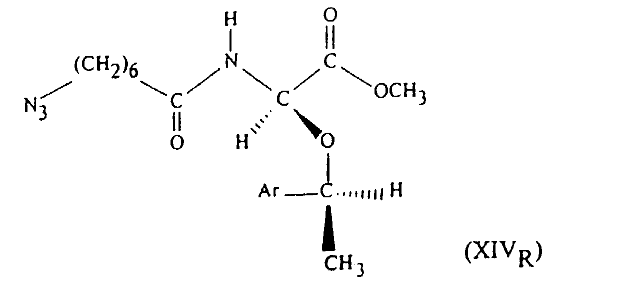 getrennt zu erhalten, bei welchen Ar einen aromatischen Rest wie vorstehend angegeben repräsentiert;
(II) Vornahme der Hydrolyse der Esterfunktion der so erhaltenen Verbindung XIVₛ unter identischen Bedingungen wie jene, die bei Variante C beschrieben sind, um die entsprechende Säure der allgemeinen Formel 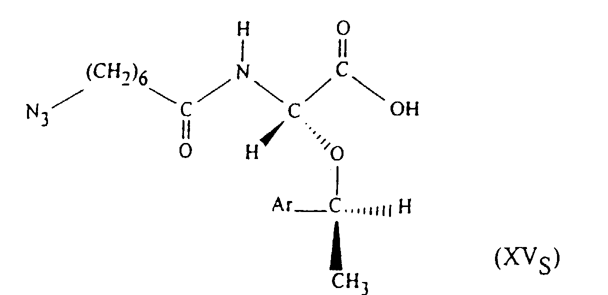 zu erhalten, bei welcher Ar dieselbe Bedeutung wie vorstehend angegeben behält;
(III) Umsetzen der so erhaltenen Verbindung XVₛ mit einem Amin der allgemeinen Formel VII: 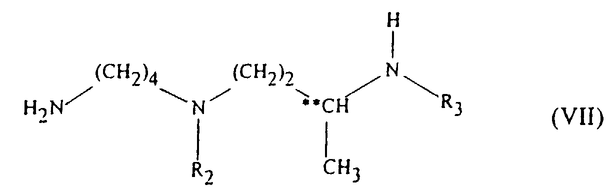 bei welcher R₂, und R₃ eine gegenüber Hydrierung empfindliche Aminoschutzgruppe darstellen und C dieselbe ein Kohlenstoffatom mit der Konfiguration (R, S) oder (R) repräsentiert,
unter identischen Bedingungen wie jene, die bei Variante C beschrieben sind, um die Verbindung der allgemeinen Formel: 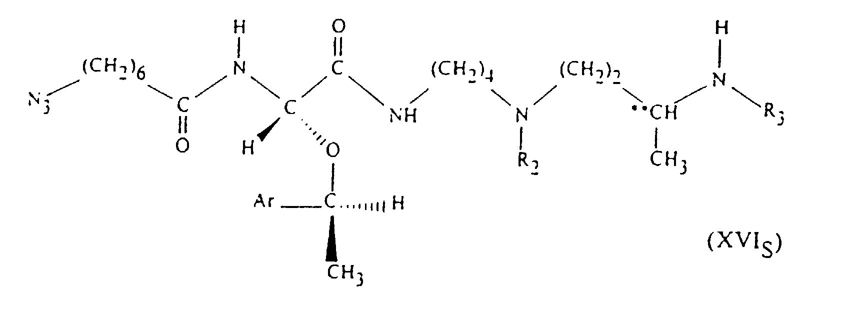 zu erhalten, bei welcher Ar, R₂, R₃ und **C dieselbe Bedeutung wie vorstehend behalten;
(IV) Behandeln der so erhaltenen Verbindung der allgemeinen Formel XVIₛ in analoger Weise wie bei Schritt (V) des Verfahrens gemäß Variante C, um die Verbindung der allgemeinen Formel II mit denselben Merkmalen wie im Fall der Variante C zu erhalten.

6. Verfahren zur Herstellung einer Verbindung der allgemeinen Formel I, bei welchem das Kohlenstoffatom *C die Konfiguration (R) oder (S) besitzt, oder eines ihrer Additionssalze, dadurch gekennzeichnet, daß es die folgenden Arbeitsmechanismen umfaßt:
- Erhalten einer Zwischenstufe in Form des Gemisches der Diastereoisomeren der allgemeinen Formel: 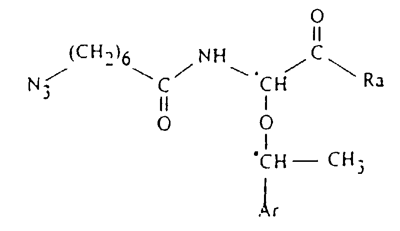 wobei:
- Ar ein aromatisches Radikal repräsentiert,
- Ra eine Alkoxygruppe mit C₁-C₃ oder eine -HN-(CH₂)₄-N(R₂)-(CH₂)₂-CH(CH₃)-NH(R₃)-Gruppe ist, welche durch Umsetzung des Amins der allgemeinen Formel VII erhalten wird,
- R₁, R₂, und R₃ jeweils identisch oder unterschiedlich sind und eine Aminoschutzgruppe darstellen,
- *C ein Kohlenstoffatom mit der Konfiguration (R,S) repräsentiert, und
- ^{#}C ein Kohlenstoffatom mit der Konfiguration (R) oder (S) ist,
und
- Trennen der beiden Isomeren mit Hilfe der dem Fachmann bekannten Verfahrensweisen, beispielsweise der präparativen Siliziumgel-Chromatographie, um die Verbindungen der allgemeinen Formel: 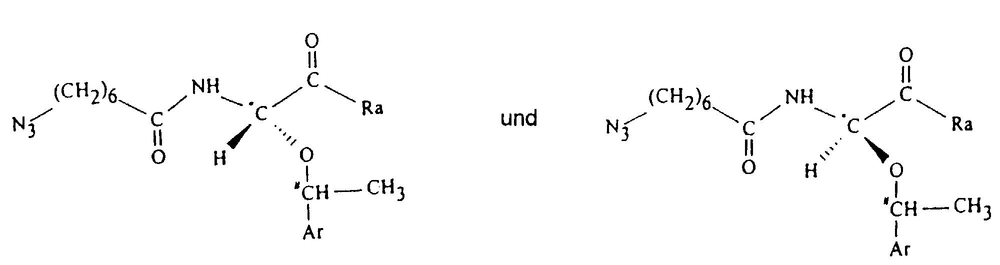 getrennt zu erhalten, wobei Ar, Ra und ^{#}C die gleiche Bedeutung wie vorstehend angegeben behalten.

7. Anwendung des Verfahrens nach Anspruch 6 zum Erhalten des S-lsomeren von 15-Desoxyspergualin [11-(S)-15-DSG], dadurch gekennzeichnet, daß die beschriebenen Reaktionen mit einer Verbindung von Spermidinstruktur der allgemeinen Formel NH₂-(CH₂)₄-N(R₂)-(CH₂)₃-NH(R₃) anstelle des Amins der allgemeinen Formel VIII vorgenommen werden.

8. Zur Herstellung von Verbindungen der allgemeinen Formel (I) und/oder ihrer Isomeren 11-(S)-15-DSG und 11-(R)-15-DSG geeignete Zwischenverbindung, dadurch gekennzeichnet, daß sie der allgemeinen Formel 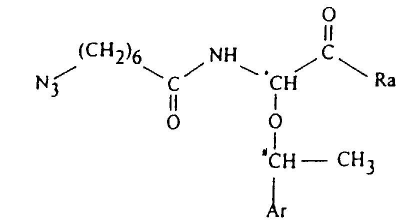 entspricht, bei welcher
- Ar einen aromatischen Substituenten repräsentiert,
- Ra eine Alkoxygruppe mit C₁-C₃ oder eine -HN-(CH₂)₄-N(R₂)-(CH₂)₂-CH(CH₃)-NH(R₃)-Gruppe ist,
- R₁, R₂, und R₃ jeweils identisch oder unterschiedlich sind und eine Aminoschutzgruppe darstellen, insbesondere vom Typ Benzyloxycarbonyl, und
- ^{#}C ein Kohlenstoffatom mit der Konfiguration (S) oder (R) ist.

9. Zur Synthese einer Verbindung der allgemeinen Formel (I) nach Anspruch 1 geeignete Zwischenverbindung, dadurch gekennzeichnet, daß sie aus der Menge der Verbindungen der allgemeinen Formel: 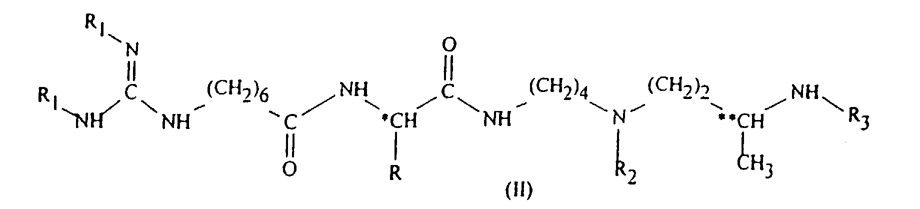 gewählt wird, bei welcher
- R ein Wasserstoffatom, eine OCH₃-Gruppe, eine OH-Gruppe, eine CH₂OH-Gruppe, eine OR'-Gruppe oder eine CH₂OR'-Gruppe repräsentiert,
- R' eine Schutzgruppe für die Hydroxylfunktioin darstellt,
- R₁, R₂, R₃ jeweils identisch oder unterschiedlich sind und eine Aminoschutzgruppe darstellen,
- *C in dem Fall, daß R kein Wasserstoffatom ist, ein asymmetrisches Kohlenstoffatom mit der (R, S), (R) oder (S) ist,
- **C ein asymmetrisches Kohlenstoffatom mit der Konfiguration (R, S) oder (R) ist.

10. Zur Synthese einer Verbindung der allgemeinen Formel (I) nach Anspruch 1 geeignete Zwischenverbindung, dadurch gekennzeichnet, daß sie der allgemeinen Formel: 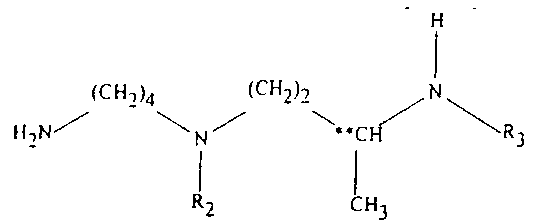 entspricht, bei welcher R₂ und R₃ identisch sind und jeweils eine 1,1-Dimethylethoxycarbonyl-Gruppe (Boc) oder eine Phenylmethoxycarbonyl-Gruppe (Z) repräsentieren und **C für ein asymmetrisches Kohlenstoffatom mit der Konfiguration (R, S) oder (R) steht.

11. Verwendung einer Substanz zur Immunsuppression, welche aus der Menge der Verbindungen der allgemeinen Formel I und ihrer nicht-toxischen Additionssalze nach Anspruch 1 gewählt ist, zur Herstellung eines Medikaments, das zur Anwendung in der Therapie bei Immunstörungen bestimmt ist.

12. Verwendung einer aus der Menge der Verbindungen der allgemeinen Formel I und ihrer nicht-toxischen Additionssalze nach Anspruch 1 gewählten Substanz zur Herstellung eines Medikaments, das zur Anwendung in der Therapie bei Paludismus bestimmt ist.

13. Verwendung einer aus der Menge der Verbindungen der allgemeinen Formel I und ihrer nicht-toxischen Additionssalze nach Anspruch 1 gewählten Substanz zur Herstellung eines Medikaments, das zur Anwendung in der Therapie bei hyperreaktiv entzündlichen Erkrankungen, vornehmlich Colitis mit Geschwürsbildung oder Asthma bestimmt ist.

14. Verwendung einer aus der Menge der Verbindungen der allgemeinen Formel I und ihrer nicht-toxischen Additionssalze nach Anspruch 1 gewählten Substanz als pharmakologisches Reagens.

15. Zusammensetzung zu therapeutischen Zwecken, dadurch gekennzeichnet, daß sie in Verbindung mit einem physiologisch verträglichen Arzneimittelträger mindestens eine aus der Menge der Verbindungen der allgemeinen Formel I und ihrer nicht-toxischen Additionssalze nach Anspruch 1 gewählten Substanz umfaßt.

## Claims

1. A compound belonging to the family of the 15-deoxypergualin analogs, characterized in that it is selected from the group consisting of:
(i) the compounds of the formula 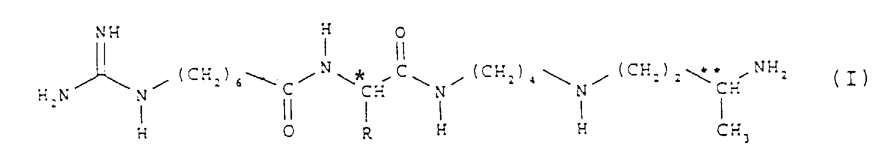 in which:
- R is a hydrogen atom, a group OH, a group OCH₃ or a group CH₂OH,
- *C, in the case where R is not a hydrogen atom, is an asymmetric carbon atom of (R,S), (R) or (S) configuration, and
- **C is an asymmetric carbon atom of (R,S) or (R) configuration; and
(ii) their addition salts.

2. A compound according to claim 1 characterized in that the asymmetric carbon atom **C is of (R) configuration.

3. A compound according to claim 1 characterized in that the asymmetric carbon atom *C is of (S) configuration and R is the group OH.

4. A method of preparing a compound of formula I or one of its addition salts according to claim 1, said method being characterized in that it comprises the steps which consist in:
(i) deprotecting a compound of formula II: 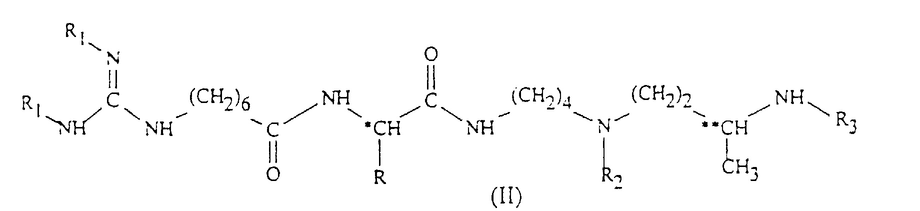 in which:
- R is a hydrogen atom, a group OCH₃, a group OH, a group CH₂OH, a group OR' or a group CH₂OR',
- R' is a protecting group for the hydroxyl group,
- R₁, R₂ and R₃, which are identical or different, are each a protecting group for the amine group,
- *C, when R is not a hydrogen atom, is an asymmetric carbon atom of (R,S), (R) or (S) configuration, and
- **C is an asymmetric carbon atom of (R,S) or (R) configuration,
by one or more reaction treatments known to those skilled in the art, in order to replace all the protecting groups R₁, R₂, R₃ and R' with a hydrogen atom, and, if necessary,
(ii) using an addition salt obtained according to step (i) to obtain the compound of formula I in the form of the free base by reaction with a strong base, and then using said free base to obtain the other addition salts.

5. A method according to claim 4 characterized in that it also comprises the steps which consist in:
(a) according to variant A:
(i) condensing an acid of the formula 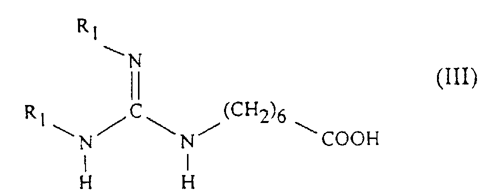 in which R₁ is an amino-protecting group, with an amino acid derivative of the formula 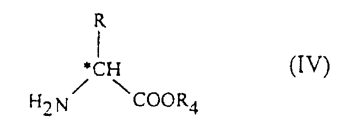 in which:
- R is a hydrogen atom or a group CH₂OR',
- R' is a protecting group for the hydroxyl group,
- R₄ is a C₁-C₃-alkyl group, and
- *C, when R is not the hydrogen atom, is an a symmetric carbon atom of (R,S), (R) or (S) configuration,
by activating the acid group with a coupling agent of the carbodiimide type, in the presence of a nucleophilic agent, in an organic solvent, at a temperature between 0 and 40°C, at a rate of 1 mol of compound III to about 1 mol of compound IV, to give a compound of the formula 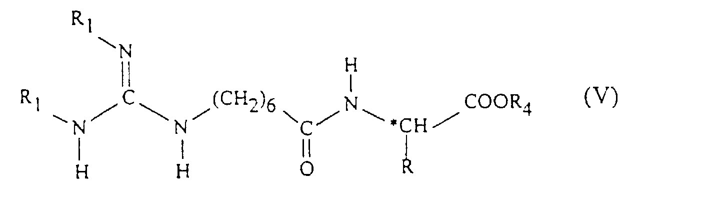 in which R, R_{1,} R₄ and *C are as defined above;
(ii) hydrolyzing the ester group of a resulting compound of formula V,
- either according to step (i) above when R is the hydrogen atom or a group CH₂OR' as described above,
- or by a known method when R is a group OR', where R' is a protecting group for the hydroxyl group, especially Si(CH₃)₂C(CH₃)₃,
by reaction with a dilute base, in the presence of a water-miscible solvent, at a temperature around room temperature (5-40°C), for about 2 to 30 minutes, to give a compound of the formula 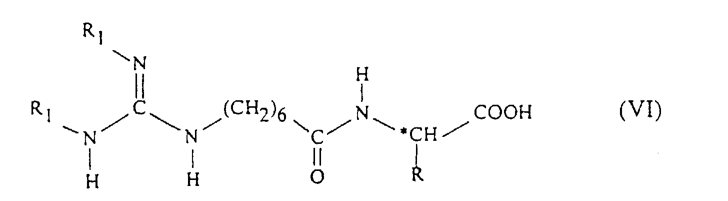 in which R₁ and *C are as defined in the compound of formula V and R is a hydrogen atom, a group CH₂OR' or a group OR', R' being a protecting group for the hydroxyl group; and
(iii) reacting a resulting compound of formula VI with a compound of the formula 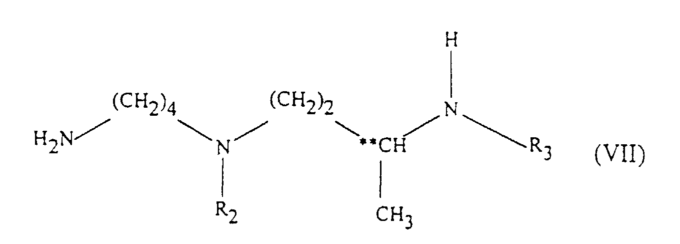 in which:
- R₂ and R₃, which are identical or different, are each an amino-protecting group, and
- **C is an asymmetric carbon of (R,S) or (R) configuration,
under conditions identical to those described in step (i) above, to give a compound of formula II: 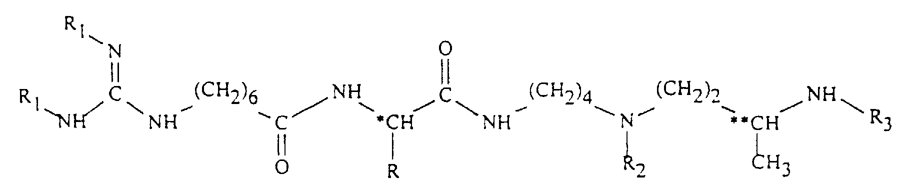 in which R, R₁, R₂, R₃, *C and **C are as defined above;
(b) according to variant B:
(i) condensing an amino acid derivative of the formula 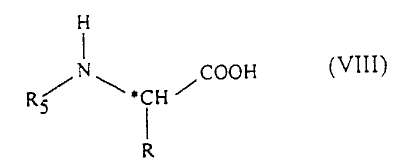 in which:
- R is a hydrogen atom or a group CH₂OR', where R' is a protecting group for the hydroxyl group,
- R₅ is a protecting group for the amine group, and
- *C, when R is not the hydrogen atom, is an asymmetric carbon atom of (R,S), (R) or (S) configuration,
with a compound of the formula 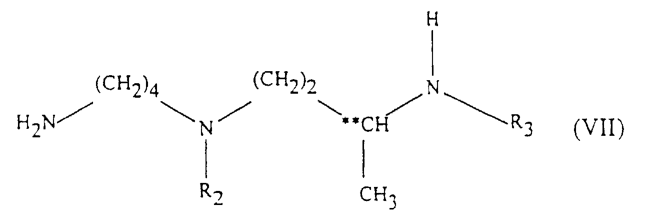 in which:
- R₂ and R₃, which are identical or different, are each an amino-protecting group, both being different from the protecting group R₅ present in the compound of formula VIII, and
- **C is an asymmetric carbon atom of (R,S) or (R) configuration,
under conditions analogous to those of the method of step (i) of variant A above, to give a compound of the formula 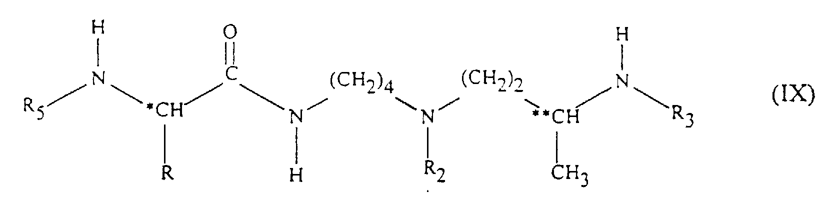 in which R, R₂, R₃, R₅, *C and **C are as defined above;
(ii) deprotecting the resulting compound IX by a specific method for scission of the N-R₅ bond to give a compound of the formula 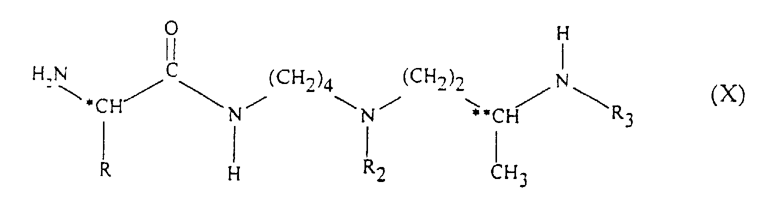 in which R, R₂, R₃, *C and **C are as defined above; and
(iii) condensing the resulting amine product of formula X with an acid of the formula 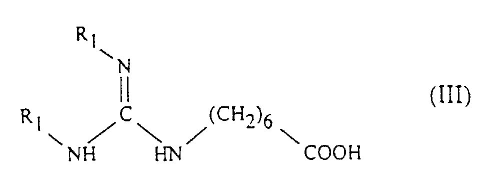 in which R₁ is an amino-protecting group, under operating conditions analogous to those described for method (i) of variant A, to give a compound of formula II: 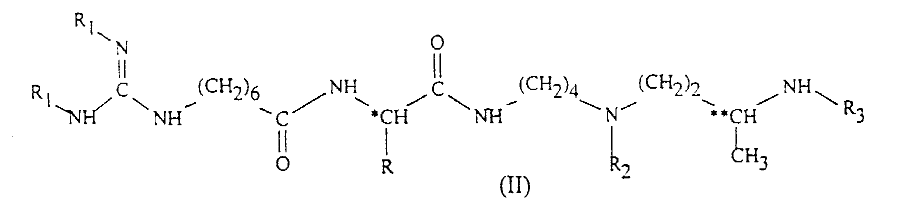 in which R, R₁, R₂, R₃, *C and **C are as defined above;
(c) according to variant C:
(iₐ) reacting 7-azidoheptanamide with methyl 2-hydroxy-2-methoxyacetate of the formula 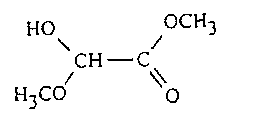 in a solvent of the halogenated hydrocarbon type, in the presence of a dehydrating agent, especially a molecular sieve, at a temperature between 25°C and the reflux temperature of the solvent, for 10 to 50 hours, to give an intermediate of the formula 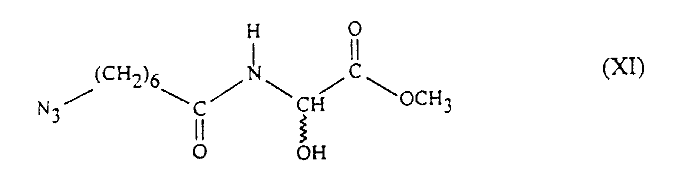
(i_{b}) reacting the resulting compound of formula XI *in situ* with thionyl chloride, at a temperature of about 40°C, for 1 to 3 hours, to give the halogenated compound of the formula 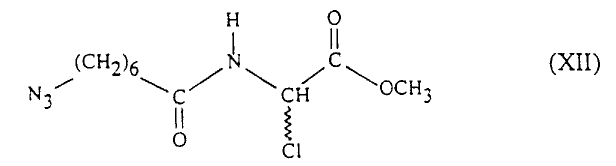
(i_{c}) reacting the resulting compound of formula XII with a chiral alcohol of determined (R) or (S) configuration, of the benzyl type, in particular of the formula 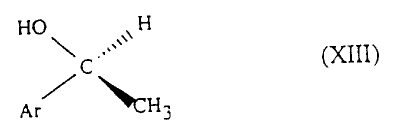 in which Ar is an aromatic radical, in a solvent of the halogenated hydrocarbon type, in the presence of a base, at a temperature between 10 and 40°C, for 5 to 50 hours, to give the compound of the formula 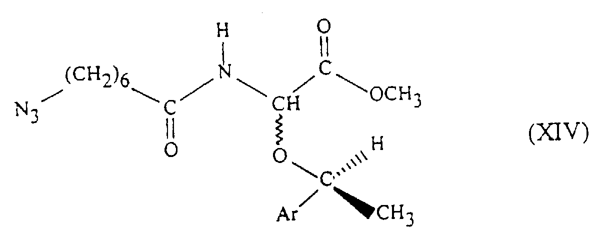 where Ar is as defined in the compound of formula XIII;
(ii) hydrolyzing the ester group of the resulting compound of formula XIV by reaction with a base in an aqueous medium, in a solvent of the ether type, at a temperature around room temperature, to give, after acidification, the acid compound of the formula 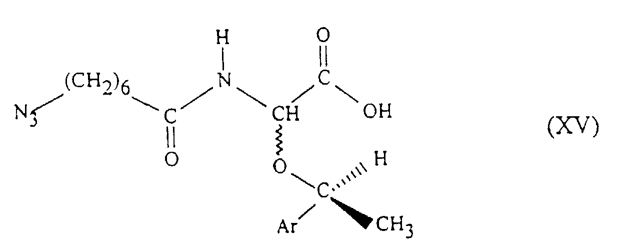 in which Ar is as defined above;
(iii) reacting the resulting compound of formula XV with an amine of the formula 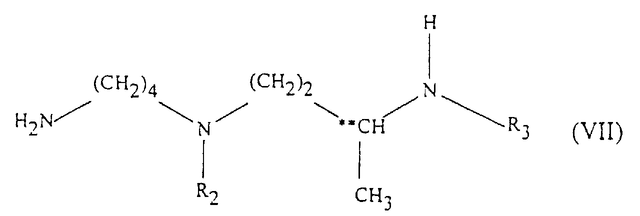 in which R₂ and R₃ are an amino-protecting group sensitive to hydrogenation and **C is an asymmetric carbon of (R,S) or (R) configuration, in a solvent, in the presence of at least one coupling activator of a type known in peptide synthesis, at a temperature close to room temperature, for 10 to 75 hours, to give the compound of the formula 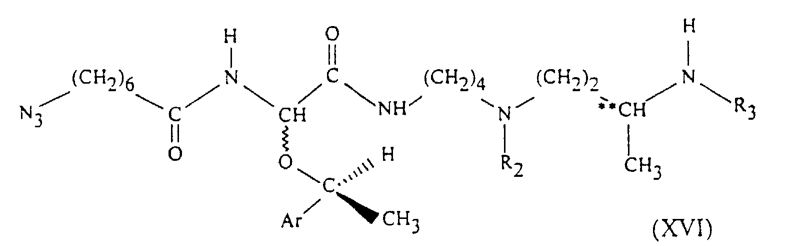 where Ar, R₂, R₃ and **C are as defined above;
(iv) separating the isomers of the compound of formula XVI, for example by means of chromatography on silica gel, to give each of the following two compounds separately: 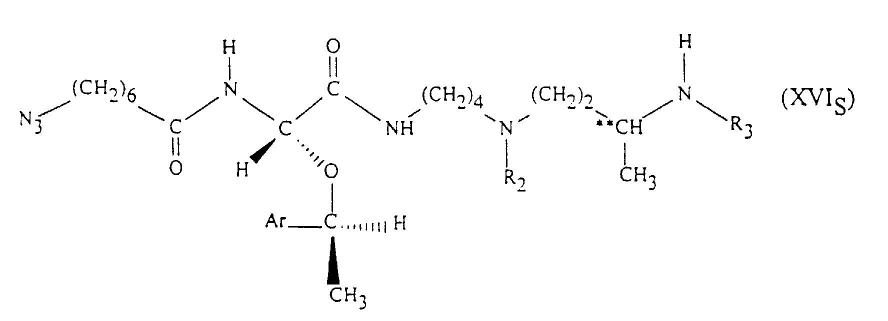 and 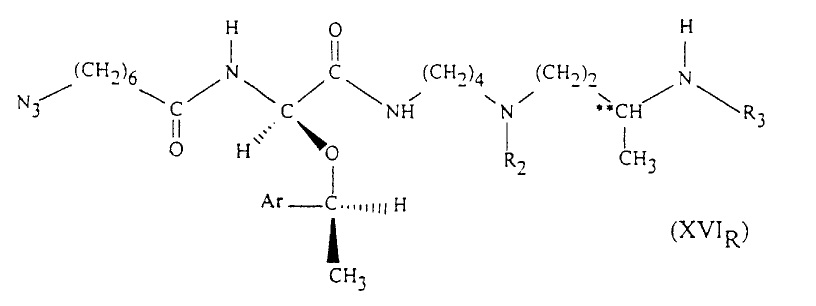 where Ar, R₁, R₂, R₃ and **C are as defined above; and
(v) reacting the resulting compound of formula XVIₛ with triphenylphosphine, in the presence of water, in an anhydrous solvent, at a temperature between 50 and 70°C, for 10 to 30 hours, to give the corresponding intermediate amine, which is reacted *in situ* with the compound of the formula 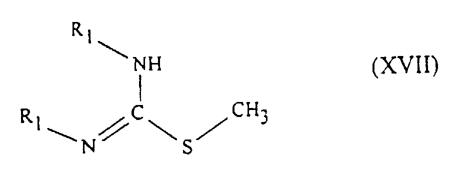 in which R₁ is an amino-protecting group, to give, after reaction for 10 to 48 h at a temperature close to room temperature, the compound of formula II: 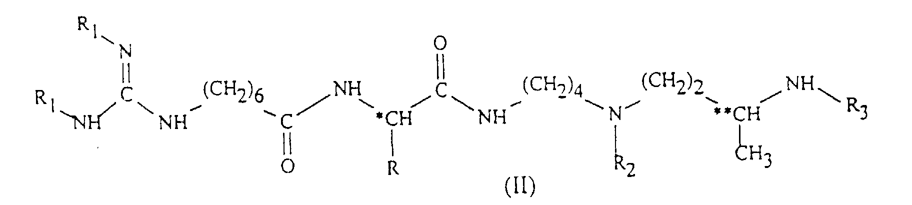 in which:
- R is the group OR',
- R₁, R₂ and R₃ are amino-protecting groups of the benzyloxycarbonyl type,
- R' is a group of the α-methylated benzyl type of the formula 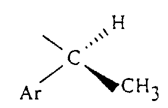
- **C is an asymmetric carbon of (R,S) or (R) configuration, and
- *C is an asymmetric carbon of (S) configuration; and
(d) according to variant D:
(i) taking a compound of formula XIV, obtained above according to the first step of the method of variant C, and separating its two diastereoisomers, especially by means of chromatography on silica gel, to give separately the two pure isomers of the formulae 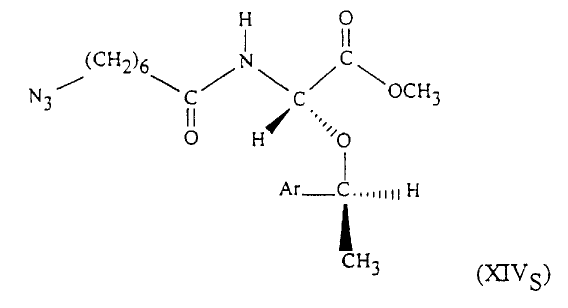 and 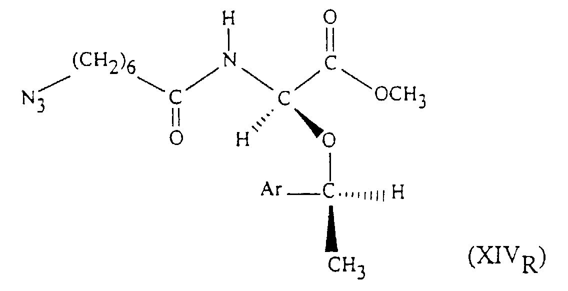 in which Ar is an aromatic radical as indicated above;
(ii) hydrolyzing the ester group of the resulting compound XIVₛ, under conditions identical to those described in variant C, to give the corresponding acid of the formula 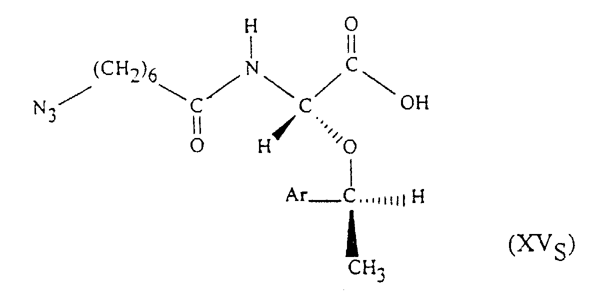 in which Ar is as defined above;
(iii) reacting the resulting compound XVₛ with an amine of formula VII: 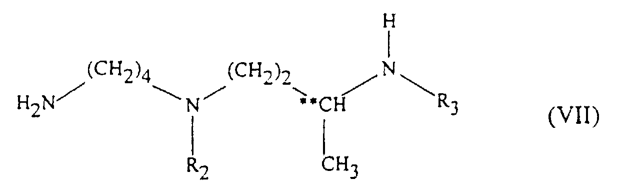 in which R₂ and R₃ are an amino-protecting group sensitive to hydrogenation and **C is a carbon atom of (R,S) or (R) configuration, under conditions identical to those described in variant C, to give the compound of the formula 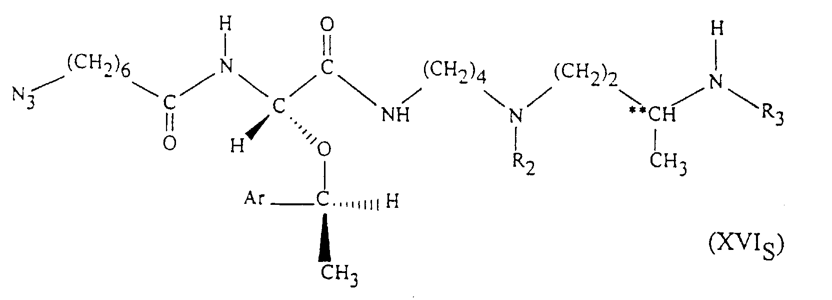 where Ar, R₂, R₃ and **C are as defined above; and then
(iv) treating the resulting compound of formula XVIs in a manner analogous to step
(v) of the method according to variant C to give the compound of formula II with the same characteristics as in the case of said variant C.

6. A method of preparing a compound of formula I in which the carbon atom *C is of (R) or (S) configuration, or one of its addition salts, characterized in that it comprises the operating mechanisms which consist in:
- obtaining, as an intermediate, a mixture of diastereoisomers of the formula 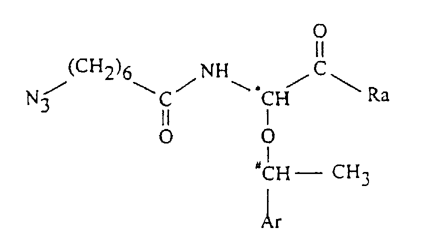 where:
- Ar is an aromatic radical,
- Ra is a C₁-C₃-alkoxy group or a group -HN-(CH₂)₄-N(R₂)-(CH₂)₂-CH(CH₃)-NH(R₃) obtained by reaction with the amine of formula VII,
- R₁, R₂ and R₃, which are identical or different, are each an amino-protecting group,
- *C is a carbon atom of (R, S) configuration, and
- ^{#}C is a carbon atom of (R) or (S) configuration; and
- separating the two isomers by methods known by those skilled in the art, for example preparative chromatography on silica gel, to give separately the compounds of the formulae 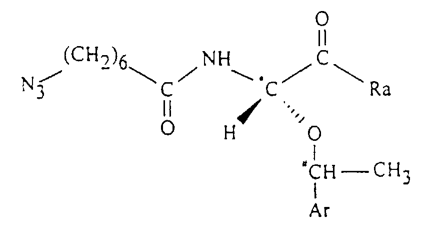 and 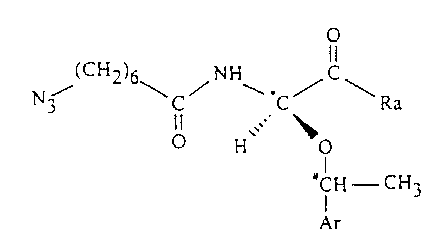 in which Ar, Ra and ^{#}C are as defined above.

7. Use of the method according to claim 6 for obtaining the S isomer of 15-deoxyspergualin [11-(S)-15-DSG], said use being characterized in that it involves the described reactions with a compound of spermidine structure of the formula NH₂-(CH₂)₄-N(R₂)-(CH₂)₃-NH(R₃) in place of the amine of formula VII.

8. An intermediate useful for the preparation of the compounds of formula (I) and/or the isomers 11-(S)-15-DSG and 11-(R)-15-DSG, characterized in that it has the formula 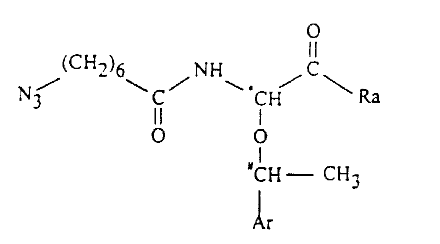 where:
- Ar is an aromatic substituent,
- Ra is a C₁-C₃-alkoxy group or a group -HN-(CH₂)₄-N(R₂)-(CH₂)₂-CH(CH₃)-NH(R₃),
- R₁, R₂ and R₃, which are identical or different, are each an amino-protecting group, especially of the benzyloxycarbonyl type, and
- ^{#}C is a carbon atom of (S) or (R) configuration.

9. An intermediate useful in the synthesis of a compound of formula (I) according to claim 1, characterized in that it is selected from the group consisting of the compounds of the formula 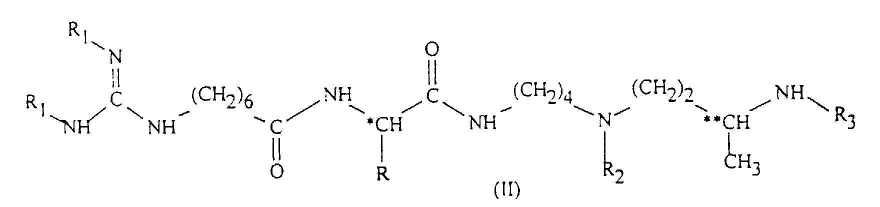 in which:
- R is a hydrogen atom, a group OCH₃, a group OH, a group CH₂OH, a group OR' or a group CH₂OR',
- R' is a protecting group for the hydroxyl group,
- R₁, R₂ and R₃, which are identical or different, are each an amino-protecting group,
- *C, when R is not the hydrogen atom, is an asymmetric carbon atom of (R,S), (R) or (S) configuration, and
- **C is an asymmetric carbon atom of (R,S) or (R) configuration.

10. An intermediate useful in the synthesis of a compound of formula I according to claim 1, characterized in that it has the formula 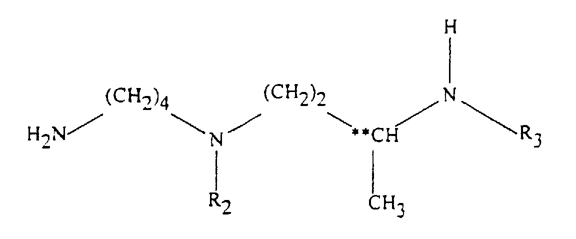 in which R₂ and R₃ are identical and are each a 1,1-dimethylethoxycarbonyl group (Boc) or a phenylmethoxycarbonyl group (Z) and **C is an asymmetric carbon atom of (R,S) or (R) configuration.

11. Use of an immunosuppressive substance selected from the group consisting of the compounds of formula I and their non-toxic addition salts according to claim 1 for the preparation of a drug intended for use in therapeutics to combat immune disorders.

12. Use of a substance selected from the group consisting of the compounds of formula I and their non-toxic addition salts according to claim 1 for the preparation of a drug intended for use in therapeutics to combat malaria.

13. Use of a substance selected from the group consisting of the compounds of formula I and their non-toxic addition salts according to claim 1 for the preparation of a drug intended for use in therapeutics to combat hyperreactive inflammatory diseases, especially ulcerative colitis or asthma.

14. Use of a substance selected from the group consisting of the compounds of formula I and their addition salts according to claim 1 as a pharmacological reagent.

15. A therapeutic composition, characterized in that it contains, in association with a physiologically acceptable excipient, at least one compound selected from the group consisting of the compounds of formula I and their non-toxic addition salts according to claim 1.
